# EUROPÄISCHE PATENTSCHRIFT

(11) **EP 0 561 830 B1**
(45) Veröffentlichungstag und Bekanntmachung des Hinweises auf die Patenterteilung: **16.04.1997**
(21) Anmeldenummer: 91920675.5
(22) Anmeldetag: 29.11.1991
(51) Int. Cl.: C07D 311/22, A01N 25/32, A01N 43/18, A01N 43/16

(54) **2-AMINO-4-OXO-4H-BENZOPYRANE, VERFAHREN ZU IHRER HERSTELLUNG UND IHRE VERWENDUNG ALS ANTIDOTS**
2-AMINO-4-OXO-4H-BENZOPYRANS, A METHOD FOR PREPARING THEM, AND THEIR USE AS ANTIDOTES
2-AMINO-4-OXO-4H-BENZOPYRANNES, LEUR PROCEDE DE FABRICATION ET LEUR UTILISATION COMME ANTIDOTES

(30) Priorität: 08.12.1990 DE 4039281
(43) Veröffentlichungstag der Anmeldung: 29.09.1993
(73) Patentinhaber: BASF Aktiengesellschaft, 67063 Ludwigshafen (DE)
(72) Erfinder: HAGEN, Helmut, D-6710 Frankenthal (DE); NILZ, Gerhard, D-67127 Roedersheim-Gronau (DE); WALTER, Helmut, D-6719 Obrigheim (DE); LANDES, Andreas, D-6703 Limburgerhof (DE); FREUND, Wolfgang, D-6730 Neustadt (DE)
(86) Internationale Anmeldenummer: EP9102268
(87) Internationale Veröffentlichungsnummer: WO9210489

(56) Entgegenhaltungen:
- EP-A- 0 094 351
- EP-A- 0 387 582
- DE-A- 2 809 720
- US-A- 3 932 466
- JOURNAL OF MEDICINAL CHEMISTRY, Bd. 28, Nr. 5, Mai 1985, Washington, DC, US, Seiten 559-568, A. NOHARA et al.
- CHEMICAL ABSTRACTS, Bd. 115, Nr. 11, 16. September 1991, Columbus, Ohio, US, Zusammenfassung-nr. 108483S
- CHEMICAL ABSTRACTS, Bd. 108, Nr. 21, 23. Mai 1988, Columbus, Ohio, US, Zusammenfassung-nr. 186576U
- CHEMICAL ABSTRACTS, Bd. 90, Nr. 7, 12. Februar 1979, Columbus, Ohio, US, Zusammenfassung-nr. 54827B
- IL FARMACO, Bd. 44, Nr. 6, Juni 1989, Pavia, IT, Seiten 565-577, A. BALBI et al.
- ARCHIV DER PHARMAZIE, Bd. 316, Nr. 1, Januar 1983, Weinheim, DE, Seiten 34-42, F. EIDEN et al.
- INDIAN JOURNAL OF CHEMISTRY, Bd. 24B, Nr. 9, September 1985, New Delhi, IN, Seiten 914-917, C.K. GHOSH et al.
- JOURNAL OF THE CHEMICAL SOCIETY, PERKIN TRANSACTIONS I, Nr. 9, September 1986, Letchworth, GB, Seiten 1643-1649, P.S. BEVAN et al.
- LIEBIGS ANNALEN DER CHEMIE, Nr. 9, September 1976, Weinheim, DE, Seiten 1659-1662, U. PETERSEN et al.

## Beschreibung

Die vorliegende Erfindung betrifft 2-Amino-4-oxo-4H-benzopyrane der allgemeinen Formel I 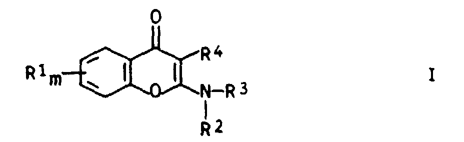 in der die Substituenten und der Index folgende Bedeutung haben:
- R¹: Wasserstoff; Hydroxy; Halogen; Nitro; C₁-C₆-Alkyl, C₁-C₆-Alkoxy ; C₁-C₆-Halogenalkyl ; C₁-C₄-Halogenalkoxy; C₁-C₄-Alkylthio oder eine Gruppe -NR⁵R⁶ mit
R⁵ Wasserstoff oder C₁-C₄-Alkyl und
R⁶ Wasserstoff, C₁-C₄-Alkyl, -CO-R⁷, -CS-R⁷ oder -SO₂-R⁸, wobei
R⁷ C₁-C₂₀-Alkyl; C₁-C₆-Halogenalkyl; C₃-C₈-Cycloalkyl; Phenyl-C₁-C₃-alkyl; Amino; C₁-C₄-Alkylamino; Di-C₁-C₄-alkylamino; eine Phenyl- oder Phenylaminogruppe, wobei die Phenylreste jeweils unsubstituiert sind oder eine bis drei der bei R¹ genannten Gruppen tragen; und
R⁸ C₁-C₄-Alkyl oder Phenyl, das eine bis drei der für R¹ genannten Gruppen tragen kann, bedeuten
- m: 0, 1 oder 2, wobei die Reste R¹ verschieden sein können, wenn m 2 bedeutet;
- R²: Wasserstoff oder C₁-C₄-Alkyl;
- R¹: Wasserstoff, C₁-C₄-Alkyl, eine Gruppe -CO-R⁹, -CS-R⁹ oder -SO₂-R¹⁰, wobei R⁹ für eine der unter R⁷ und R¹⁰ für eine der unter R⁸ genannten Bedeutungen stehen;
oder
- R² und R³: zusammen mit dem Stickstoffatom, an das sie gebunden sind, einen 3- bis 8-gliedrigen gesättigten oder ungesättigten Ring, der seinerseits gewünschtenfalls noch ein bis drei C₁-C₄-Alkylreste tragen kann,
oder eine Gruppe =CR²R¹¹, mit
R¹¹ Wasserstoff; Hydroxyl; C₁-C₄-Alkyl; C₁-C₈-Alkoxy; C₃-C₈-Cycloalkyl; Phenyl; Benzyl; Phenyl, welches eine bis drei der für R¹ genannten Gruppen trägt; eine Gruppe -O-CO-R¹² oder -O-CS-R¹², wobei
R¹² für C₁-C₂₀-Alkyl; C₁-C₆-Halogenalkyl; C₃-C₈-Cycloalkyl; Phenyl oder Phenyl-C₁-C₃-alkyl, wobei die Phenylreste jeweils unsubstituiert sein oder einen bis drei der für R¹ genannten Reste tragen können, steht;
- R⁴: Cyano; eine Gruppe -CO-R¹³, -CS-R¹³ oder -CH=N-R¹⁴, wobei
R¹³ für Wasserstoff; C₁-C₆-Alkyl; C₁-C₆-Halogenalkyl; Amino; C₁-C₄-Alkylamino; Di-C₁-C₄-alkylamino; Hydrazino; C₁-C₆-Alkylhydrazino; Phenylhydrazino, wobei der Phenylrest unsubstituiert oder durch eine bis drei der bei R¹ genannten Gruppen substituiert ist, und
R¹⁴ für einen der Reste R¹¹ steht; sowie die landwirtschaftlich brauchbaren Salze der Verbindungen 1,
ausgenommen 2-[(Methylcarbonyl)amino]-4-oxo-4H-chromen-3-carboxamid, 2-[(Methylcarbonyl)amino]-6-(tert.-butyl)-4-oxo-4H-chromen-3-carboxamid, 2-Methylamino-6-(tert.-butyl)-4-oxo-4H-chromen-3-carboxamid, 2-Methylamino-7-(tert.-butyl)-4-oxo-4H-chromen-3-carboxamid, 2-[(Methylaminocarbonyl)amino]-6-(tert.-butyl)-4-oxo-4H-chromen-3-carboxamid, 3-Cyano-2-dimethylamino-8-isopropyl-5-methyl-4-oxo-4H-chromen, 3-Cyano-2-diethylamino-7-methoxy-4-oxo-4H-chromen, 2-Dimethylamino-8-isopropyl-5-methyl-4-oxo-4H-chromen-3-carboxaldehydoxim,
2-Diethylamino-7-methoxy-4-oxo-4H-chromen-3-carboxaldehydoxim, 2-(Piperidin-1-yl)-4-oxo-4H-chromen-3-carbaldehyd, 3-Methylcarbonyl-2-(piperidin-1-yl)-4-oxo-4H-chromen, 3-Methylcarbonyl-2-amino-4-oxo-4H-chromen, 3-Methylcarbonyl-2-(N-methyl-N-isopropylamino)-4-oxo-4H-chromen, sowie diejenigen Verbindungen I, bei denen entweder R⁴ für Cyano, eine Gruppe CONH₂ oder die Formylgruppe, R² und R³ für Wasserstoff und R¹ für Wasserstoff, Hydroxy, Halogen, C₁-C₆-Alkyl, C₁-C₆-Alkoxy, Phenyl, Nitro, Amino und/oder Dimethylamino stehen,
oder bei denen R⁴ für die Formylgruppe, R² und R³ beide für Methyl oder Ethyl und R¹ für Wasserstoff, eine Methoxygruppe, 2 Methylgruppen oder eine Methyl- und eine Isopropylgruppe stehen,
oder bei denen R⁴ für eine Gruppe -CH=N-OH und R¹, R² und R³ für Wasserstoff stehen.

Außerdem betrifft die Erfindung Verfahren zur Herstellung der Verbindungen I sowie herbizider Mittel, die
A) 2-(4-Heteroaryloxy)- oder 2-(4-Aryioxy)-phenoxyessigsäurederivate der Formel VIII 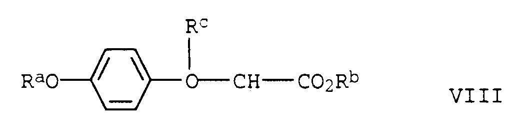
   - R^{a}: ein Phenylring, ein Pyridiylring, ein Benzoxazylrest, ein Benzthiazylrest oder ein Benzypyrazinylrest, wobei diese aromatischen Ringsysteme bis zu zwei der folgenden Reste tragen können:
   Halogen, Nitro, C₁-C₄-Alkyl, C₁-C₄-Halogenalkyl und/oder C₁-C₄-Halogenalkoxy,
   - R^{b}: Wasserstoff, eine C₁-C₄-Alkylgruppe oder das Äquivalent eines pflanzenverträglichen Kations und
   - R^{c}: Wasserstoff oder eine Methylgruppe
   und/oder
B) Cyclohexenonoximeter der Formel IX 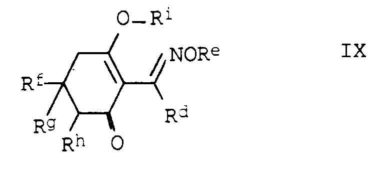 in welcher die Substituenten folgende Bedeutung haben:
   - R^{d}: eine C₁-C₄-Alkylgruppe;
   - R^{e}: eine C₁-C₄-Alkylgruppe, eine C₃-C₄-Alkenylgruppe, eine C₃-C₄-Alkinylgruppe oder eine partiell oder vollständig halogenierte C₃-C₄-Alkenylgruppe;
   eine C₁-C₄-Alkylen- oder C₃-C₄-Alkenylenkette, die beide noch einen C₁-C₃-Alkylrest und/oder ein Halogenatom tragen können, oder eine gewünschtenfalls durch C₁-C₃-Alkyl substituierte 3- bis 6-gliedrige Alkylen- oder 4- bis 6-gliedrige Alkenylenkette, die jeweils ein dem Oximetherteil nicht direkt benachbartes Sauerstoff- oder Schwefelatom als Kettenglied enthält, wobei alle vorstehend genannten Ketten endständig den Phenylring tragen, der seinerseits durch ein bis drei Reste substituiert sein kann, ausgewählt aus einer Gruppe bestehend aus einem Benzyloxycarbonyl- oder Phenylrest und jeweils einem bis drei der folgenden Reste: Nitro, Cyano, Halogen, C₁-C₄-Alkyl, C₁-C₄-Alkoxy, C₁-C₄-Alkylthio, partiell oder vollständig halogeniertes C₁-C₄-Alkyl, partiell oder vollständig halogeniertes C₁-C₄-Alkoxy, Carboxyl und C₁-C₄-Alkoxycarbonyl, und wobei der Phenylring noch zusätzlich so viele Halogenatome tragen kann, daß die Gesamtzahl der Reste 4 oder 5 beträgt; eine Thienylgruppe, die noch ein Halogenatom tragen kann;
   - R^{f}: eine C₁-C₄-Alkylgruppe, welche einfach durch C₁-C₄-Alkylthio oder C₁-C₄-Alkoxy substituiert sein kann;
   ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, wobei dieser Ring bis zu drei der folgenden Reste tragen kann: Hydroxy, Halogen, C₁-C₄-Alkyl, partiell oder vollständig halogeniertes C₁-C₄-Alkyl, C₁-C₄-Alkoxy und/oder C₁-C₄-Alkylthio;
   einen 10-gliedrigen gesättigten oder einfach ungesättigten Heterocyclus, welcher zwei Sauerstoffatome oder Schwefelatome enthält und durch bis zu drei C₁-C₄-Alkylgruppen und/oder Methoxygruppen substituiert sein kann;
   die Phenylgruppe, die Pyridylgruppe, die Thiazolylgruppe, die Pyrazolylgruppe, die Pyrrolylgruppe oder die Isoxazolylgruppe, wobei diese Gruppen jeweils einen bis drei Reste tragen können, ausgewählt aus einer Gruppe bestehend aus C₁-C₄-Alkyl, partiell oder vollständig halogeniertem C₁-C₄-Alkyl, C₁-C₄-Alkoxy, C₁-C₄-Alkylthio, C₃-C₆-Alkenyloxy, C₃-C₆-Alkinyloxy, C₁-C₄-Dialkoxy-C₁-C₃-alkyl, C₂-C₈-Alkoxyalkyl, C₂-C₈-Alkylthioalkyl, Formyl, Halogen und Benzoylamino;
   - R^{g}: Wasserstoff, Hydroxy oder, wenn R^{f} die Bedeutung C₁-C₆-Alkylgruppe hat, eine C₁-C₆-Alkylgruppe;
   - R^{h}: Wasserstoff, die Cyanogruppe, ein Halogenatom, eine C₁-C₄-Alkoxycarbonylgruppe oder eine C₁-C₄-Alkylketoximgruppe und
   - Rⁱ: Wasserstoff oder das Äquivalent eines landwirtschaftlich brauchbaren Kations,
   und 2-Amino-4-oxo-4H-benzopyrane I', wobei I' der Bedeutung von I ohne die Ausnahmen entspricht, als Antidots enthalten, sowie Verfahren zur selektiven Bekämpfung von unerwünschtem Pflanzenwuchs mit diesen herbiziden Mitteln.

Die erfindungsgemäßen Verbindungen I sind auf verschiedenen Wegen zugänglich. 2-Amino-4-oxo-4H-benzopyrane erhält man z.B. durch Formylierung von gegebenenfalls substituierten o-Hydroxyacetophenonen mit Phosphoroxytrichlorid und Dimethylformamid (H. Harnisch, Liebigs Ann. Chem. 765 (1972) 8; A. Nohara et. al., Tetrahedron Lett. 1973, 1995), Umsetzung des Aldehyds zum Oxim und dessen alkalikatalysierte Umlagerung (U. Petersen et. al., Liebigs Ann. Chem. 1976, 1659). Ein weiterer Weg ist die Umsetzung von o-Acetoxybenzoesäurechloriden mit Malonsäuredinitril (G.P. Ellis, J. Chem. Soc. Perkin Trans. I, 1986, 1643). Weitere Synthesewege für 2-Amino-4-oxo-4H-benzopyrane sind in DE-A-2 809 720; Chemical Abstracts vol. 90, no. 7, 12. Februar 1979, Columbus, Ohio, US, abstract no. 54827B, Seite 599; II Farmaco Bd. 44, Nr. 6, Juni 1989, Pavia, IT, Seiten 565-577; Archiv der Pharmazie Bd. 316, Nr. 1, Januar 1983, Weinheim, DE, Seiten 34-42, sowie in Indian Journal of Chemistry Bd. 24B, Nr. 9, September 1985, New Dehli, IN, Seiten 914-927 offenbart. Eine pflanzenschützende Wirkung der Verbindungen ist dieser Literatur nicht zu entnehmen. Einzig A. Nohara et. al. nennen in Chemical Abstracts vol. 115, no. 11, 16. September 1991, Columbus, Ohio, US, abstract no. 108483S für bestimmte 2-Amino-6-alkyl- und -6-halogen-4-oxo-4H-chromen-3-carboxamide und einzelne 2-Acylamino-6-tert.-butyl-4-oxo-4H-chromen-3-carboxamide, sowie in Chemical Abstracts vol. 108, no. 21, 23. Mai 1988, Columbus, Ohio, US, abstract no. 186576U für 2-Methylamino-6-tert.-butyl-4-oxo-4H-chromen-3-carboxamid eine fungizide Aktivität.

Der Erfindung lag nun die Aufgabe zugrunde, Verbindungen zu finden, welche die Nachteile, die bei der Verwendung der obengenannten Herbizide der Formeln VIII und IX bestehen, zumindest so stark zu verringern, daß die Herbizide für die Kulturpflanzen aus der Familie der Gräser verträglich werden.

Entsprechend der Aufgabe wurden die eingangs definierten 2-Amino-4-oxo-4H-benzopyrane I gefunden. Weiterhin wurden Verfahren zur Herstellung dieser Verbindungen I sowie zur gemeinsamen Anwendung dieser Verbindungen mit den Herbiziden VIII und IX zur Beeinflussung unerwünschten Pflanzenwachstums gefunden. Weiterhin betrifft die Erfindung Mittel, welche die Verbindungen I' enthalten, wobei es unerheblich ist, ob der herbizide Wirkstoff und die antidotische Verbindung gemeinsam oder getrennt formuliert und ausgebracht werden, bzw. bei getrennter Ausbringung, in welcher Reihenfolge herbizider Wirkstoff und Antidot appliziert werden.

Die erfindungsgemäßen Verbindungen I sind auf mehreren Wegen zugänglich. So erhält man 2-Amino-4-oxo-4H-benzopyrane beispielsweise gemäß der eingangs zitierten Literatur dadurch, daß man 2-Hydroxyacetophenone VI in einem inerten, aprotischen, polaren Lösungsmittel mit Dimethylformamid/Phosphoroxytrichlorid formyliert, den Aldehyd ins Oxim überführt und dieses unter alkalischer Katalyse in den o-Aminoaldehyd II umlagert.

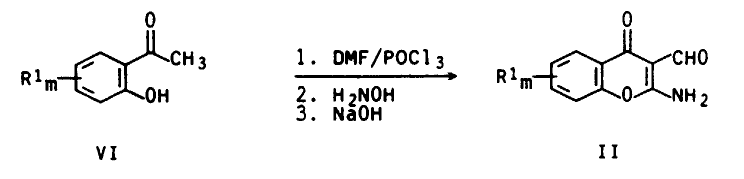

üblicherweise setzt man die Ausgangsstoffe VI und POCl₃ in stöchiometrischem Verhältnis ein. Ein Überschuß des einen oder anderen kann in Einzelfällen aber durchaus vorteilhaft sein.

Die Umsetzung kann kontinuierlich oder diskontinuierlich bei Normal-, über- oder Unterdruck nach den dafür üblichen Techniken durchgeführt werden. Die Reaktionstemperatur liegt im allgemeinen bei (-10) bis 100°C, insbesondere bei 0 bis 40°C.

Als Lösungsmittel dienen beispielsweise aliphatische und aromatische chlorierte Kohlenwasserstoffe wie Dichlormethan, Chloroform und Chlorbenzol oder im Überschuß vorhandenes Dimethylformamid.

Die Synthese der Verbindungen mit R⁴ = CN erfolgt z.B. ausgehend von den Aldehyden (R⁴ = CHO) durch Umsetzung mit Hydroxylammoniumchlorid in Ameisensäure (P. Kurtz in Houben-Weyl, Methoden der Organischen Chemie), Band 8, Seite 325-330 oder Olah, Synthesis 1979, 122).

Triocarbonsäureamide (R⁴ = -CS-NH₂) werden durch Anlagerung von Schwefelwasserstoff an das entsprechende Nitril (R⁴ = CN) in Pyridin/Triethylamin als Lösungsmittel erzeugt. Anschließend kann R¹³ = NH₂ durch aromatische oder aliphatische Amine substituiert werden (A. Schöberl in Houben-Weyl, Methoden der Organischen Chemie, Band 9, Seite 762-769).

Carbonsäurederivate (R⁴ = -CO-R¹³) sind durch Hydrolyse der Nitrile (R⁴ = CN) und Reaktion der entstandenen Carbonsäure mit Alkoholoen oder Aminen zugänglich. Das Carbonsäureamid (R⁴ = -CO-NH₂) wird bei Addition von Wasser an das Nitril (R⁴ = CN) erhalten (H. Henecka in Houben-Weyl, Methoden der Organischen Chemie, Band 8, Seiten 427-432 und 661-665).

Schiff'sche Basen (R⁴ = -CH=N-R¹⁴) werden z.B. durch Umsetzung der Aldehyde (R⁴ = CHO) mit primären Aminen in aromatischen Kohlenwasserstoffen unter Säurekatalyse und Abscheidung des Reaktionswassers gewonnen (H. Freytag in Houben-Weyl, Methoden der Organischen Chemie, Band 11/2, Seite 73-98).

Die Derivatisierung der Aminogruppen erfolgt durch Umsetzung des 2-Aminonitrils (R², R³ = H; R⁴ = CN) oder 2-Aminooxims (R², R³ = H; R⁴ = -CH=NOH) mit Carbonsäureanhydriden oder -chloriden (H. Henecka in Houben-Weyl, Methoden der Organischen Chemie, Band 8, Seite 655-658).

Im Hinblick auf die bestimmungsgemäße Verwendung der Verbindungen I als pflanzenschützende Mittel kommen als Substituenten folgende Reste in Betracht:
- m: 0, 1 oder 2, wobei die Reste R¹ verschieden sein können, wenn m 2 bedeutet;
- R¹: Wasserstoff; Hydroxy; Nitro;
Halogen wie Fluor, Chlor, Brom und Jod, insbesondere Chlor und Brom; C₁-C₆-Alkyl wie Methyl, Ethyl, n-Propyl, 1-Methylethyl, n-Butyl, 1-Methylpropyl und 1,1-Dimethylethyl, besonders bevorzugt Methyl und Ethyl; C1-C6-Alkoxy wie Methoxy, Ethoxy, n-Propoxy, 1-Methylethoxy, n-Butoxy, 1-Methylpropoxy, 2-Methylpropoxy, Hexoxy, insbesondere Methoxy und Ethoxy;
C₁-C₆-Halogenalkyl, insbesondere C₁- und C₂-Halogenalkyl, wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluormethyl, 2-Chlor-2,2-difluorethyl, 2,2-Dichlor-2-fluorethyl, 2,2,2-Trichlorethyl und Pentafluorethyl, besonders bevorzugt Trichlormethyl und Trifluormethyl;
C₁-C₄-Halogenalkoxy, insbesondere C₁- und C₂-Halogenalkoxy, wie Difluormethoxy, Trifluormethoxy, Chlor-difluormethoxy, Dichlorfluormethoxy, 1-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, besonders bevorzugt 2,2,2-Trifluorethoxy;
C₁-C₄-Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio;
eine Gruppe NR⁵R⁶; mit
R⁵ Wasserstoff oder C₁-C₄-Alkyl wie Methyl, Ethyl, Isopropyl und tert.-Butyl und
R⁶ Wasserstoff, C₁-C₄-Alkyl wie Methyl, Ethyl, Isopropyl und tert.-Butyl, -CO-R⁷, -CS-R⁷ oder -SO₂-R⁸;
R⁷ C₁-C₂₀-Alkyl wie bei R¹ genannt, sowie Octyl, Dodecyl, Hexadecyl und Octadecyl, bevorzugt C₁-C₆-Alkyl, insbesondere Methyl und Ethyl;
Halogenalkyl wie bei R¹ genannt;
C₃-C₈-Cycloalkyl wie Cyclopropyl, Cyclobutyl, Cyclopentyl, Cyclohexyl, Cycloheptyl, Cyclooctyl, insbesondere Cyclohexyl;
Phenyl-C₁-C₃-alkyl wie Benzyl, 2-Phenylethyl, 3-Phenylpropyl, vorzugsweise Benzyl ;
Amino, welches ein oder zwei der für R¹ genannten Alkylreste, insbesondere Methyl, tragen kann, wobei diese Reste bei zweifach substituiertem Amino verschieden sein können, insbesondere Methylbenzylamino; Aminophenyl, wobei der aromatische Ring eine bis drei der bei R¹ genannten Gruppen tragen kann;
Phenyl, das eine bis drei der für R¹ genannten Gruppen tragen kann;
R⁸ C₁-C₄-Alkyl wie bei R¹ genannt, insbesondere Methyl und Ethyl;
Phenyl, das eine bis drei der für R¹ genannten Gruppen tragen kann;
- R²: Wasserstoff;
C₁-C₄-Alkyl wie Methyl, Ethyl, n-Propyl, Isopropyl, n-Butyl und tert.-Butyl, vorzugsweise Methyl und Ethyl,
- R³: Wasserstoff; C₁-C₄-Alkyl wie Methyl, Ethyl, n-Propyl, Isopropyl, n-Butyl und tert.-Butyl, vorzugsweise Methyl und Ethyl;
eine Gruppe -CO-R⁹ oder -CS-R⁹, insbesondere -CO-(C₁-C₆-Alkyl),
eine Gruppe -SO₂-R¹⁰, insbesondere -SO₂-CH₃, -SO₂C₂H₅, -SO₂C₆H₅;
R⁹ C₁-C₂₀-Alkyl wie bei R¹ genannt sowie Octyl, Dodecyl, Hexadecyl, Octadecyl, bevorzugt C₁-C₆-Alkyl, insbesondere Methyl und Ethyl;
Halogenalkyl wie bei R¹ genannt;
C₃-C₈-Cycloalkyl wie Cyclopropyl, Cyclobutyl, Cyclopentyl, Cyclohexyl, Cycloheptyl, Cyclooctyl, insbesondere Cyclohexyl;
Phenyl-C₁-C₃-alkyl wie Phenylmethyl, 2-Phenylethyl, 3-Phenylpropyl, insbesondere Phenylmethyl;
Amino, welches ein oder zwei der für R¹ genannten Alkylreste, insbesondere Methyl, tragen kann, wobei diese Reste bei zweifach substituiertem Amino verschieden sein können, insbesondere Methylbenzylamino; Aminophenyl, wobei der aromatische Ring eine bis drei der bei R¹ genannten Gruppen tragen kann;
Phenyl, das eine bis drei der für R¹ genannten Gruppen tragen kann;
R¹⁰ C₁-C₄-Alkyl wie bei R¹ genannt, insbesondere Methyl und Ethyl;
Phenyl, das eine bis drei der für R¹ genannten Gruppen tragen kann;
- R⁴: Cyano;
eine Gruppe -CO-R¹³, -CS-R¹³ oder -CH=N-R¹⁴;
- R¹³: Wasserstoff;
Alkyl oder Halogenalkyl wie bei R¹ genannt, insbesondere Methyl und Ethyl, Trifluormethyl, Trichlormethyl, Amino,
welches einen oder zwei der bei R¹ genannten Alkylreste, insbesondere Methyl, tragen kann, wobei diese Reste bei zweifach substituiertem Amino verschieden sein können, insbesondere Methylbenzylamino;
Hydrazino, welches durch eine der bei R² genannten Gruppen oder Phenyl, das eine bis drei der bei R¹ genannten Gruppen tragen kann, substituiert sein kann;
oder
- R² und R³: zusammen mit dem gemeinsamen Stickstoffatom, an das sie gebunden sind
einen 3 bis 8-gliedrigen, insbesondere 5- oder 6-gliedrigen gesättigten oder ungesättigten Ring bilden, der unsubstituiert oder durch ein bis drei C₁-C₄-Alkylgruppen, z.B. Methylgruppen substituiert ist, beispielsweise seien Piperidyl oder eine ggf. substituierte Pyrrolylgruppe genannt, die durch Umsetzung des Amins (R², R³ = H) mit ggf. substituierten 2,5-Dimethoxytetrahydrofuranen oder 1,4-Diketonen wie Acetonylaceton zugänglich ist (Robba, Chem. Pharm. Bull. 33 (1985) 2798);
oder eine Gruppe =CR²R¹¹
- R¹¹: Wasserstoff, Hydroxyl;
C₃-C₈-Cycloalkyl wie Cyclopropyl, Cyclopentyl, Cyclohexyl, Cycloheptyl und Cyclooctyl, insbesondere Cyclohexyl;
Phenyl,das eine bis drei der für R¹ genannten Gruppen tragen kann; Benzyl;
C₁-C₄-Alkyl wie Methyl, Ethyl, n-Propyl, Isopropyl, n-Butyl und tert.-Butyl, vorzugsweise Methyl und Ethyl;
C₁-C₆-Alkoxy wie bei R¹ genannt;
eine Gruppe -O-CO-R¹² oder -O-CS-R¹²
- R¹²: C₁-C₂₀-Alkyl wie bei R¹ genannt, sowie n-Octyl, Dodecyl, Hexadecyl und Octadecyl, insbesondere C₁-C₆-Alkyl wie bei R¹ genannt, vorzugsweise Methyl und Ethyl;
Halogenalkyl wie bei R¹ genannt, insbesondere Chlorethyl;
C₃-C₈-Cycloalkyl wie Cyclopropyl, Cyclopentyl, Cyclohexyl, Cycloheptyl und Cyclooctyl, insbesondere Cyclohexyl;
Phenyl-C₁-C₃-alkyl wie Benzyl, 2-Phenylethyl und 3-Phenylpropyl, vorzugsweise Benzyl;
Phenyl, das einen bis drei der bei R¹ genannten Reste tragen kann;

Als Salze der Verbindung I kommen landwirtschaftlich brauchbare Salze, beispielsweise Alkalimetallsalze, insbesondere das Kalium- oder Natriumsalz, Erdalkalimetallsalze, insbesondere das Calcium-, Magnesium- oder Bariumsalz, Mangan-, Kupfer-, Zink- oder Eisensalze sowie Ammonium-, Phosphonium-, Tetraalkylammoniumsalze, Benzyltrialkylammoniumsalze, Trialkylsulfoniumsalze oder Trialkylsulfoxoniumsalze in Betracht.

Die 2-Amino-4-oxo-4H-benzopyrane I und I' eignen sich als Antidots, um herbizide Wirkstoffe für Kulturpflanzen wie Kulturhirse, Reis, Mais, Getreidearten (Weizen, Roggen, Gerste, Hafer), Baumwolle, Zuckerrüben, Zuckerrohr und Soja verträglicher zu machen. Sie wirken antagonistisch auf Herbizide verschiedenster Stoffklassen wie Triazine, Phenylharnstoffderivate, Carbamate, Thiolcarbamate, Halogenacetanilide, Benzoesäurederivate sowie insbesondere Halogenphenoxyessigsäureester, substituierte Phenoxyphenoxyessigsäureester, Phenoxyphenoxypropionsäureester und Cyclohexenonderivate.

Herbizide Wirkstoffe aus der Gruppe der 2-(4-Heteroaryloxy)- oder 2-(4-Aryloxy)-phenoxyessigsäurederivate der Formel VIII, 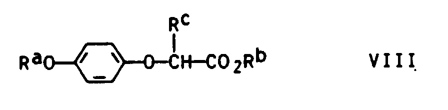 in der die Substituenten folgende Bedeutung haben:
- R^{a}: ein Phenylring, ein Pyridylring, ein Benzoxazylrest, ein Benzthiazylrest oder ein Benzpyrazinylrest, wobei diese aromatischen Ringsysteme bis zu zwei der folgenden Reste tragen können: Halogen, Nitro, C₁-C₄-Alkyl, C₁-C₄-Halogenalkyl und/oder C₁-C₄-Halogenalkoxy,
- R^{b}: Wasserstoff, eine C₁-C₄-Alkylgruppe oder das Äquivalent eines pflanzenverträglichen Kations und
- R^{c}: Wasserstoff oder eine Methylgruppe,
sind aus der Literatur, z. B. aus DE-A-22 23 894, DE-A-24 33 067, DE-A-25 76 251, DE-A-30 04 770, BE-A-868 875 und BE-A-858 618 bekannt.

Sie dienen der Bekämpfung von unerwünschten Pflanzen aus der Familie der Gramineen. Die Verträglichkeit dieser Substanzen für Kulturpflanzen variiert jedoch zwischen kommerziell acceptabel und unverträglich, je nach Substituenten und Aufwandmenge.

Ebenso verhält es sich mit Cyclohexenonderivaten der Formel IX, 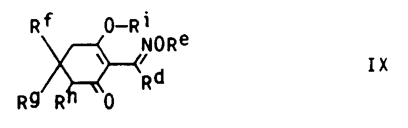 in welcher die Substituenten folgenden Bedeutung haben:
- R^{d}: eine C₁-C₄-Alkylgruppe wie Methyl, Ethyl, n-Propyl, Isopropyl, n-Butyl und tert.-Butyl, vorzugsweise Ethyl und n-Propyl;
- R^{e}: eine C₁-C₄-Alkylgruppe wie Methyl, Ethyl, n-Propyl, Isopropyl, n-Butyl und tert.-Butyl, vorzugsweise Ethyl und n-Propyl, eine C₃-C₄-Alkenylgruppe, vorzugsweise Prop-2-enyl, eine C₃-C₄-Alkinylgruppe oder eine C₃-C₄-Halogenalkenylgruppe, vorzugsweise 3-Chlor-prop-2-en-1-yl,
eine C₁-C₄-Alkylen- oder C₁-C₄-Alkenylenkette, die beide noch ein bis drei C₁-C₃-Alkylreste und/oder Halogenatome tragen können, oder eine gewünschtenfalls durch C₁-C₃-Alkyl substituierte 3- bis 6-gliedrige Alkylen- oder 4- bis 6-gliedrige Alkenylenkette, die jeweils ein dem Oximetherteil nicht direkt benachbartes Sauerstoff- oder Schwefelatom als Kettenglied enthält, wobei alle vorstehend genannten Ketten endständig den Phenylring tragen, der seinerseits durch ein bis drei Reste substituiert sein kann, ausgewählt aus einer Gruppe bestehend aus einem Benzyloxycarbonyl- oder Phenylrest und jeweils einem bis drei der folgenden Reste: Nitro, Cyano, Halogen, C₁-C₄-Alkyl, C₁-C₄-Alkoxy, C₁-C₄-Alkylthio, partiell oder vollständig halogeniertes C₁-C₄-Alkyl, partiell oder vollständig halogeniertes C₁-C₄-Alkoxy, Carboxyl und C₁-C₄-Alkoxycarbonyl, und wobei der Phenylring noch zusätzlich so viele Halogenatome tragen kann, daß die Gesamtzahl der Reste 4 oder 5 beträgt;
besonders bevorzugt sind 4-(p-Fluorphenyl)-but-3-enyl, 4-(p-Chlorphenyl)-but-3-enyl und 2-(p-Chlorphenoxy)-propyl; die Thienylgruppe, die noch ein Halogenatom tragen kann;
- R^{f}: eine C₁-C₄-Alkylgruppe wie bei R^{d} genannt, welche ein- oder zweifach durch C₁-C₄-Alkylthio oder C₁-C₄-Alkoxy substituiert sein kann;
ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, vorzugsweise Tetrahydropyranyl, Dihydropyranyl und Tetrahydrothiopyranyl, wobei das Ringsystem noch einen bis drei Reste tragen kann, ausgewählt aus einer Gruppe bestehend aus Hydroxy, Halogen, C₁-C₄-Alkyl, C₁-C₄-Halogenalkyl, C₁-C₄-Alkoxy und C₁-C₄-Alkylthio;
einen 10-gliedrigen gesättigten oder einfach ungesättigten Heterocyclus, welcher zwei nicht benachbarte Sauerstoffatome oder Schwefelatome enthält und der durch bis zu drei C₁-C₄-Alkylgruppen und/oder Methoxygruppen substituiert sein kann;
die Phenylgruppe, eine Pyridylgruppe, eine Thiazolylgruppe, eine Pyrazolylgruppe, eine Pyrrolylgruppe oder eine Isoxazolylgruppe, wobei jede dieser Gruppen gewünschtenfalls noch ein bis drei Reste tragen kann, ausgewählt aus einer Gruppe bestehend aus: C₁-C₄-Alkyl, C₁-C₄-Halogenalkyl, C₁-C₄-Alkoxy, C₁-C₄-Alkylthio, C₃-C₆-Alkenyloxy, C₃-C₆-Alkinyloxy, C₁-C₄-Alkoxy-C₁-C₃-alkyl, C₁-C₄-Dialkoxy-C₁-C₃-alkyl, Formyl, Halogen und Benzoylamino;
- R^{g}: Wasserstoff, Hydroxy oder, wenn R^{f} die Bedeutung C₁-C₆-Alkylgruppe hat, eine C₁-C₆-Alkylgruppe, vorzugsweise Wasserstoff;
- R^{h}: Wasserstoff, die Cyanogruppe, ein Halogenatom, eine C₁-C₄-Alkoxycarbonylgruppe oder eine C₁-C₄-Alkylketoximgruppe, vorzugsweise Wasserstoff;
- Rⁱ: Wasserstoff oder das Äquivalent eines landwirtschaftlich brauchbaren Kations.

Sie sind in der Literatur (z.B. EP-A 228 598, EP-A 230 235, EP-A 238 021, EP-A 368 227, US-A 4 432 786, DE-A 24 39 104, DE-A 40 14 986 und DE-A 40 33 423) ebenfalls als Herbizide beschrieben und dienen vorwiegend zur Bekämpfung unerwünschter Gräser in dicotylen Kulturen und in Gräsern, die nicht zur Familie der Gramineen zählen. Je nach Struktur der Substituenten und Dosis der Anwendung können Verbindungen aus dieser Gruppe auch zur selektiven Bekämpfung von unerwünschten Gräsern in Gramineen-Kulturen wie Weizen und Reis eingesetzt werden.

Cyclohexenonderivate der Formel IX, in denen R^{e} für einen unsubstituierten oder substituierten Alkyl- oder Alkenyl-, z.B. Butyl- oder Butenylphenylrest steht, können in an sich bekannter weise aus schon bekannten Derivaten der Formel X (EP-A-80 301, EP-A-125 094, EP-A 142 741, US-A 4 249 937, EP-A 137 174 und EP-A 177 913) und den entsprechenden Hydroxylaminen der Formel XI (Houben-Weyl, 10/1, S. 1181 ff) hergestellt werden (EP-A 169 521).

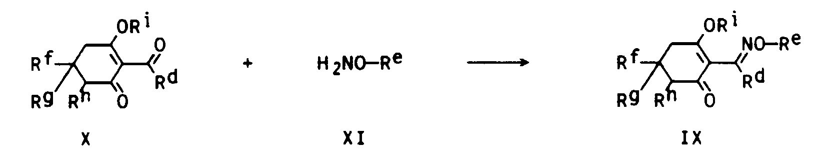

Zweckmäßig führt man die Umsetzung in heterogener Phase in einem Lösungsmittel, bei einer ausreichenden Temperatur unterhalb von etwa 80°C, in Gegenwart einer Base durch und verwendet das Hydroxylamin XI in Form seines Ammoniumsalzes.

Geeignete Basen sind z. B. Carbonate, Hydrogencarbonate, Acetate, Alkoholate oder Oxide von Alkali- oder Erdalkalimetallen, insbesondere Natriumhydroxid, Kaliumhydroxid, Magnesiumoxid, Calciumoxid. Außerdem können organische Basen wie Pyridin oder tertiäre Amine Verwendung finden. Die Base wird beispielsweise in einer Menge von 0,5 bis 2 Mol-Äquivalent bezogen auf die Ammoniumverbindung zugesetzt.

Als Lösungsmittel eignen sich beispielsweise Dimethylsulfoxid, Alkohole wie Methanol, Ethanol und Isopropanol, aromatische Kohlenwasserstoffe wie Benzol und Toluol, chlorierte Kohlenwasserstoffe wie Chloroform und Dichlorethan, aliphatische Kohlenwasserstoffe wie Hexan und Cyclohexan, Ester wie Essigsäureethylester sowie Ether wie Diethylether, Dioxan und Tetrahydrofuran. Vorzugsweise führt man die Umsetzung in Methanol mit Natriumhydrogencarbonat als Base durch.

Die Reaktion ist nach wenigen Stunden beendet. Das Produkt kann z.B. durch Einengen der Mischung, Verteilung des Rückstandes in Methylenchlorid/ Wasser und Abdestillieren des Lösungsmittels unter vermindertem Druck isoliert werden.

Man kann für diese Umsetzung aber auch unmittelbar die freie Hydroxylaminbase, z.B. in Form einer wäßrigen Lösung, verwenden; je nach verwendetem Lösungsmittel für die Verbindung X erhält man ein ein- oder zweiphasiges Reaktionsgemisch.

Geeignete Lösungsmittel für diese Variante sind beispielsweise Alkohole wie Methanol, Ethanol, Isopropanol und Cyclohexanol, aliphatische und aromatische, gegebenenfalls chlorierte Kohlenwasserstoffe wie Hexan, Cyclohexan, Methylenchlorid, Toluol und Dichlorethan, Ester wie Essigsäureethylester, Nitrile wie Acetonitril sowie cyclische Ether wie Dioxan und Tetrahydrofuran.

Alkalimetallsalze der Verbindungen IX können durch Behandeln der 3-Hydroxyverbindungen mit Natrium- oder Kaliumhydroxid bzw. -alkoholat in wäßriger Lösung oder in einem organischen Lösungsmittel wie Methanol, Ethanol, Aceton und Toluol erhalten werden.

Andere Metallsalze wie Mangan-, Kupfer-, Zink-, Eisen-, Calcium-, Magnesium- und Bariumsalze können aus den Natriumsalzen in üblicher Weise hergestellt werden, ebenso Ammonium- und Phosphoniumsalze mittels Ammoniak, Ammonium-, Phosphonium-, Sulfonium- oder Sulfoxoniumhydroxiden.

Die Verbindungen des Typs X können z.B. aus den entsprechenden Cyclohexan-1,3-dionen der Formel XII 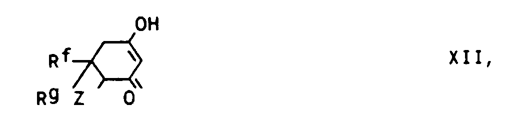 in der
- Z: Wasserstoff oder Methoxycarbonyl und
- R^{g}: Wasserstoff bedeuten
nach bekannten Methoden (Tetrahedron Lett., 2491 (1975)) hergestellt werden.

Es ist auch möglich, die Verbindungen der Formel X über die Zwischenstufe der Enolester herzustellen, die bei der Umsetzung von Verbindungen der Formel XII mit Säurechloriden in Gegenwart von Basen anfallen und anschließend mit bestimmten Imidazol-oder Pyridinderivaten umgelagert werden (JP-OS 79/063 052).

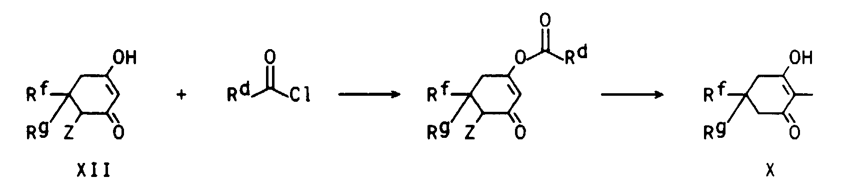

Zu den Verbindungen der Formel XII gelangt man über eine Reihe bekannten Verfahrensschritte ausgehend von bekannten Vorprodukten.

Die Synthese der Hydroxylamine XI, in denen R^{e} für einen ggf. substituierten Phenylbutylrest steht, erfolgt gemäß dem nachstehenden Reaktionsschema beispielsweise über
α) Die Alkylierung von cyclischen Hydroxyimiden XIII mit geeigneten Phenylbutylhalogeniden und anschließende Schutzgruppenabspaltung beispielsweise mit Hydrazin oder Ethanolamin analog Beispielen aus EP-A-244 786 bzw. Houben-Weyl, Methoden der organischen Chemie, Band X/1, Seite 1152ff.
β) Hydrierung von N-4-Phenylbutenyloxyphthalimiden, deren Herstellung in DE-A 38 38 310 beschrieben ist, mittels geeigneten Katalysatoren wie z.B. Palladium auf Aktivkohle, in geeigneten inerten Lösungsmitteln wie z.B. Methanol, Tetrahydrofuran, Dioxan und anschließende Schutzgruppenabspaltung wie voranstehend beschrieben.
Vorteilhaft wird die Hydrierung bei Temperaturen von 20°C bis zum Siedepunkt des Lösungsmittels, insbesondere bei Raumtemperatur nach den dafür üblichen Techniken bei Atmosphärendruck, über- oder Unterdruck durchgeführt. Bevorzugt ist ein Druckbereich von 1 bis 10, insbesondere 1 bis 2 bar.

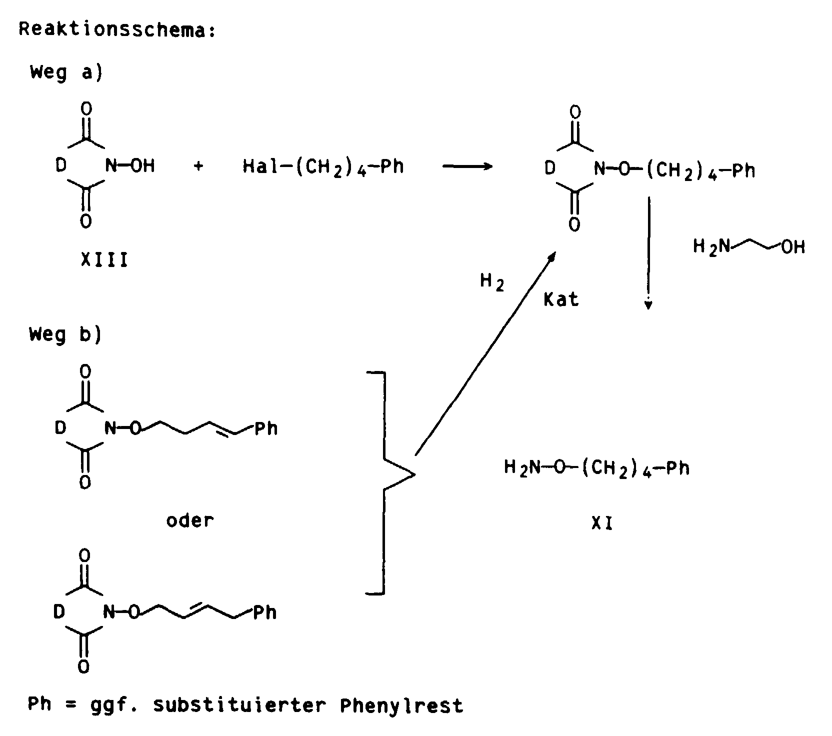

Als cyclische Hydroxyimide XIII kommen beispielsweise folgende Substanzen in Betracht:

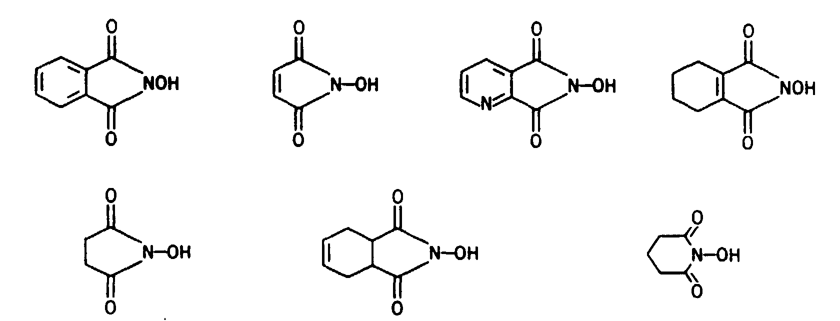

Die Synthese der Hydroxylamine XI, in denen R^{e} für einen unsubstituierten oder substituierten Butenylphenylrest steht, wobei der nachstehend mit Ph abgekürzte Phenylrest seinerseits substituiert oder unsubstituiert sein kann, erfolgt gemäß dem nachstehenden Reaktionsschema ausgehend von Anilinderivaten durch Diazotierung und anschließende Kupplung des Diazoniumsalzes mit einem entsprechend substituierten Butadien XIV. Das so erhaltene Gemisch aus XVa und XVb wird mit einem cyclischen Hydroxyimid XVII gekoppelt und das erhaltene geschützte Hydroxylaminderivat XVI mit 2-Aminoethanol zum freien Hydroxylamin XI gespalten:

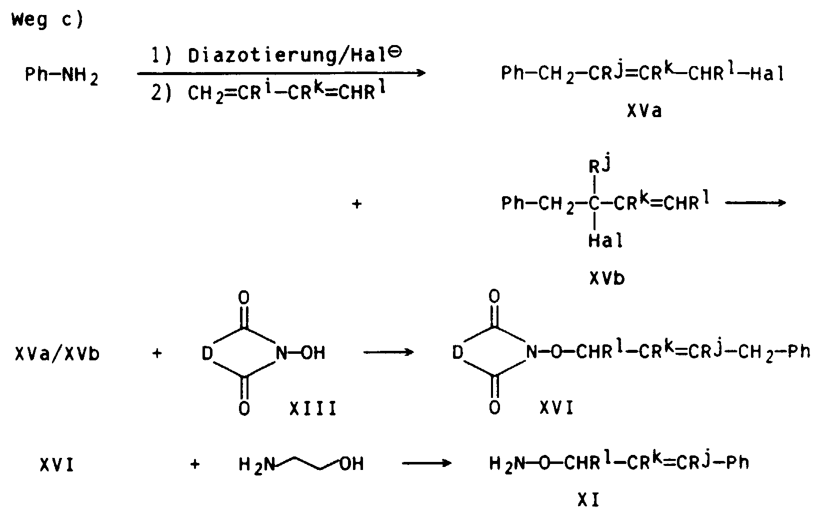

Die Reste R^{j}, R^{k} und R^{l} bedeuten hierbei unabhängig voneinander Wasserstoff, C₁-C₃-Alkylgruppen und/oder Halogenatome. Hal bedeutet Halogenatom, vorzugsweise Chloratom.

Die zur obigen Synthese der Hydroxylamine der Formel XI benötigten Halogenide XVa lassen sich nach literaturbekannten Verfahren im Gemisch mit XVb herstellen, beispielsweise durch die Umsetzung von Diazoniumsalzen aromatischer und heteroaromatischer Aniline mit Dienen. Die Anwendungsbreite der Reaktion ist in Organic Reactions 11 (1960) 189 bzw. 24 (1976) 225 abgehandelt.

Bei der Kopplung der isomeren Halogenide XVa und XVb an ein cyclisches Hydroxyimid der Formel XIII entstehen ausschließlich die cyclischen Imidether der Formel XVI, die nach Abspaltung der Schutzgruppe am Stickstoff die Hydroxylamine XI liefern.

Die Umsetzung mit einem Hydroxyimid XIII (Weg a und c) erfolgt in Gegenwart eines säurebindenden Mittels und eines Lösungsmittels. Aus Kostengründen kommt als Hydroxyimid XIII bevorzugt Hydroxyphthalimid zum Einsatz.

Als säurebindende Mittel eignen sich Alkalimetallcarbonate wie Kalium- oder Natriumcarbonat, Alkalimetallhydrogencarbonate wie Kalium- und Natriumhydrogencarbonat, tertiäre Amine wie Trimethyl- und Triethylamin und basische Heterocyclen wie Pyridin. Aus Kostengründen kommen bevorzugt Kalium- und Natriumcarbonat in Betracht.

Als Lösungsmittel eignen sich aprotisch dipolare organische Lösungsmittel wie z.B. Dimethylformamid, Dimethylsulfoxid und/oder Sulfolan.

Ferner ist auch eine Alkylierung unter Phasentransferbedingungen möglich. Als organische Lösungsmittel werden hierbei mit Wasser nicht mischbare Verbindungen wie Kohlenwasserstoffe oder Chlorkohlenwasserstoffe eingesetzt. Als Phasentransferkatalysatoren eignen sich quartäre Ammonium- und Phosphoniumsalze.

Die Spaltung der cyclischen Imidether XVI gelingt analog einem in EP-A 244 786 beschriebenen Verfahren mit Alkanolaminen. Die Hydroxylamine XI können nach diesem Verfahren als freie Basen oder nach Fällung mit Säuren als Salze isoliert werden. Gut kristallisierende Salze erhält man durch Umsetzung der Basen mit Oxalsäure.

Spezielle Beispiele für herbizide (Heteroaryloxy)- bzw. Aryloxyphenoxyessigsäurederivate der Formel VIII, deren Kulturpflanzenverträglichkeit durch 2-Amino-4-oxo-4H-benzopyrane der Formel I oder I' verbessert werden kann, sind in der folgenden Tabelle 1 aufgeführt.

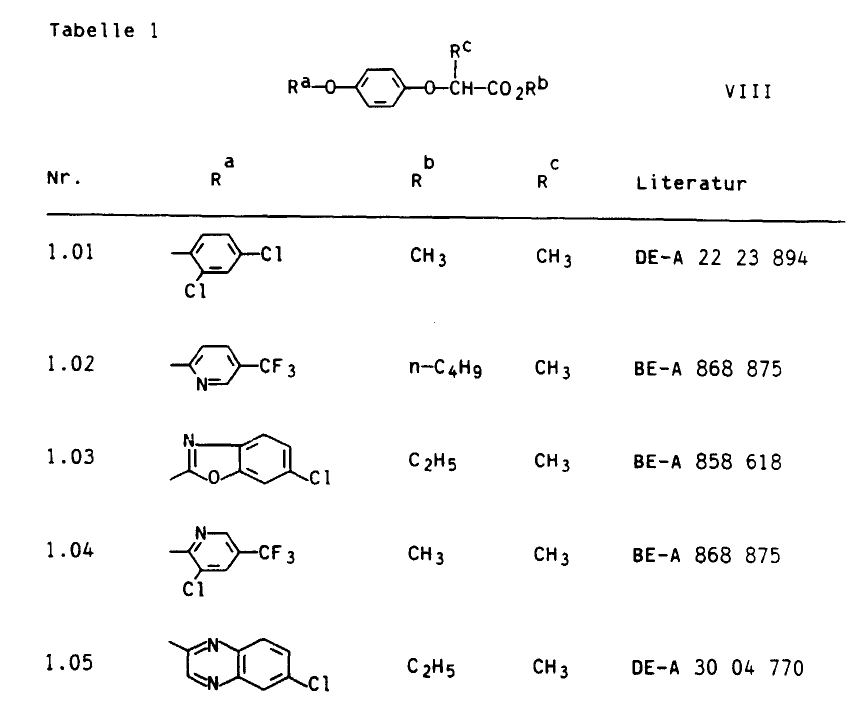

Spezielle Beispiele für Cyclohexenone der Formel IX, deren Kulturpflanzenverträglichkeit durch 2-Amino-4-oxo-4H-benzopyrane I und I' verbessert werden kann, sind in den folgenden Tabellen 2 bis 13 aufgeführt.

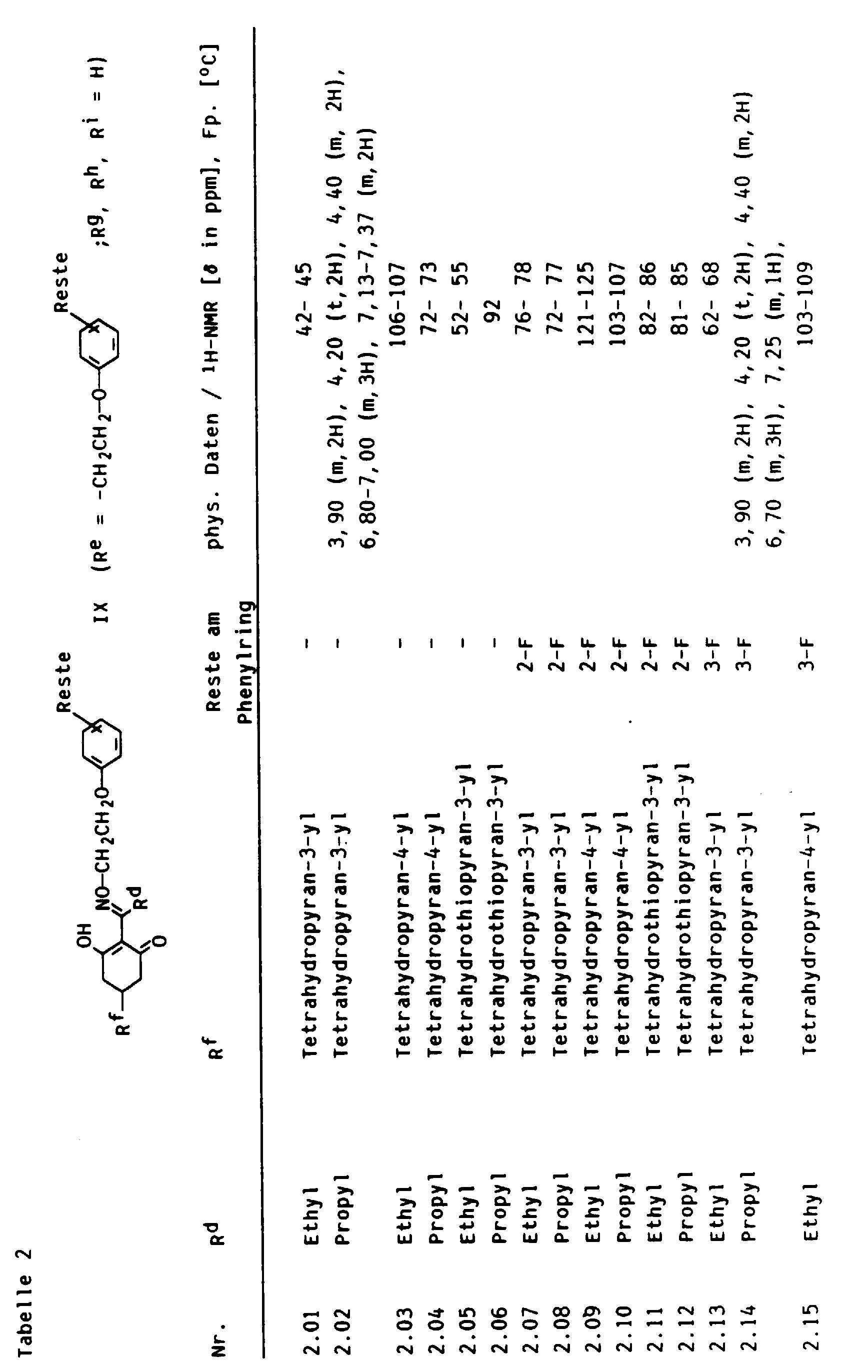

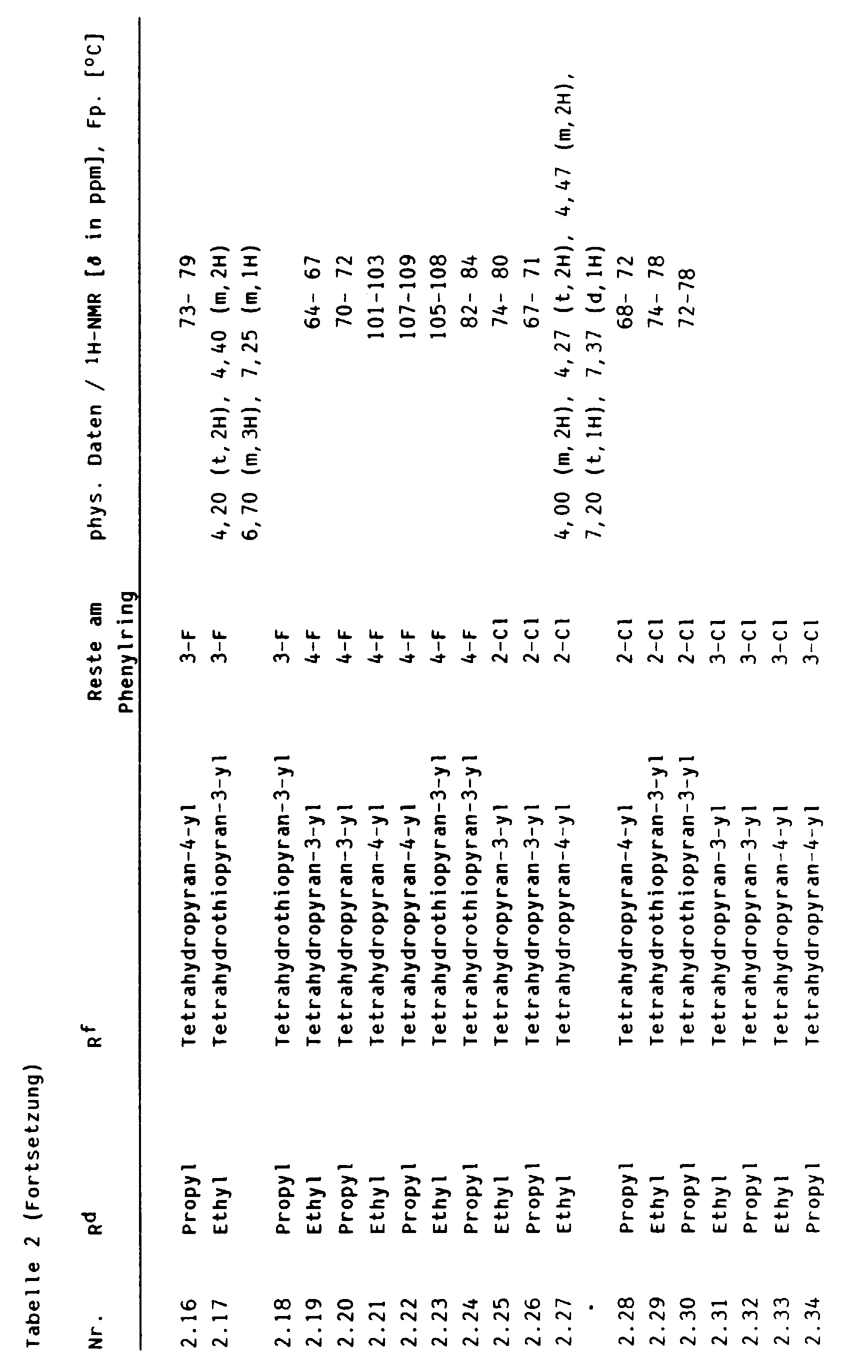

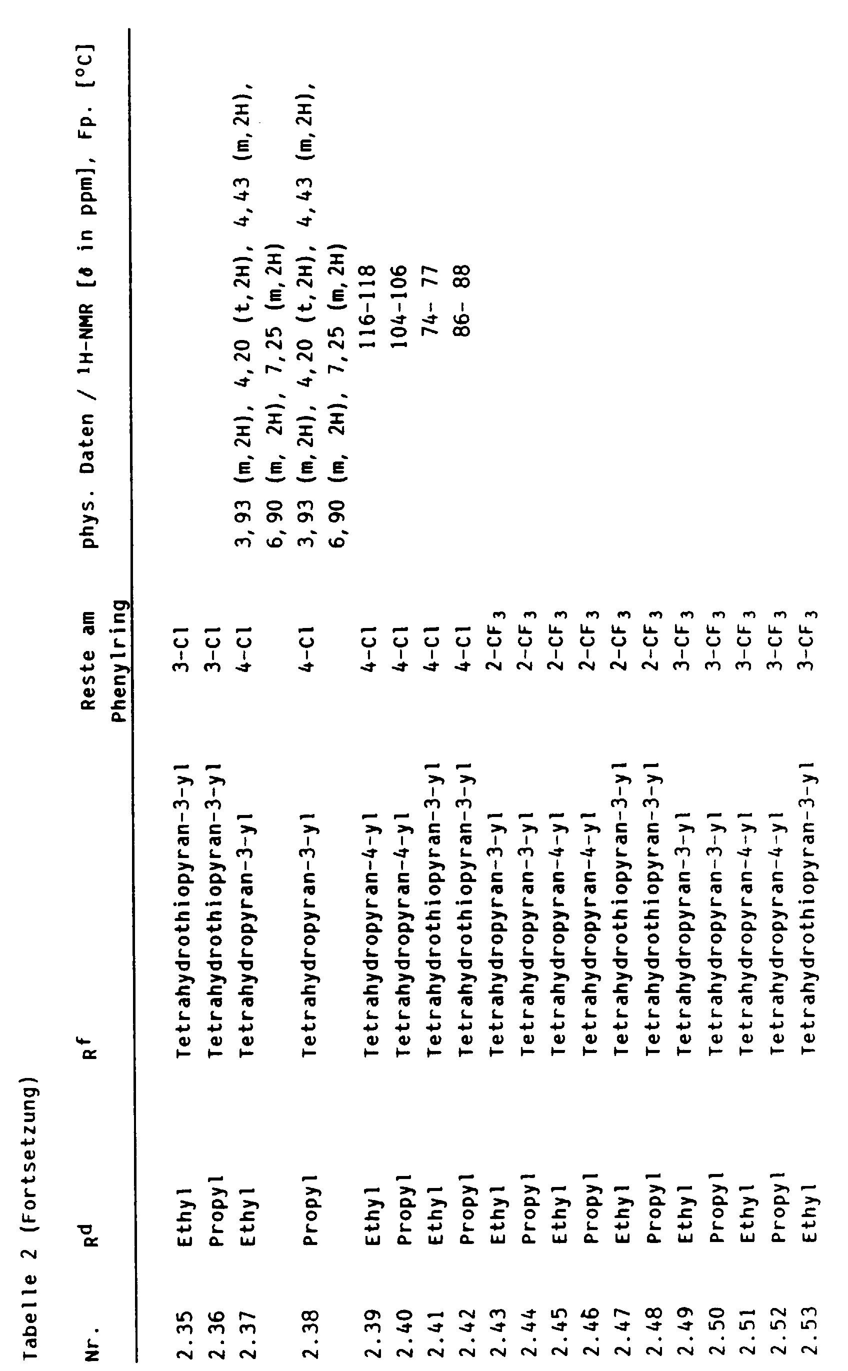

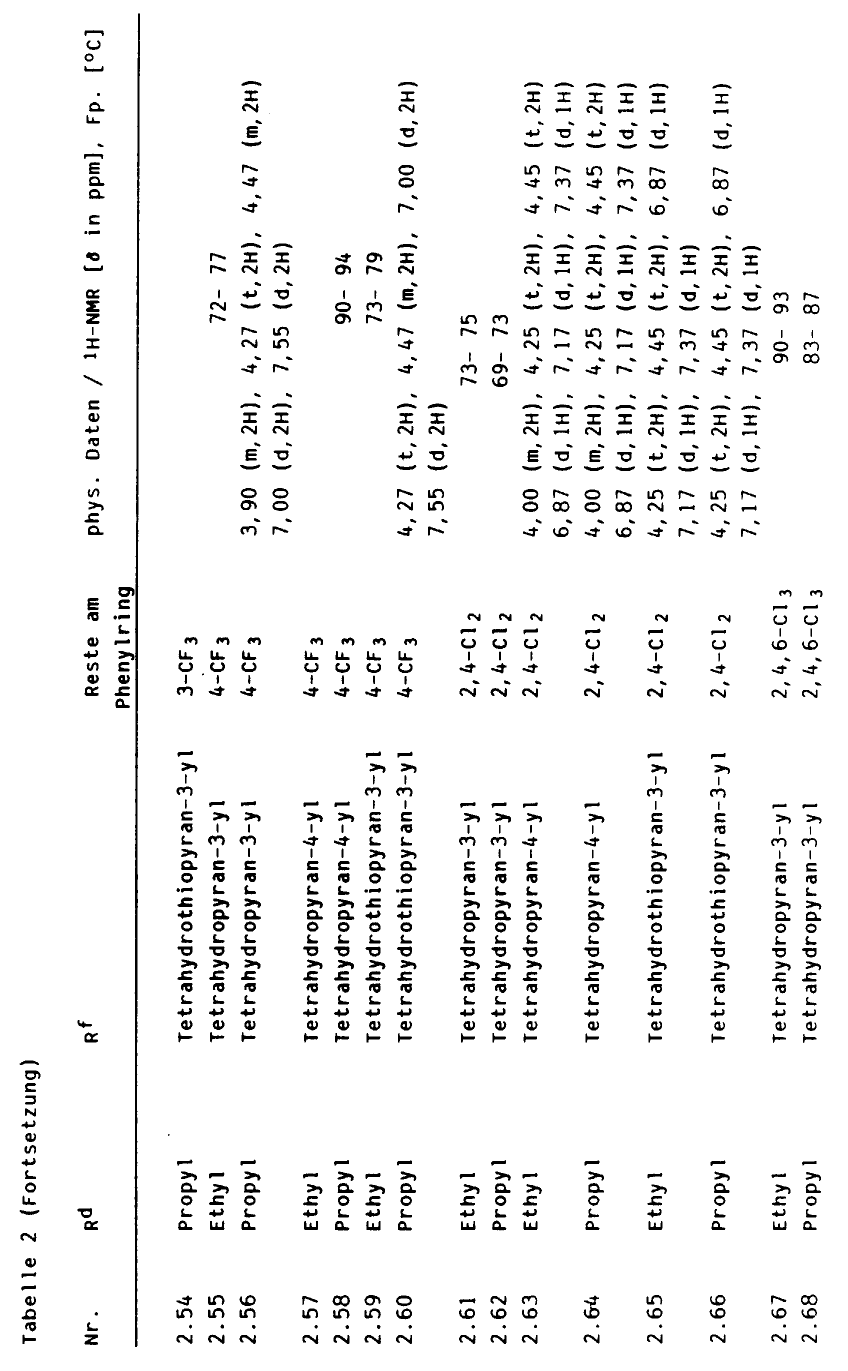

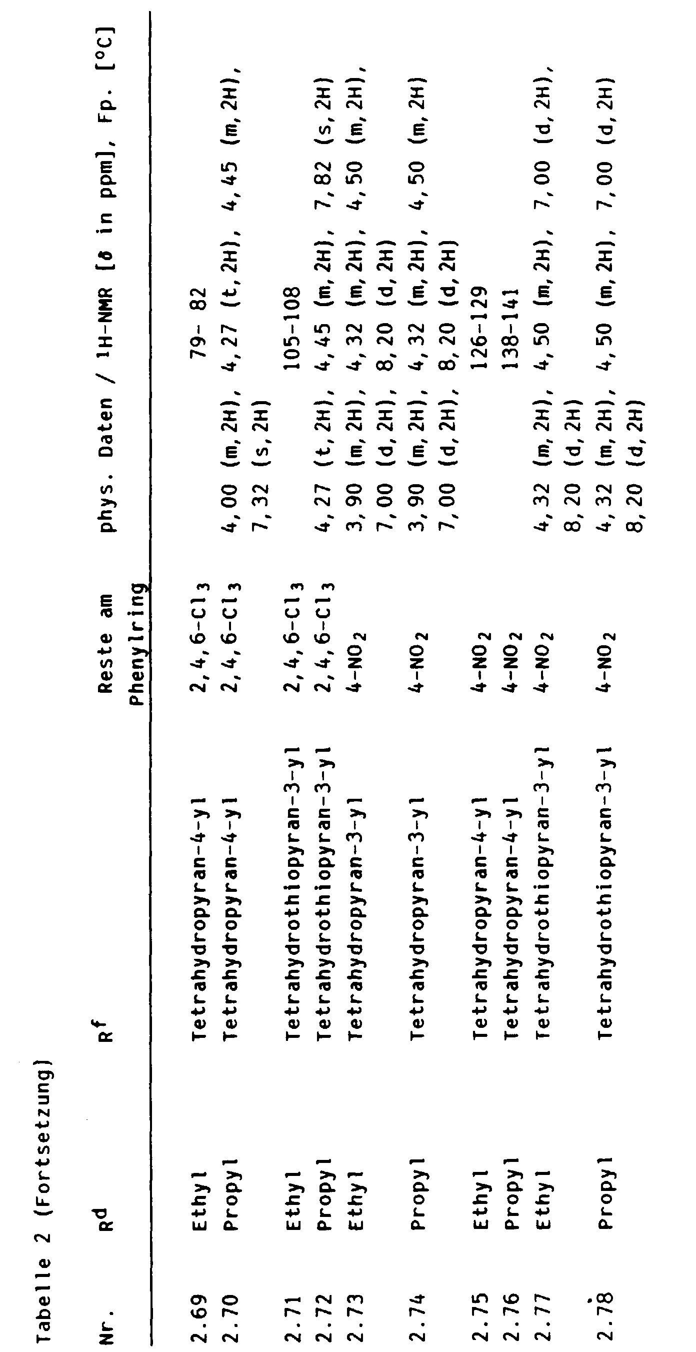

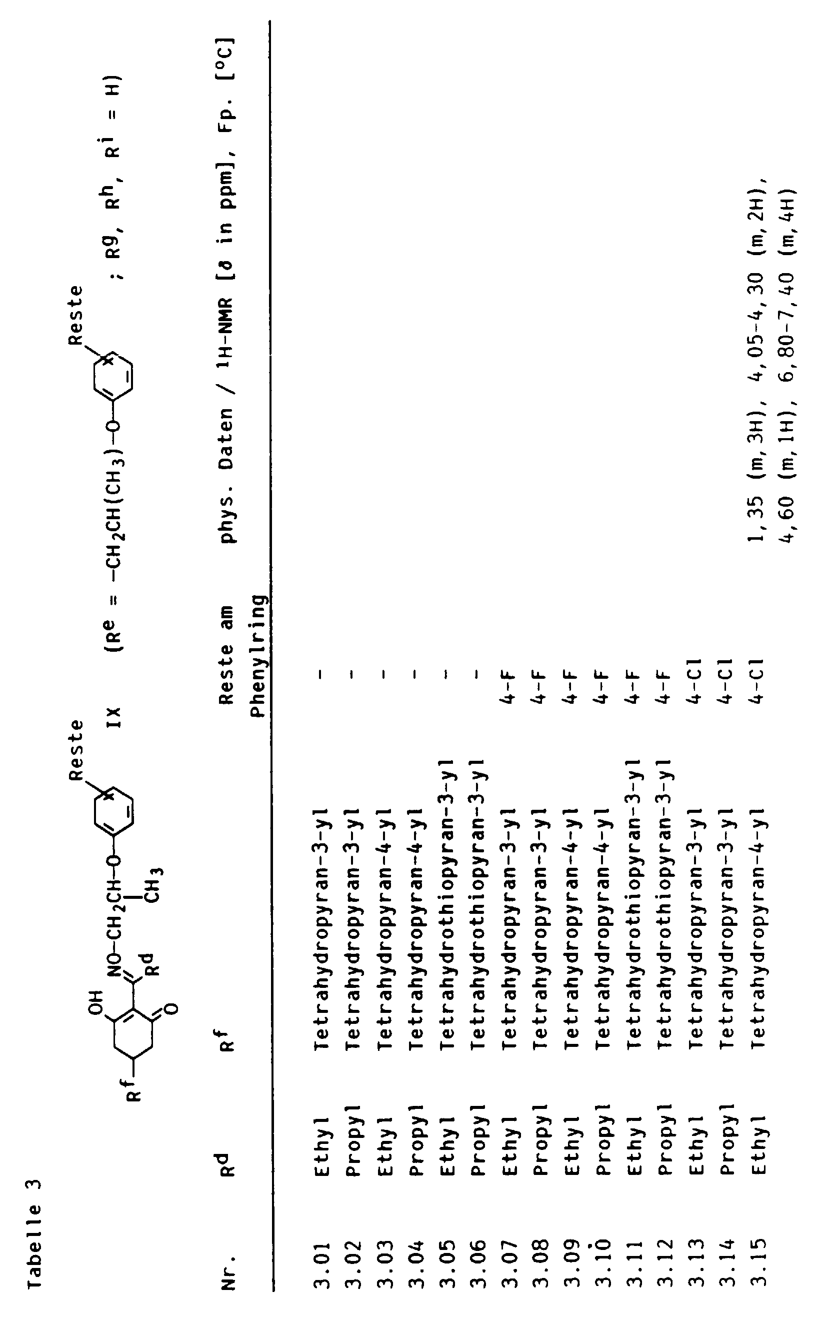

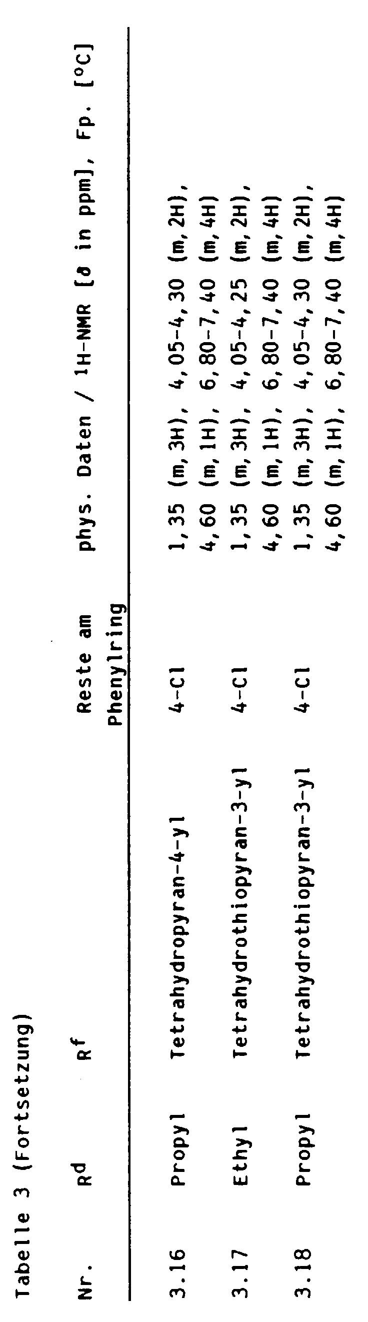

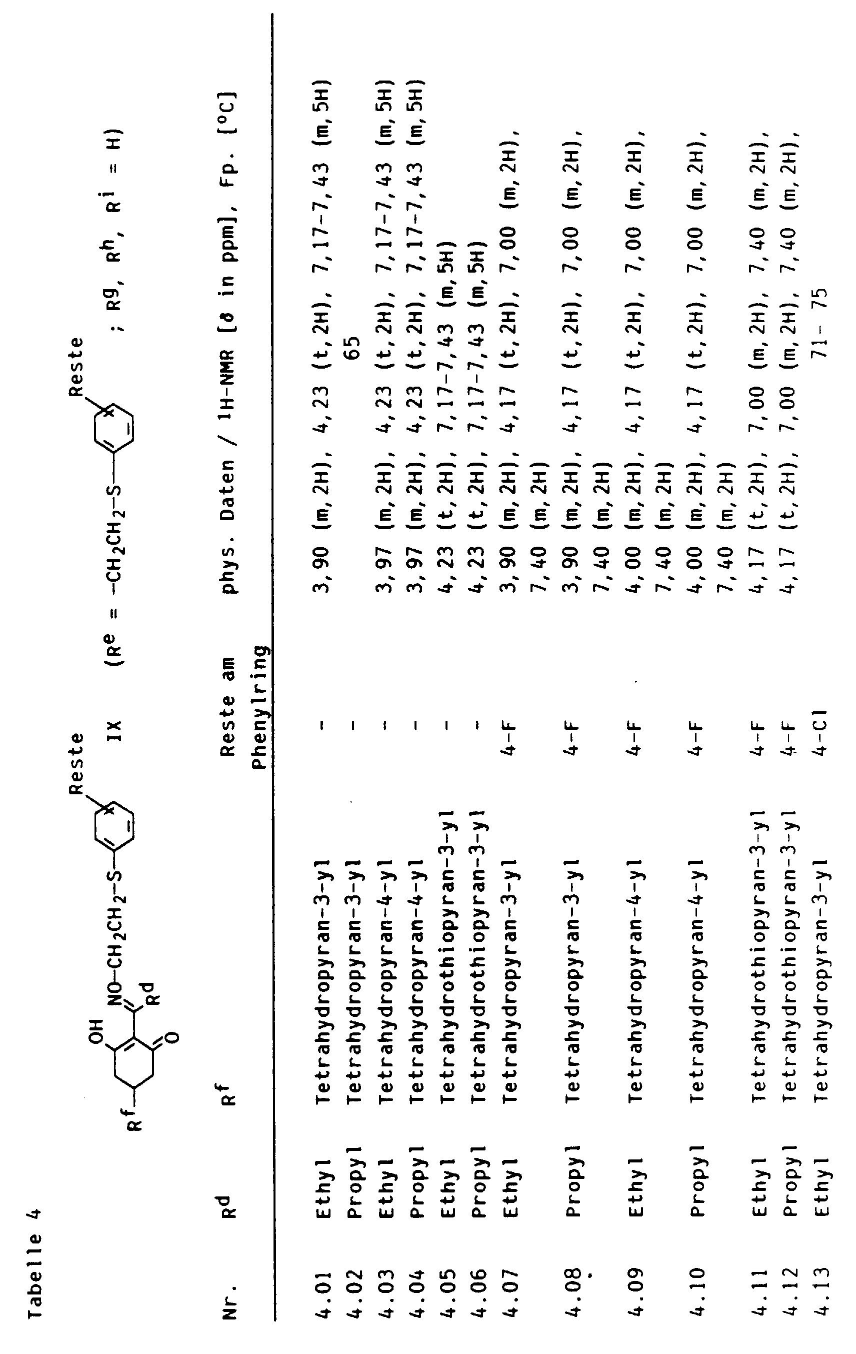

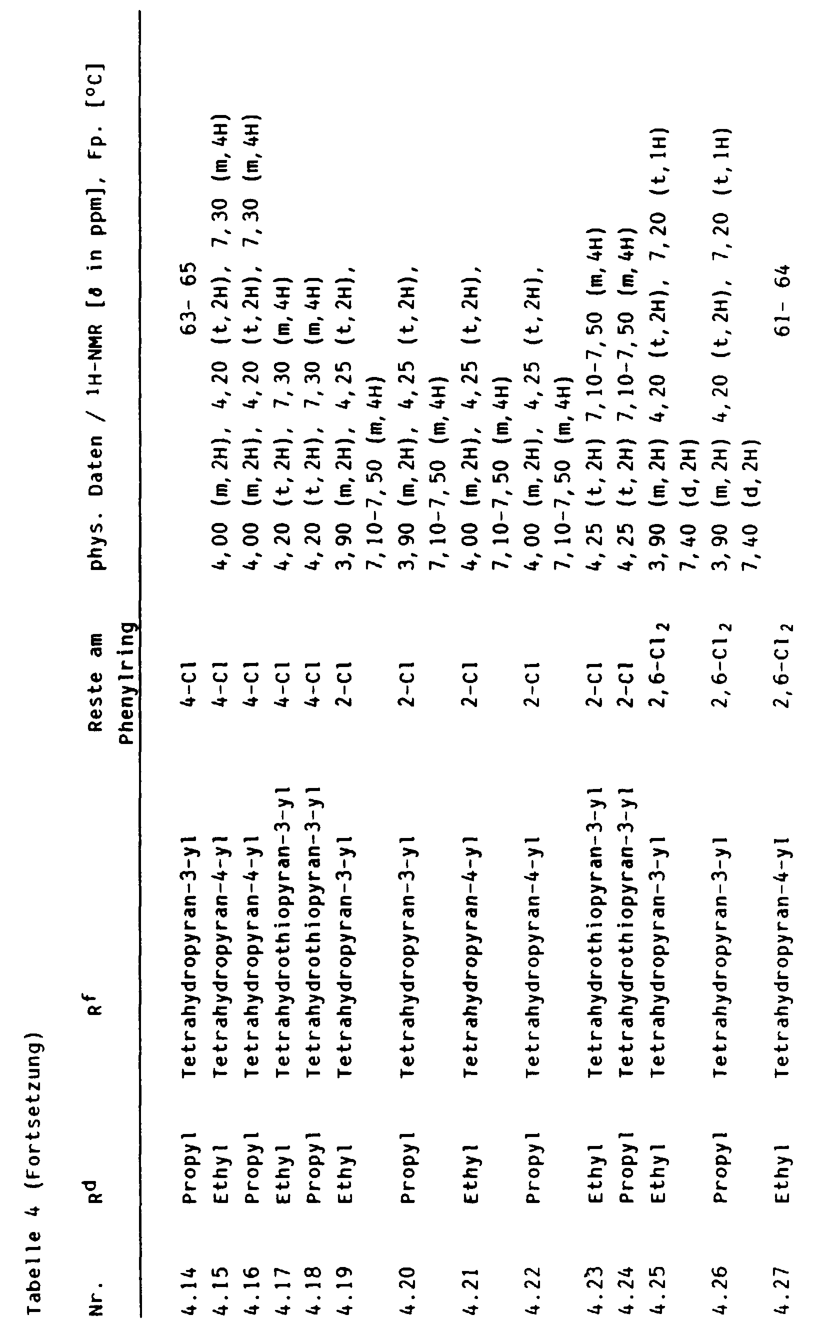

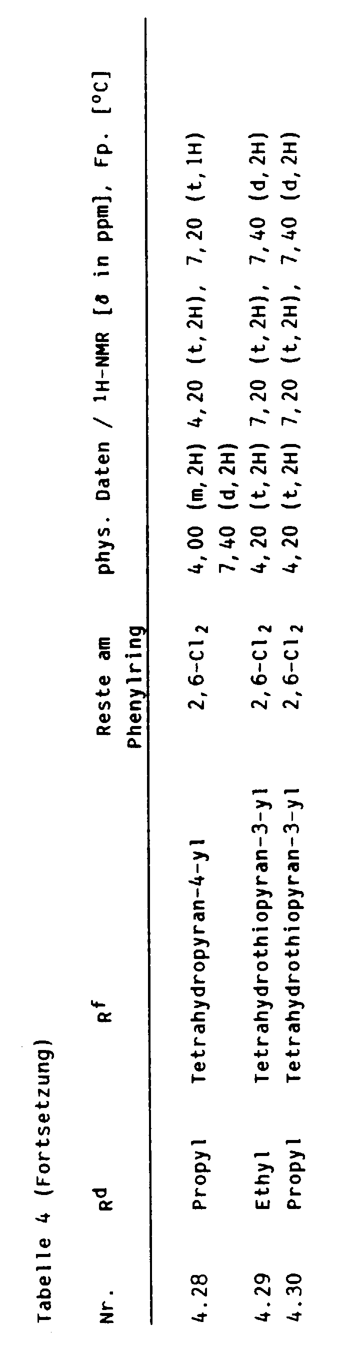

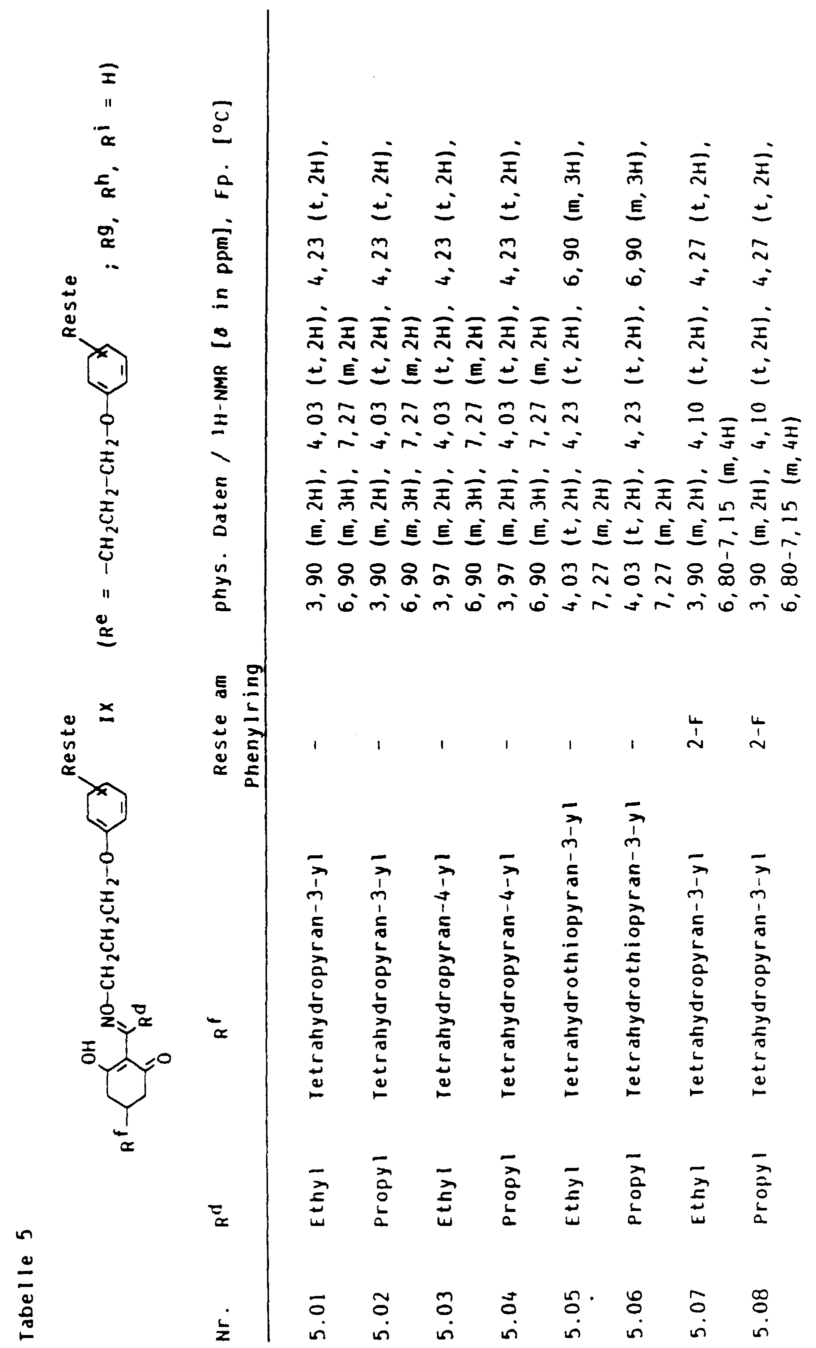

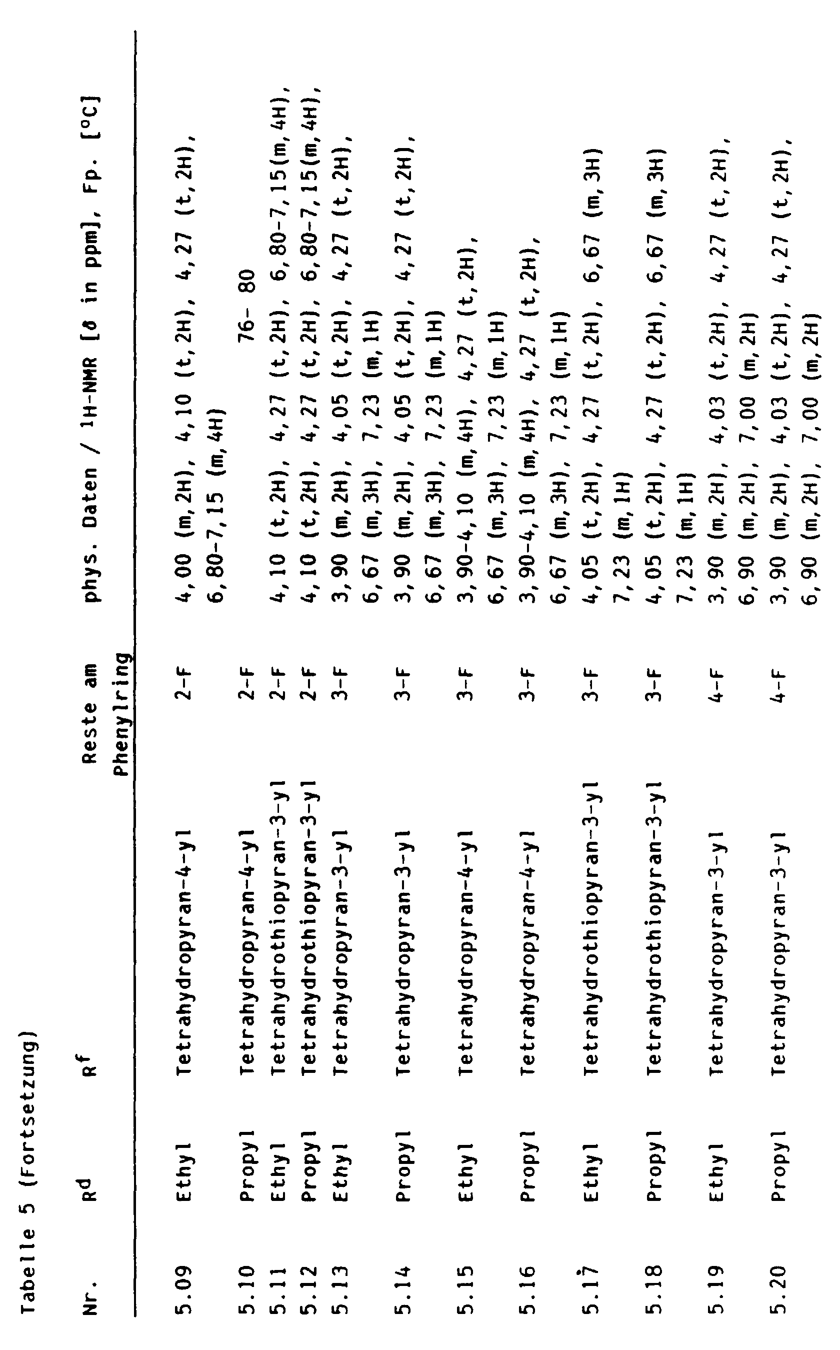

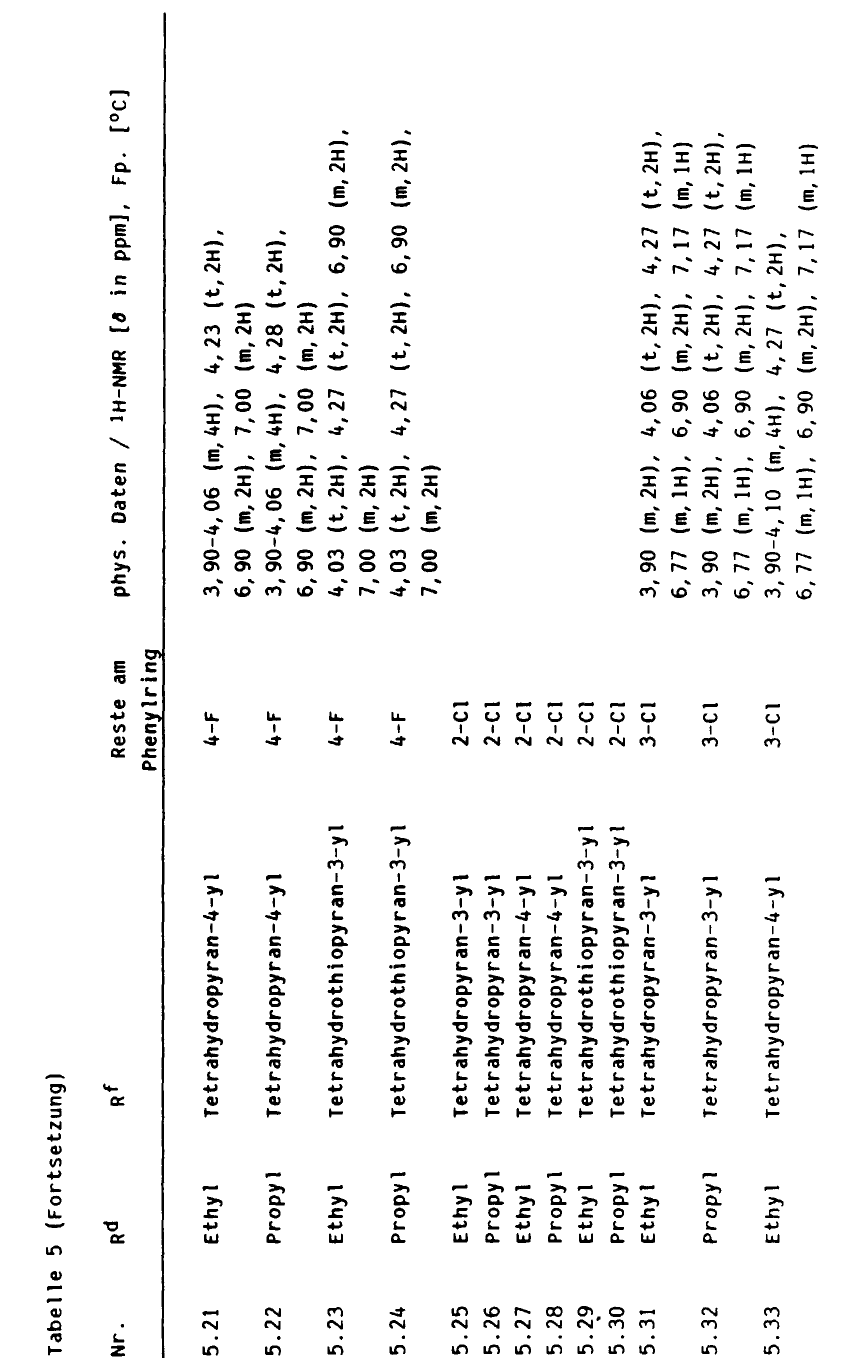

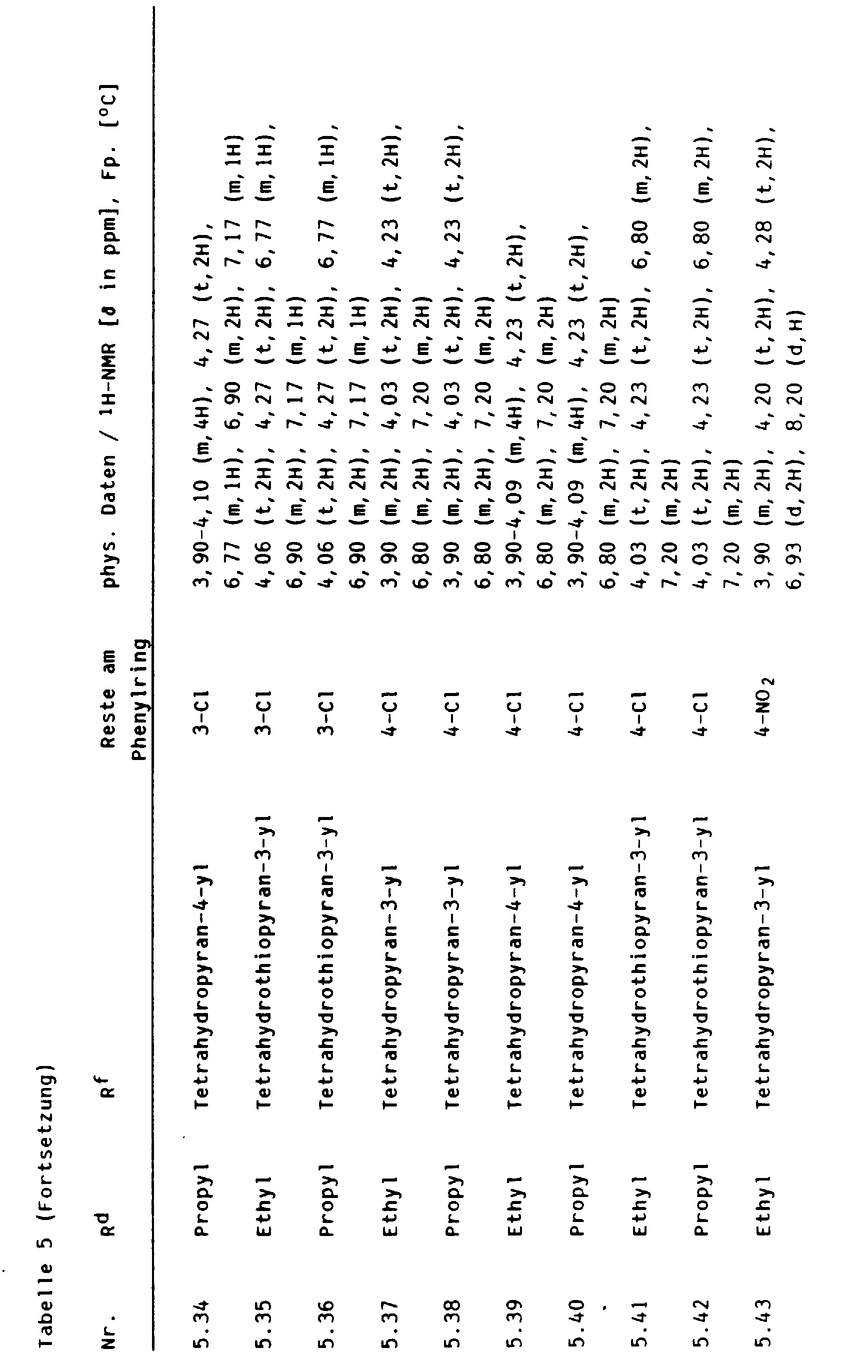

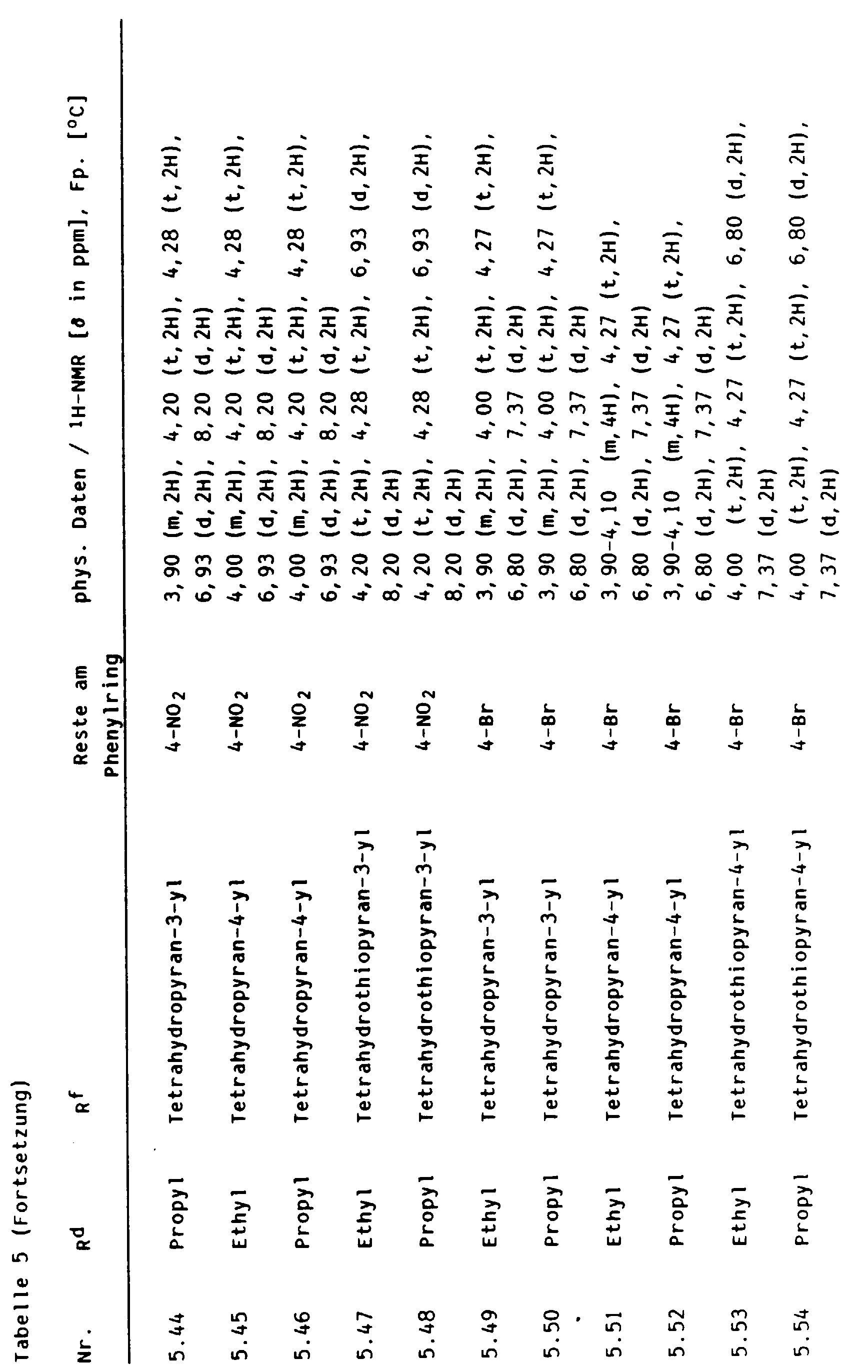

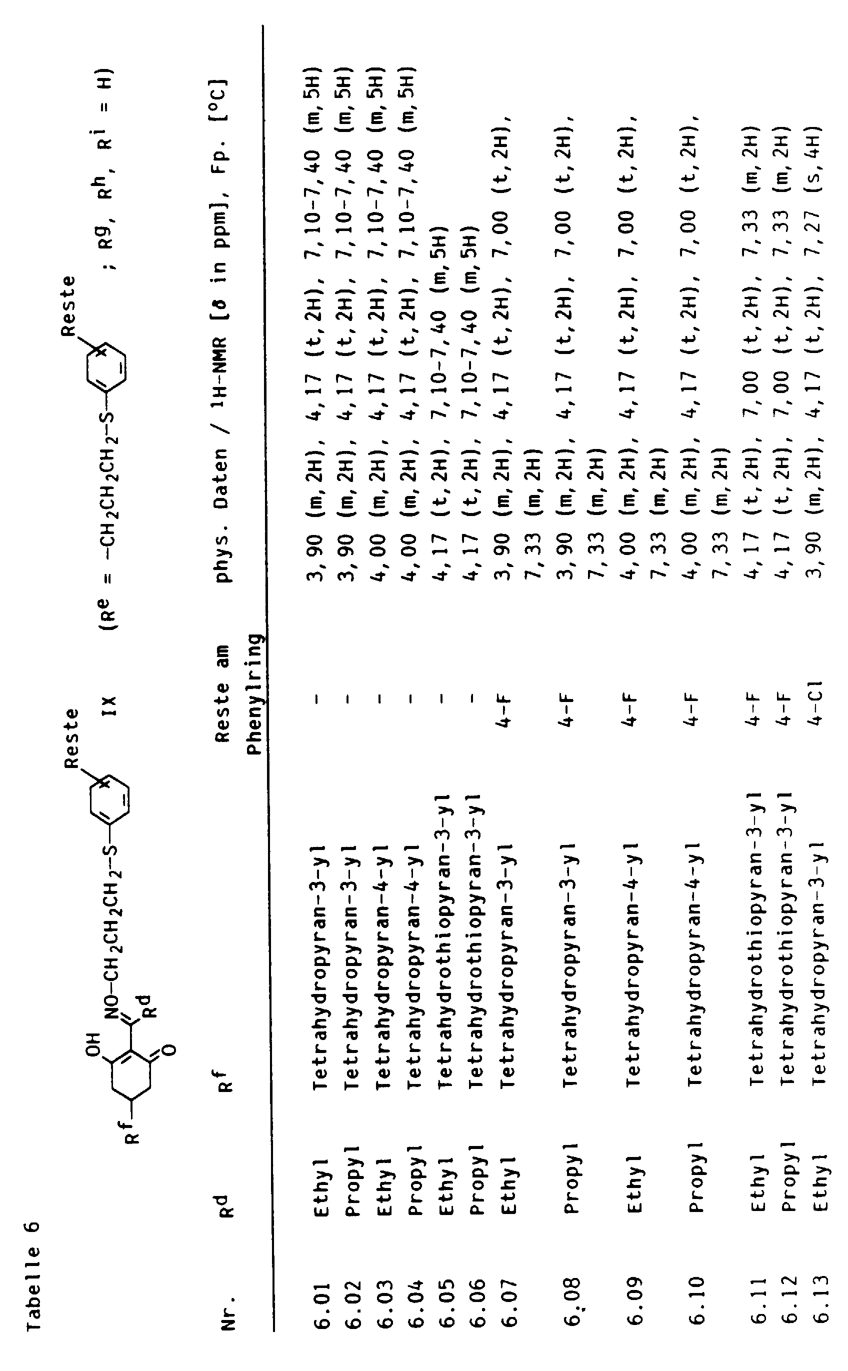

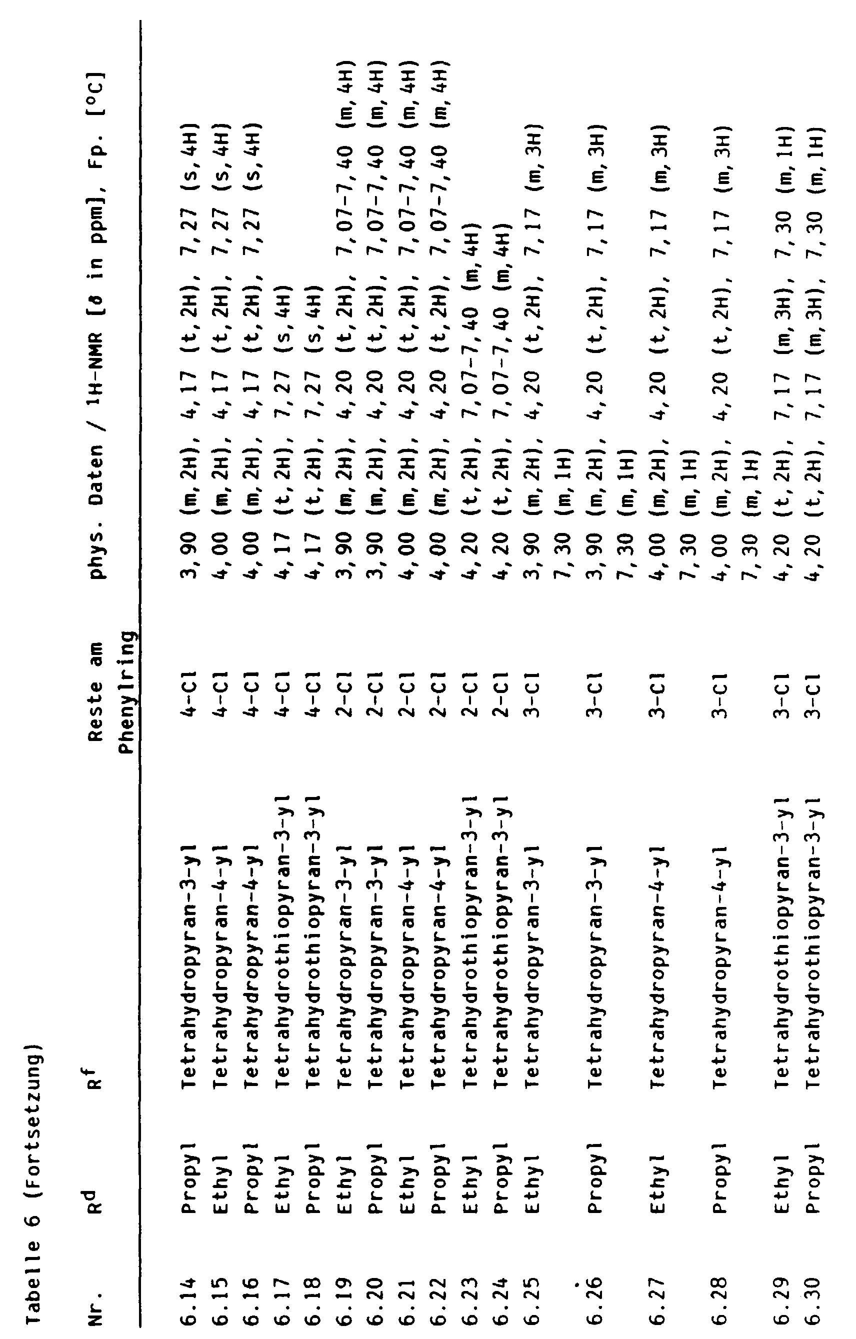

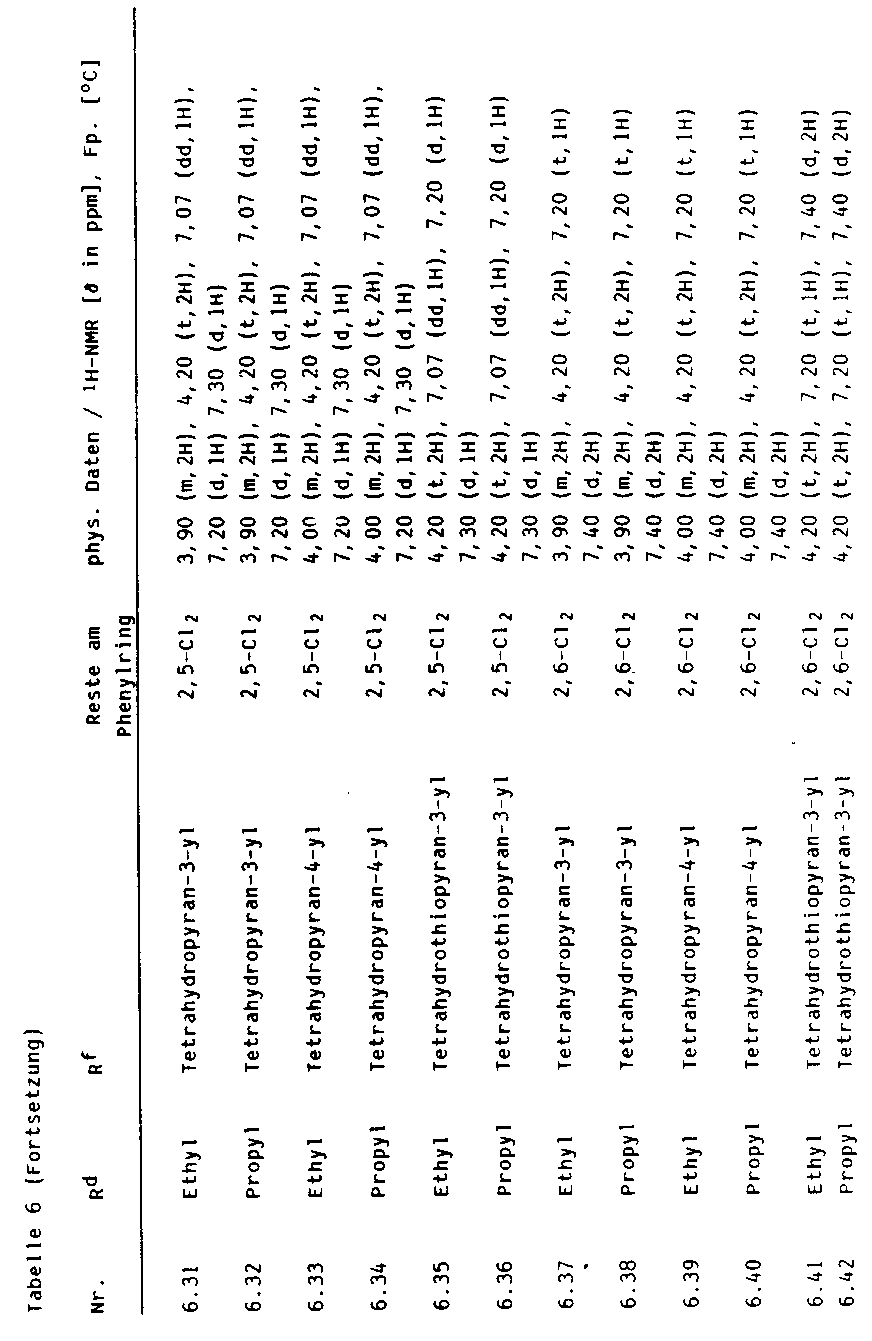

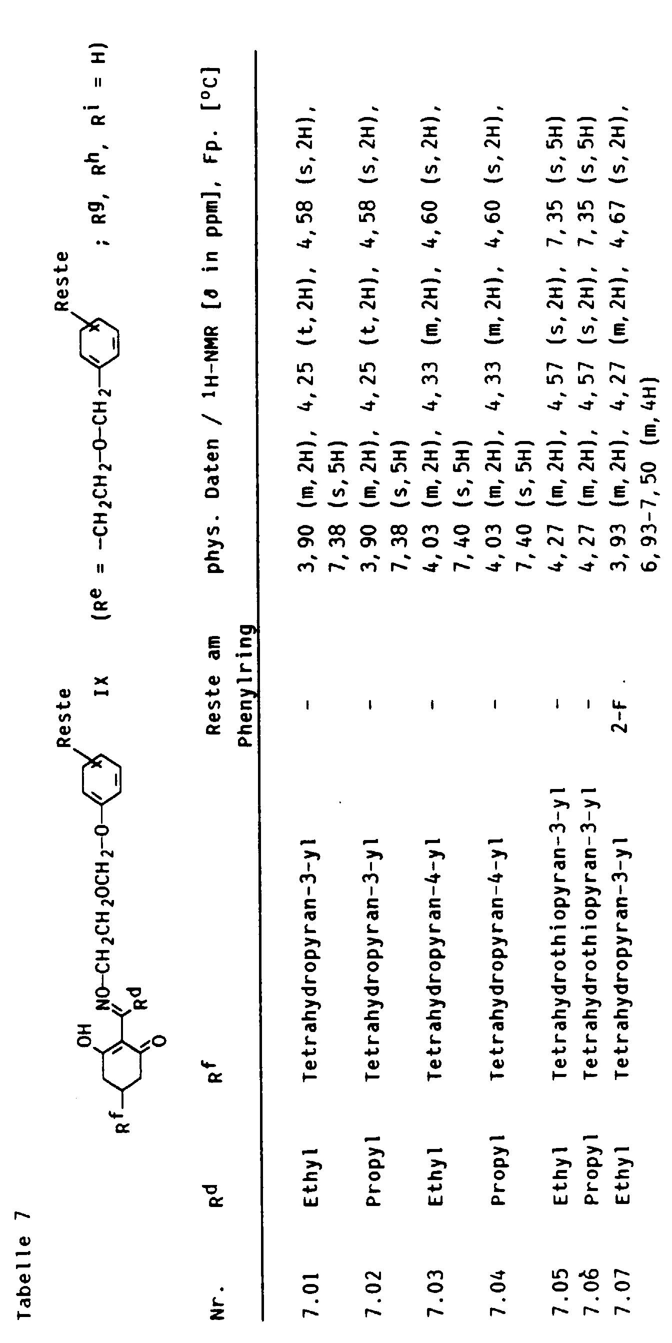

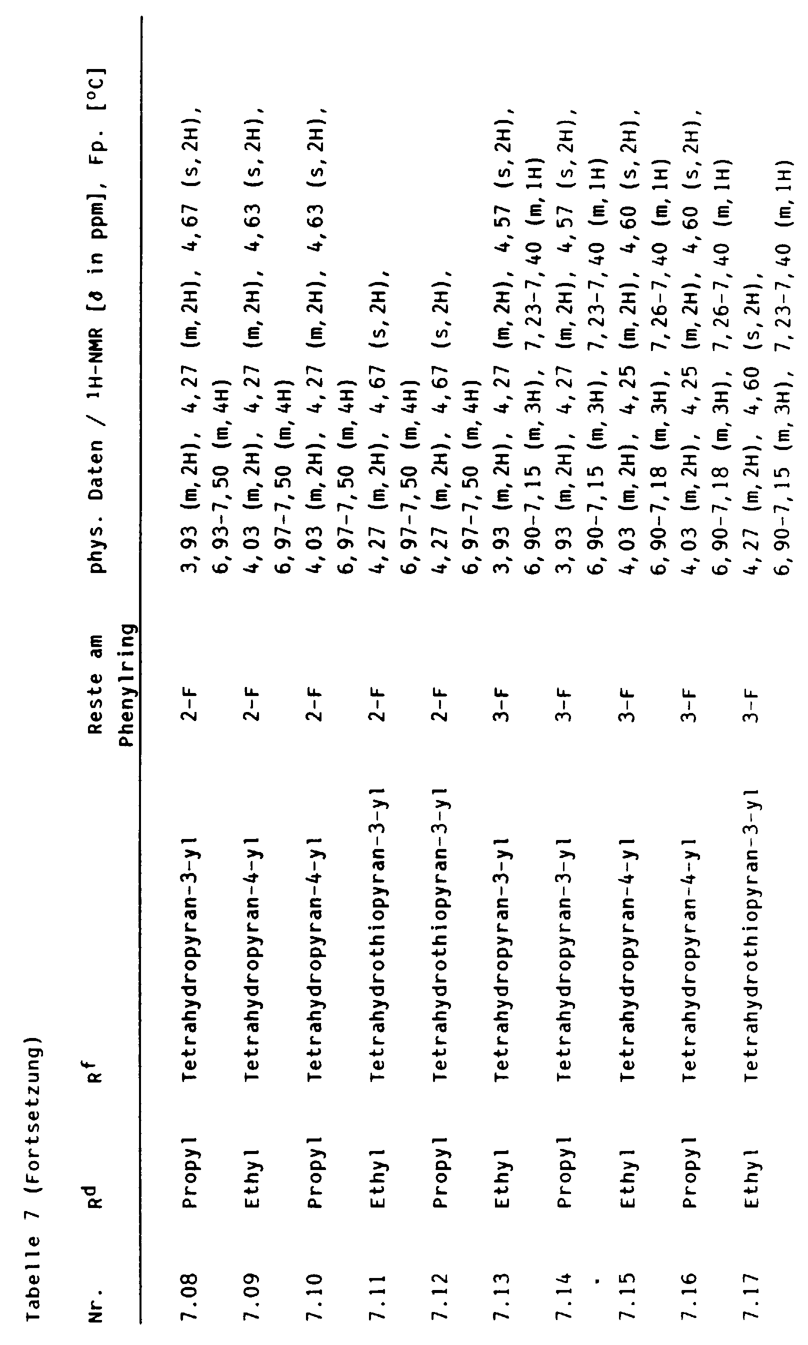

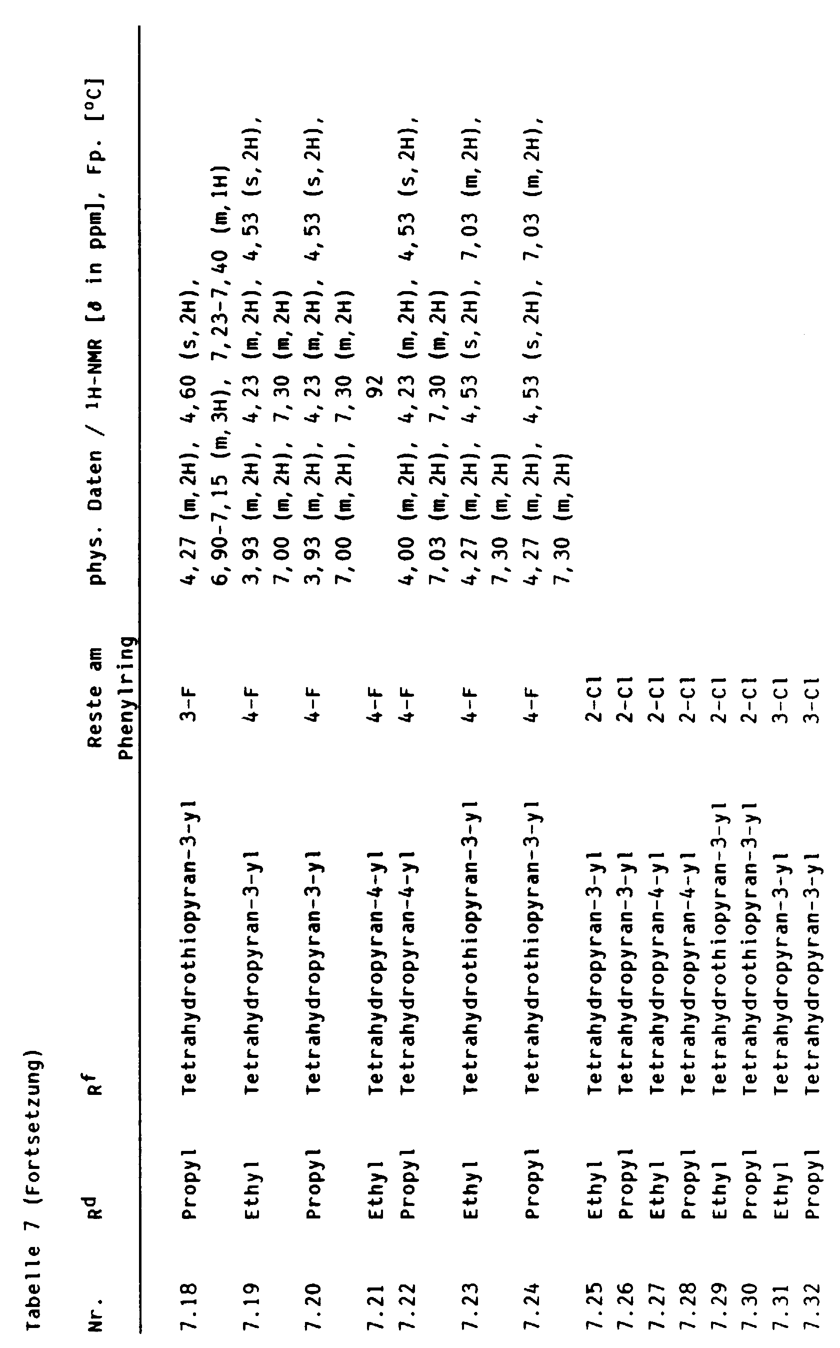

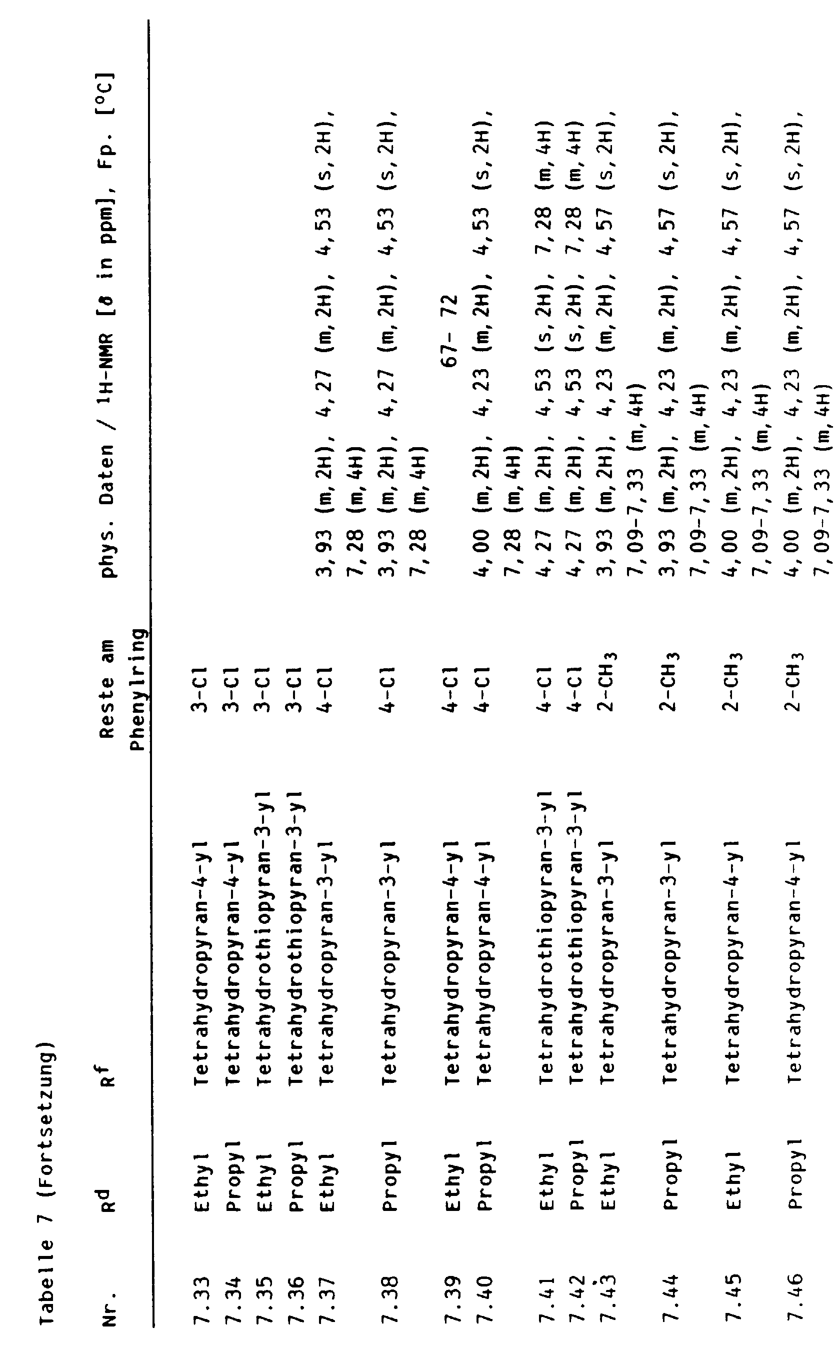

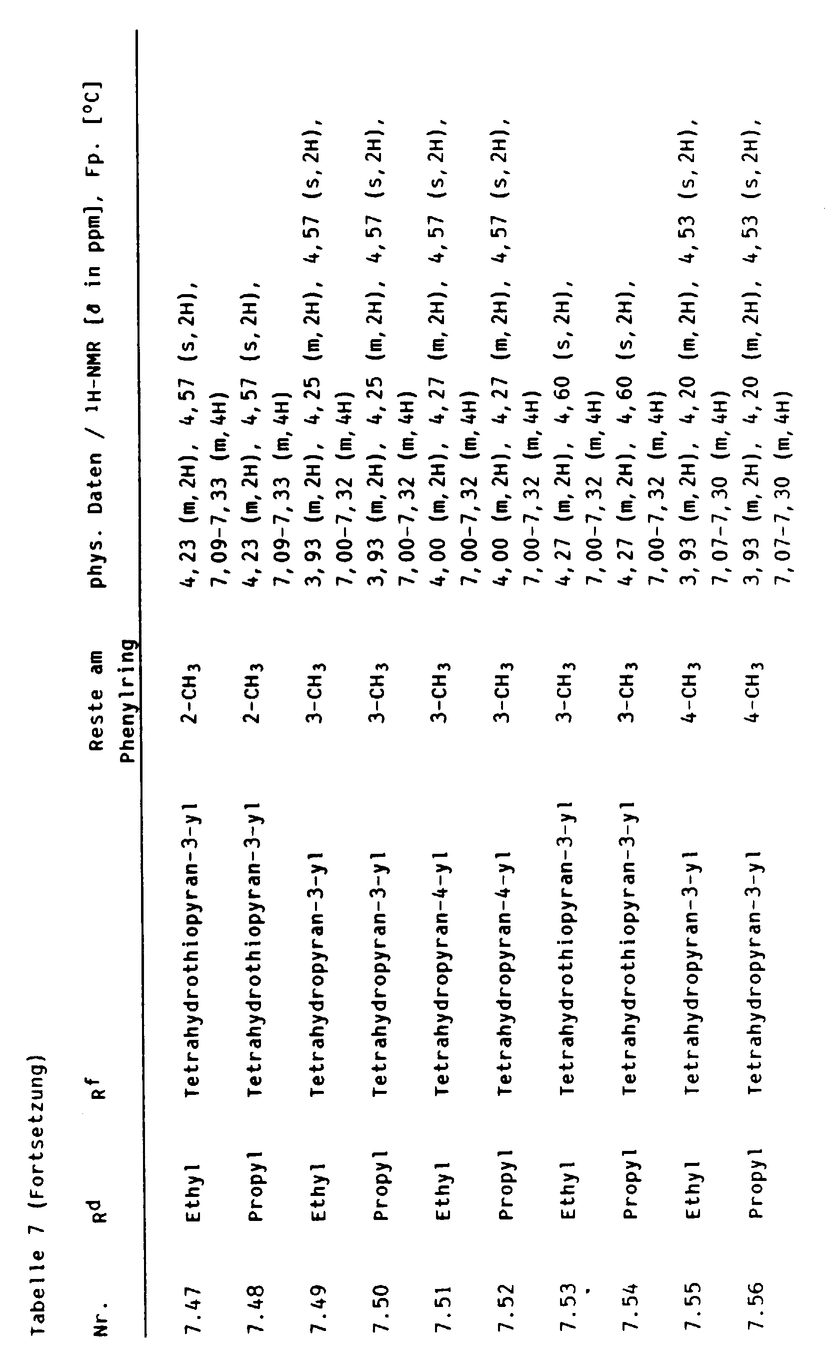

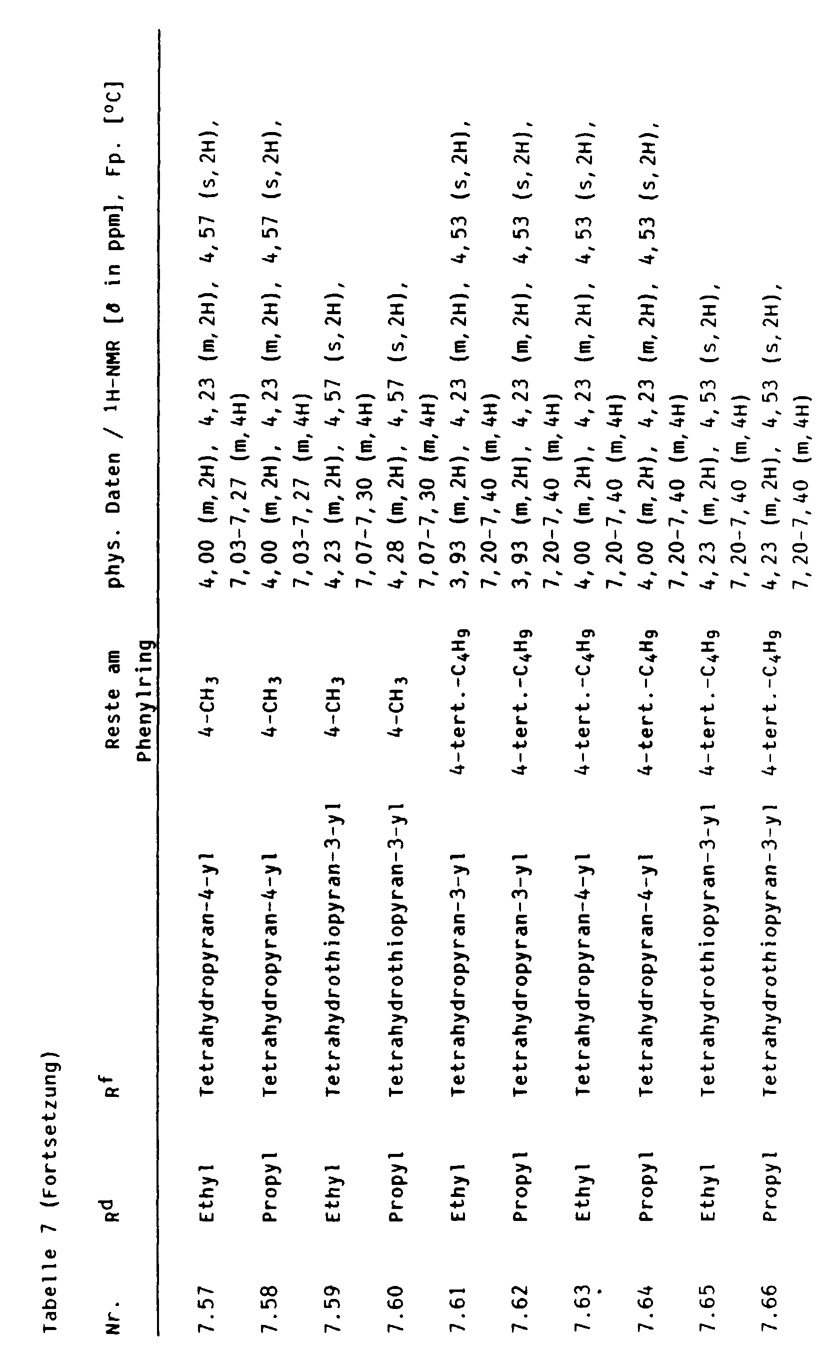

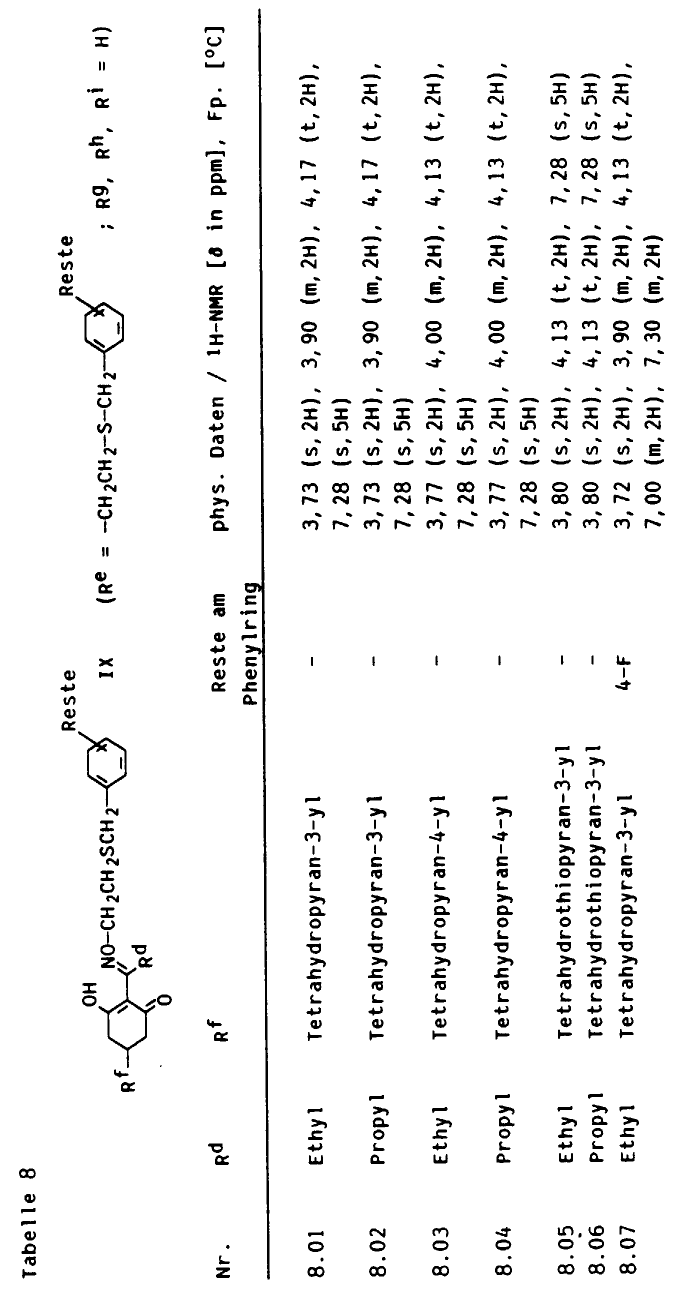

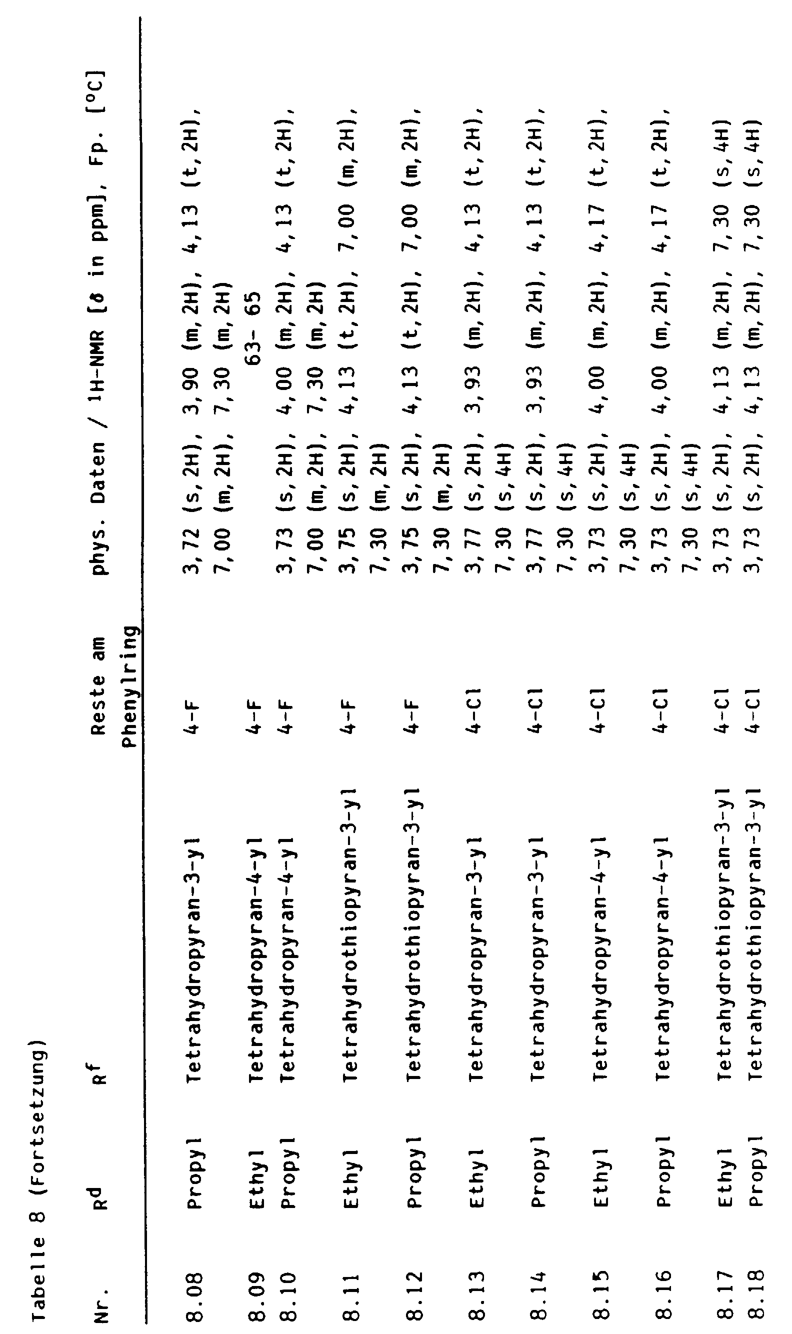

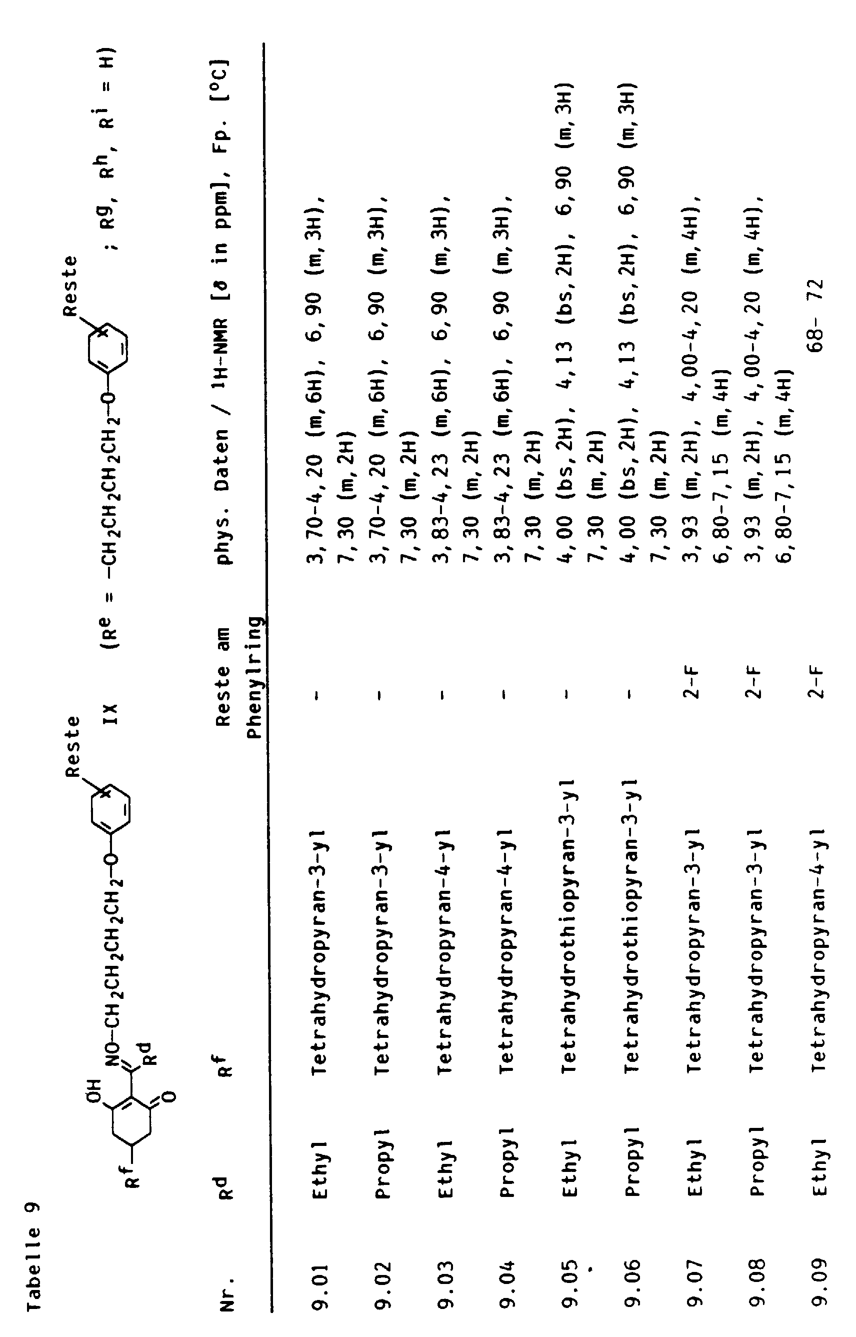

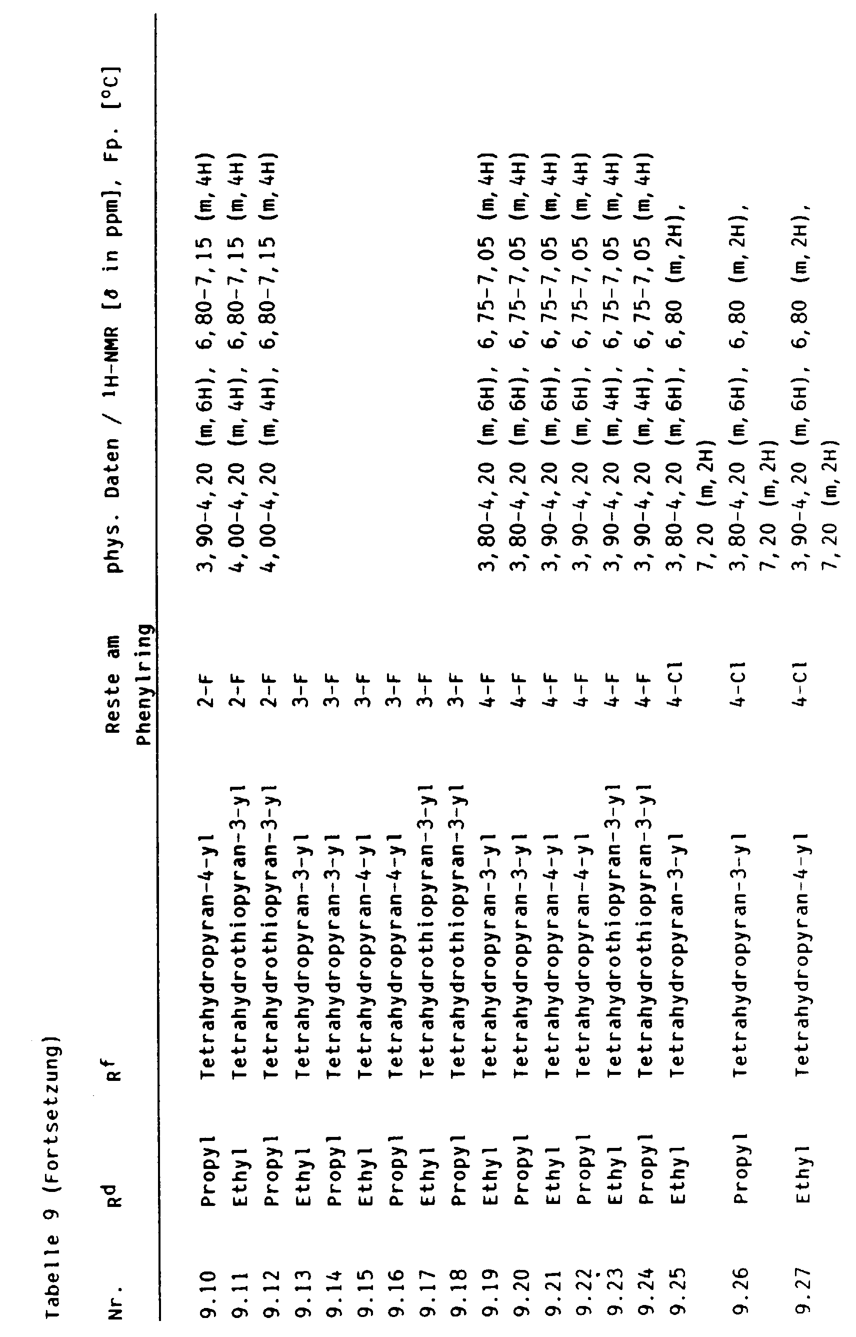

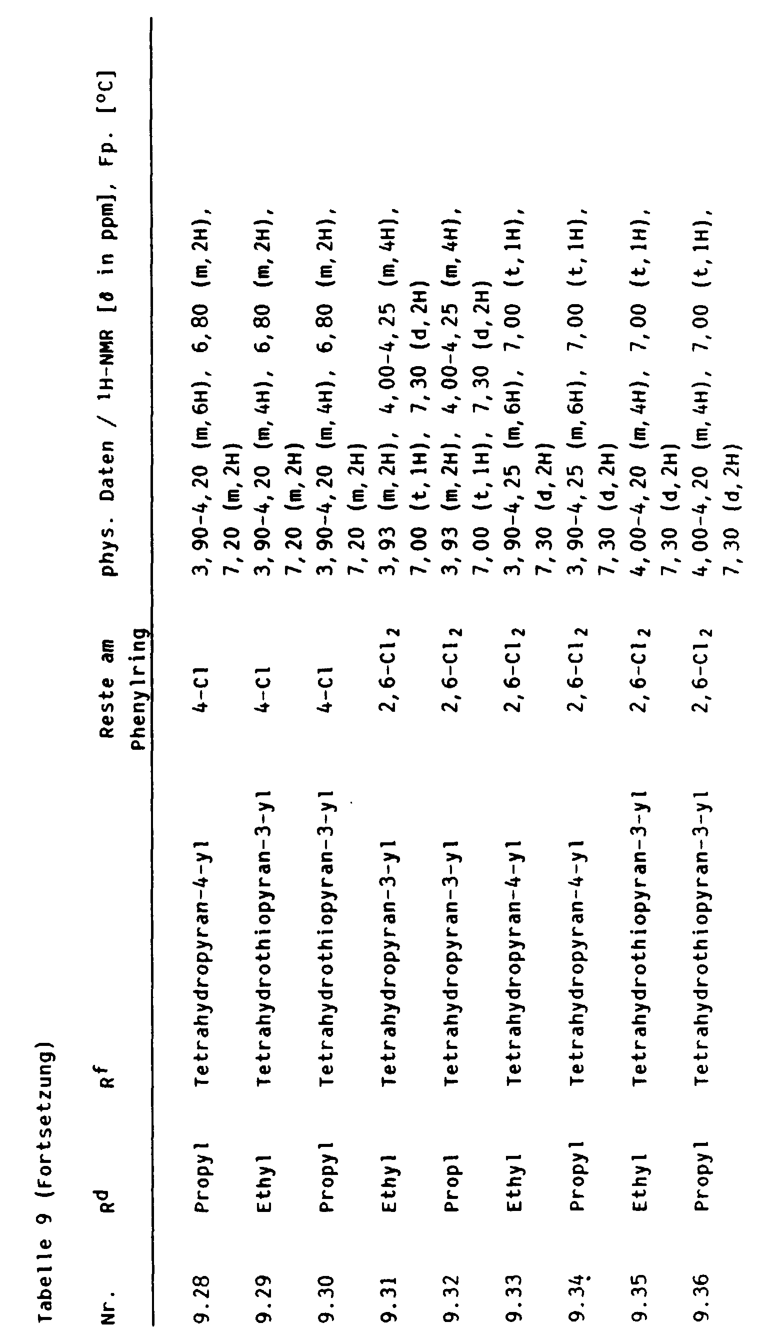

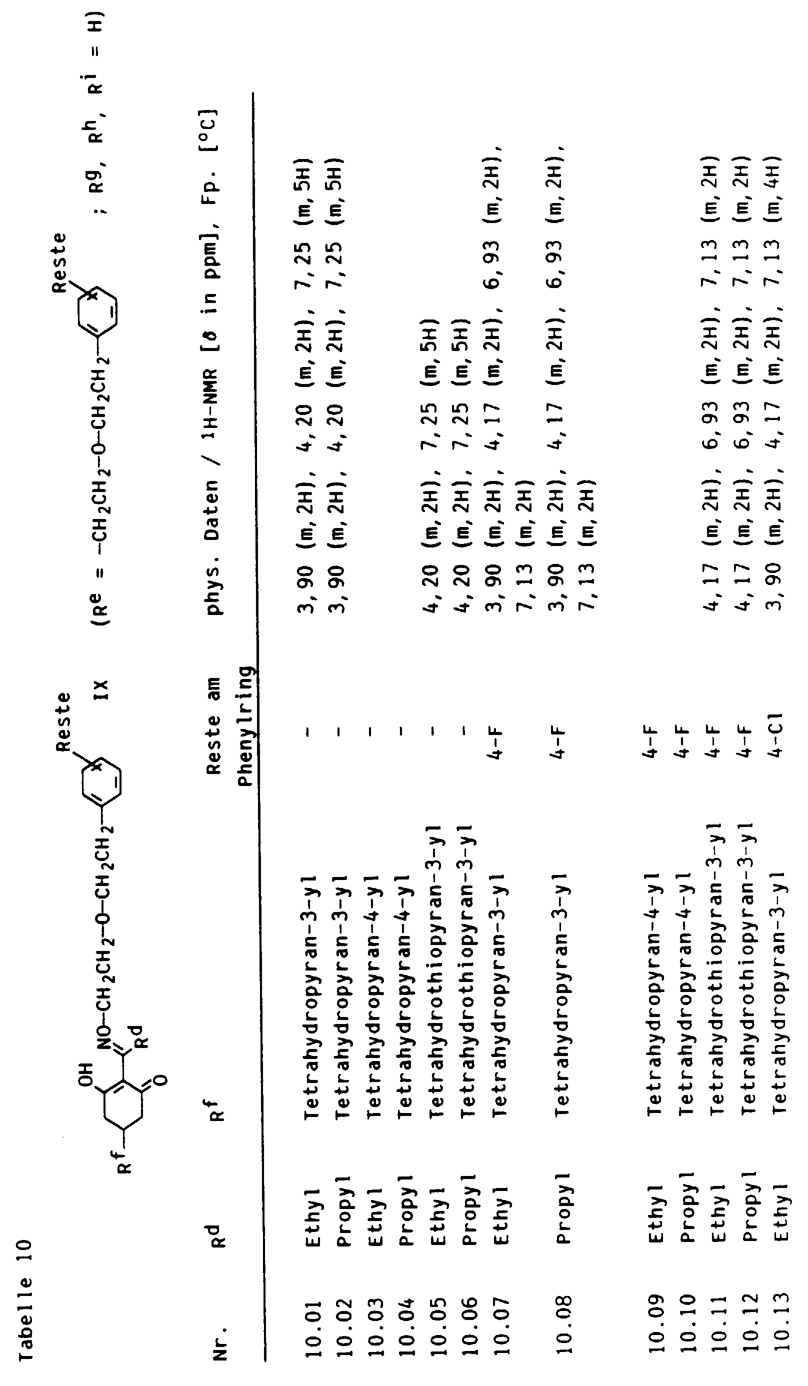

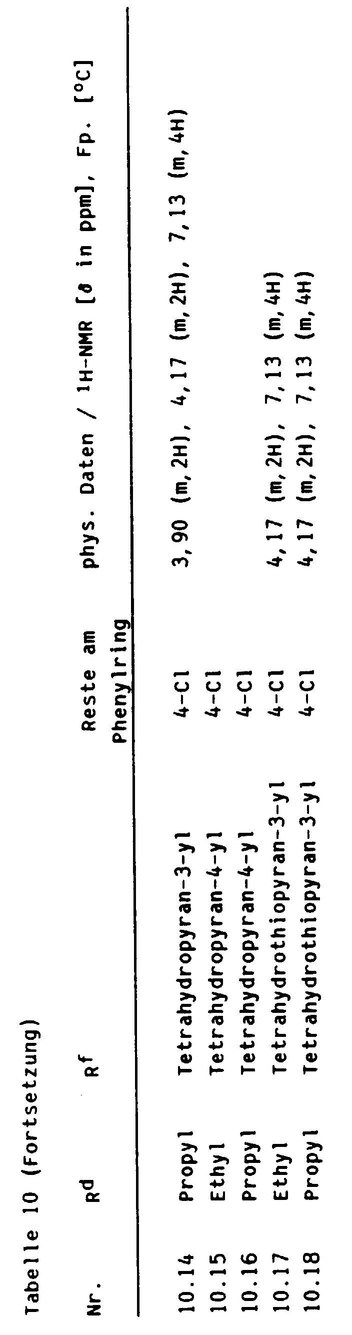

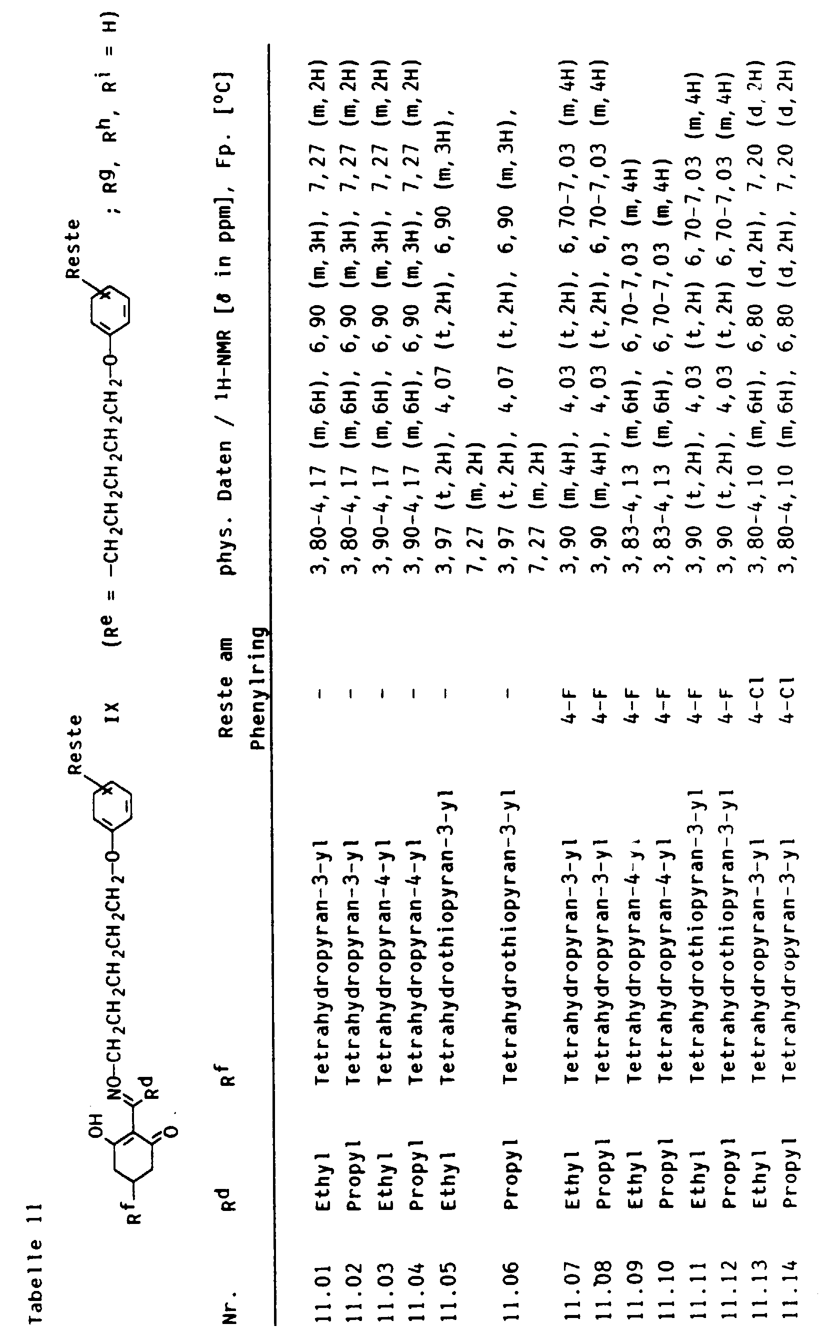

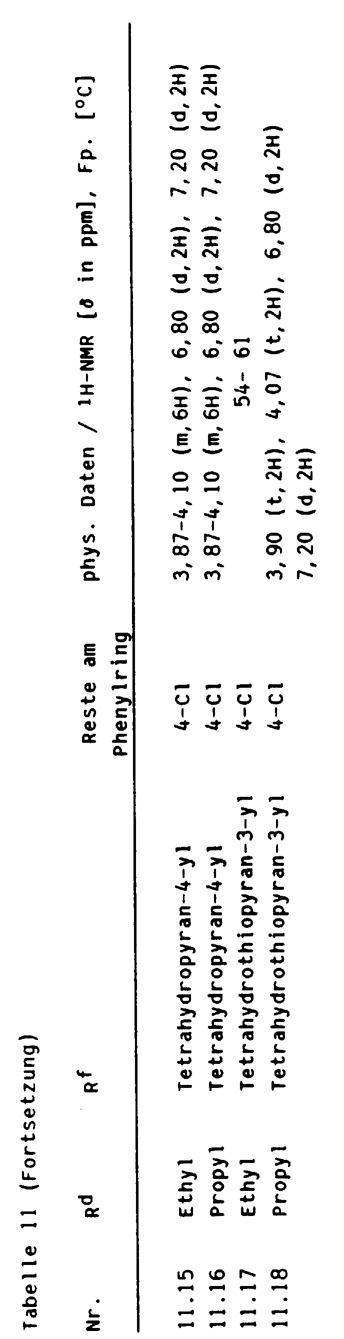

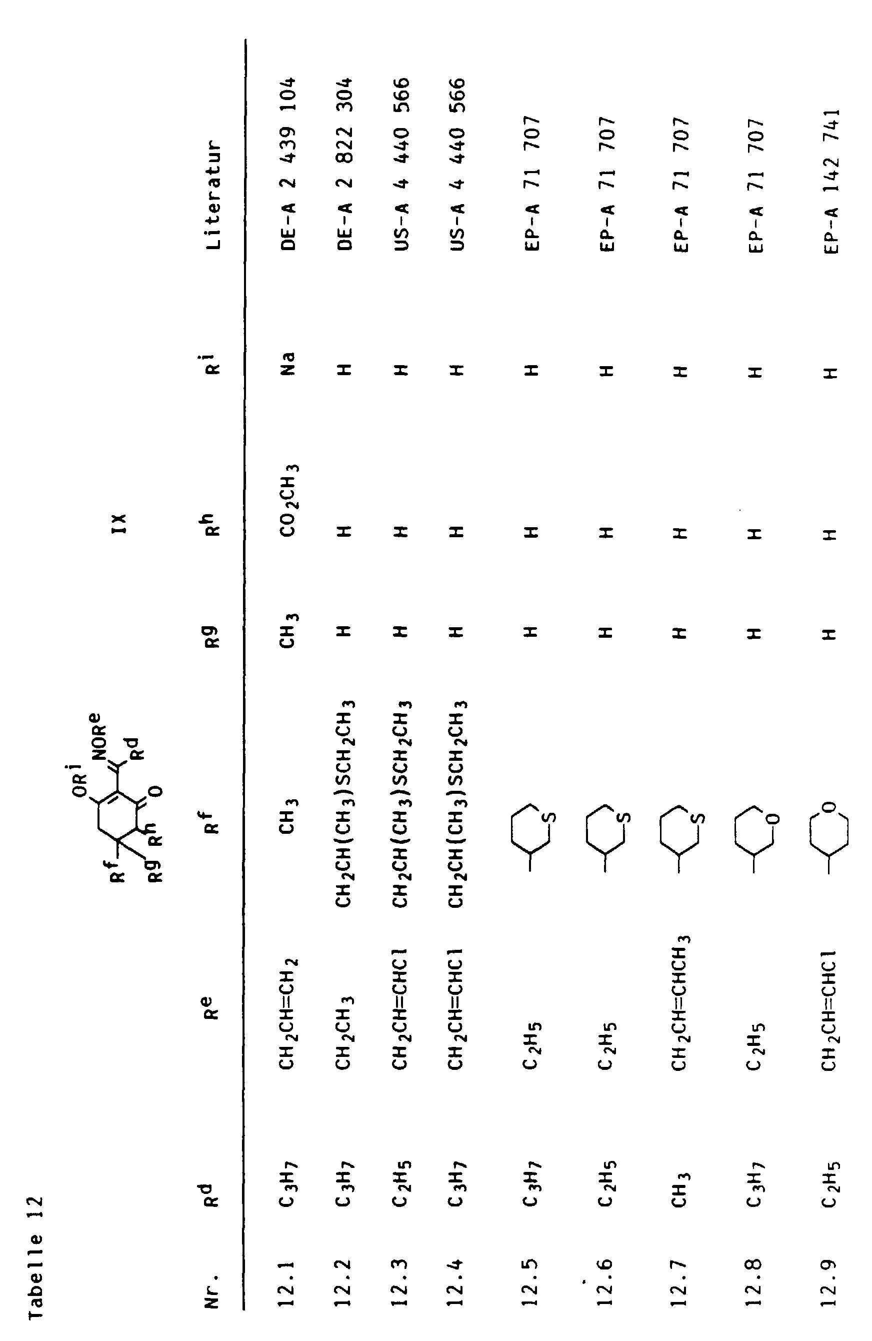

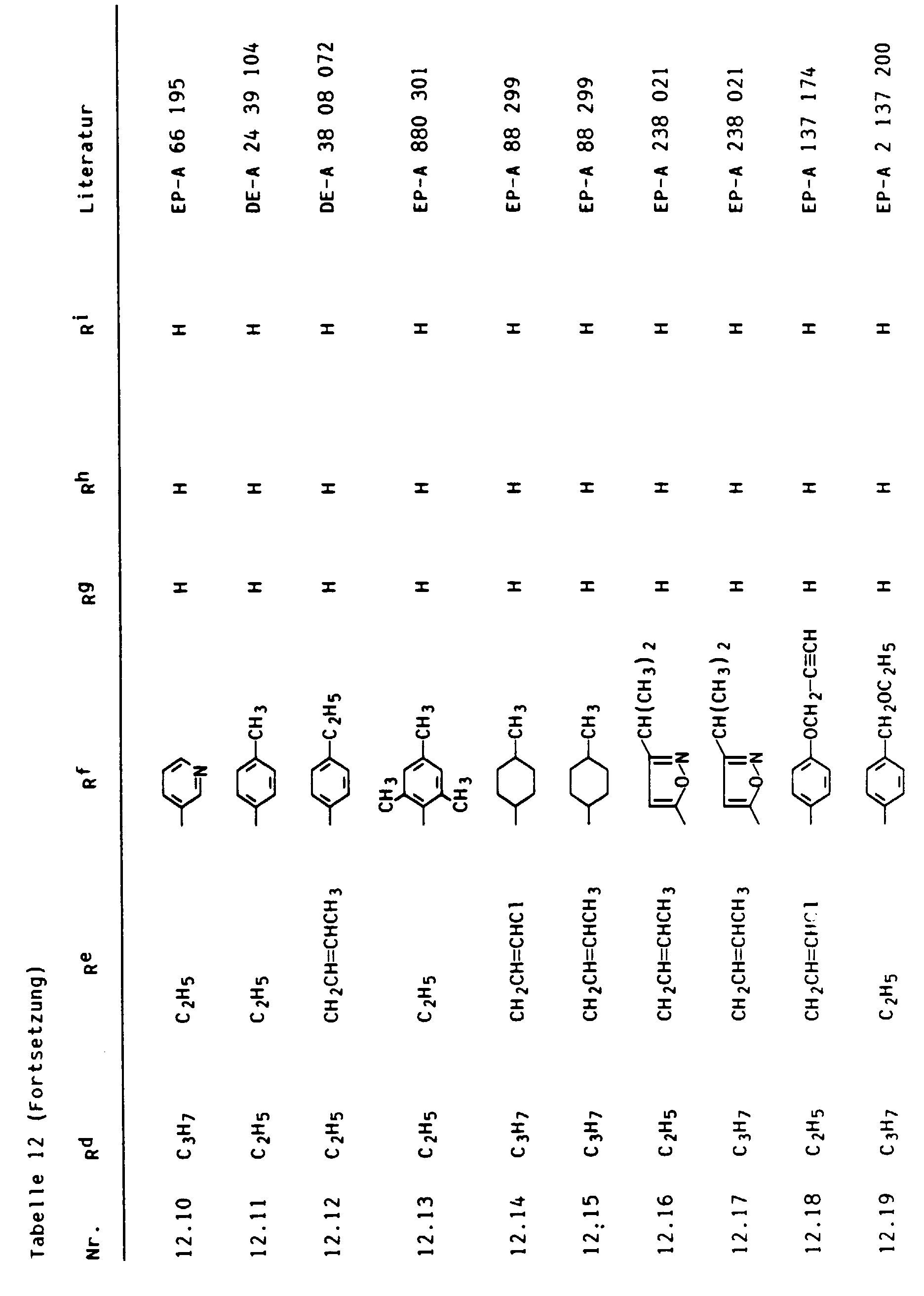

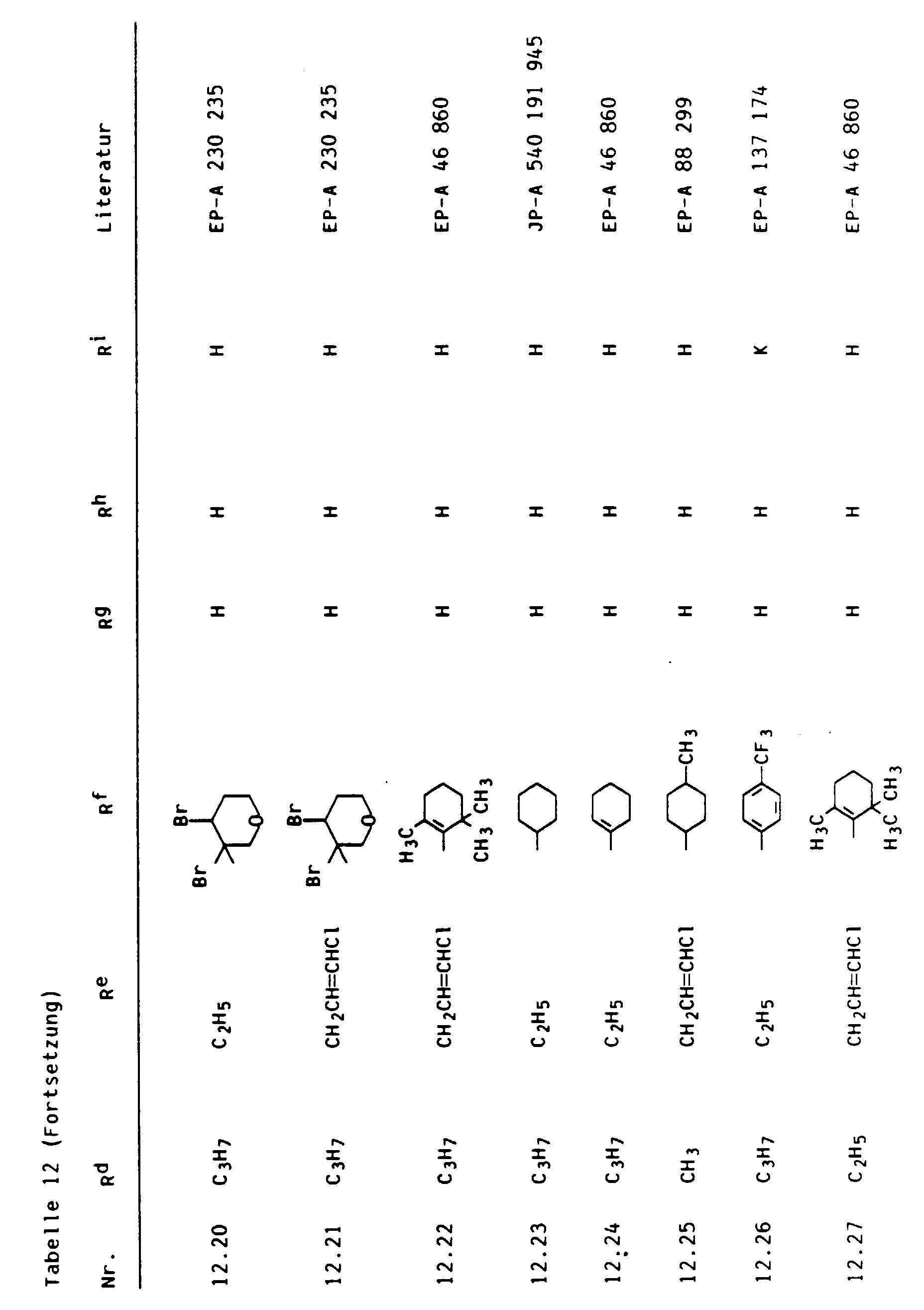

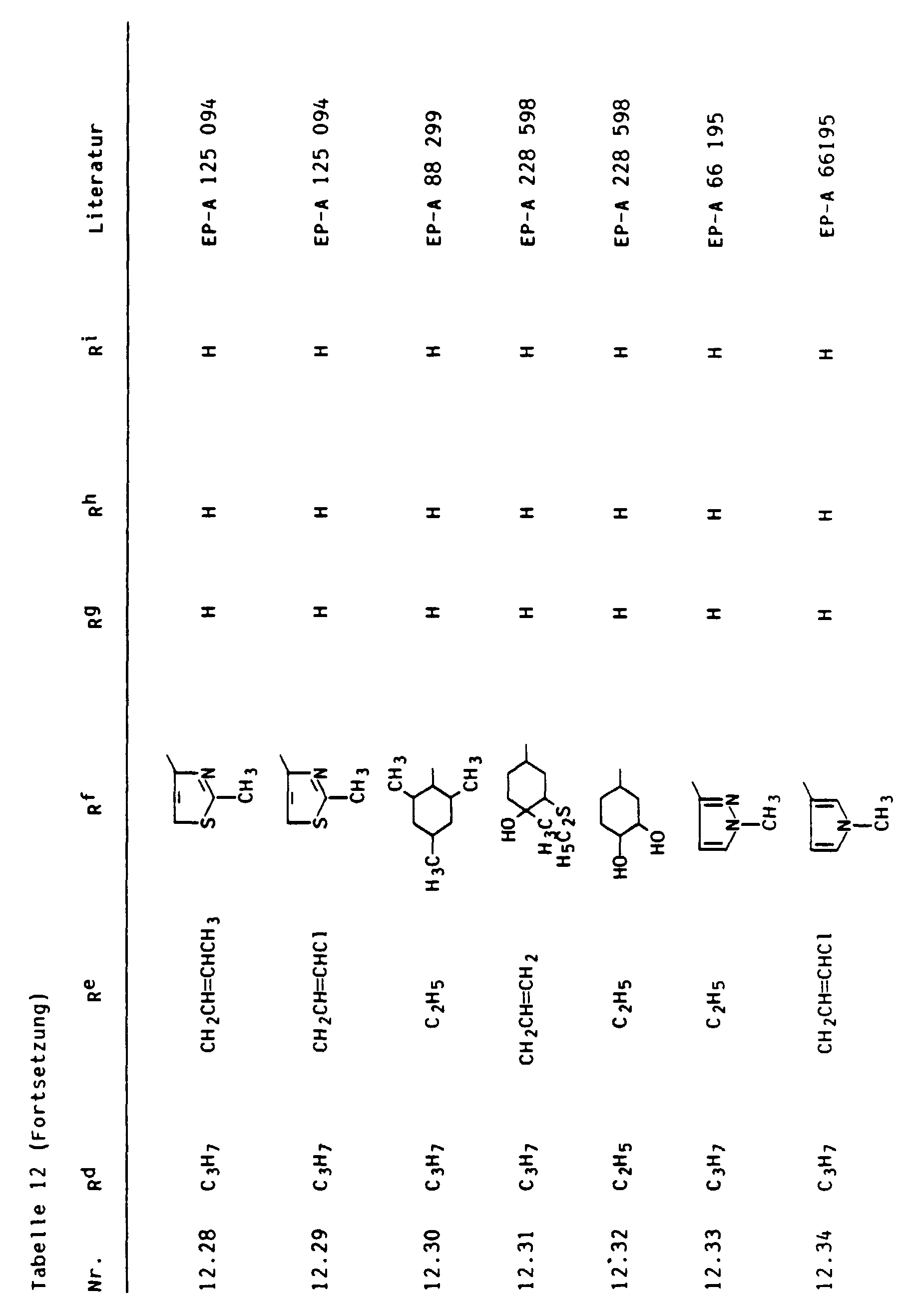

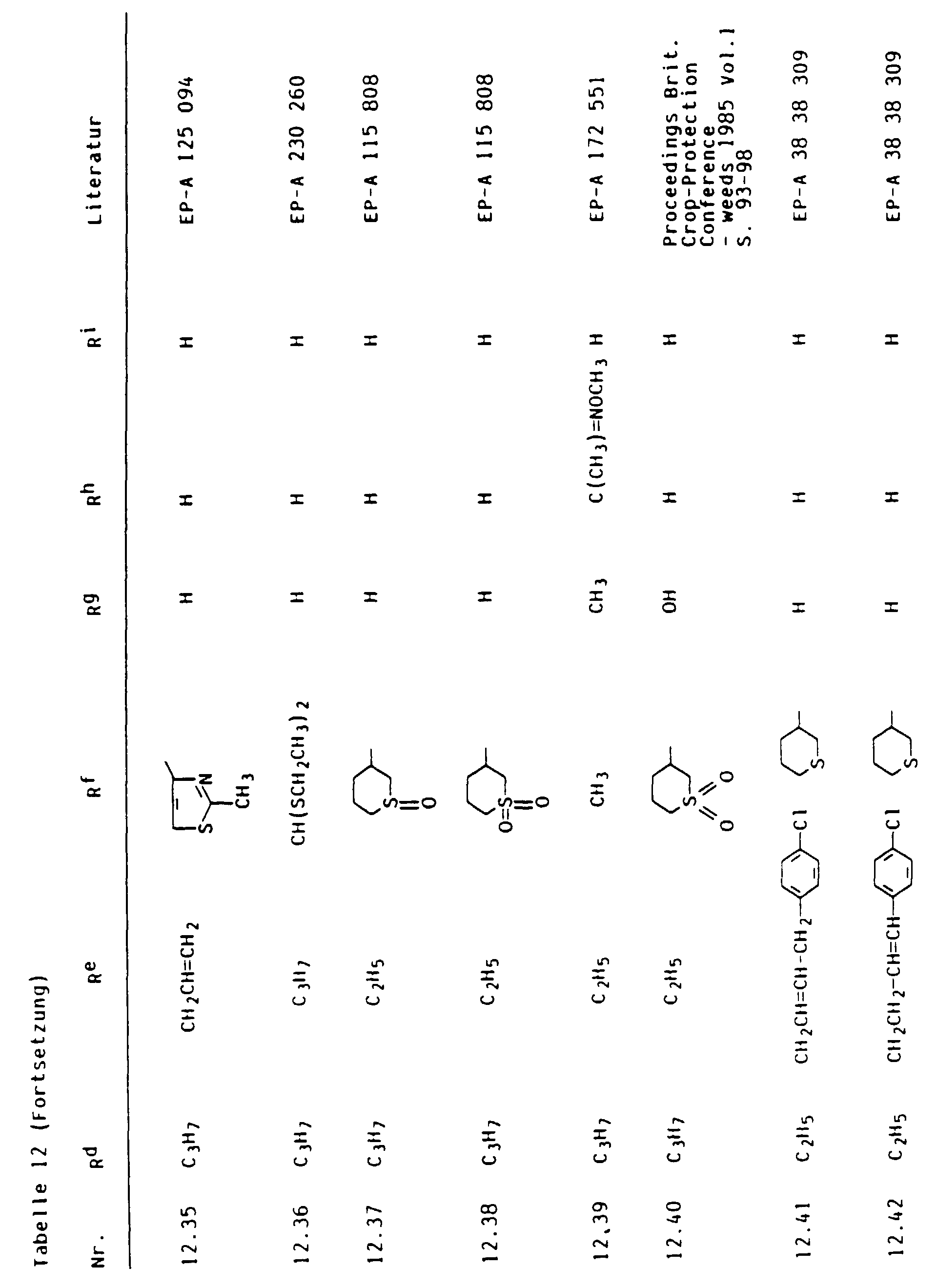

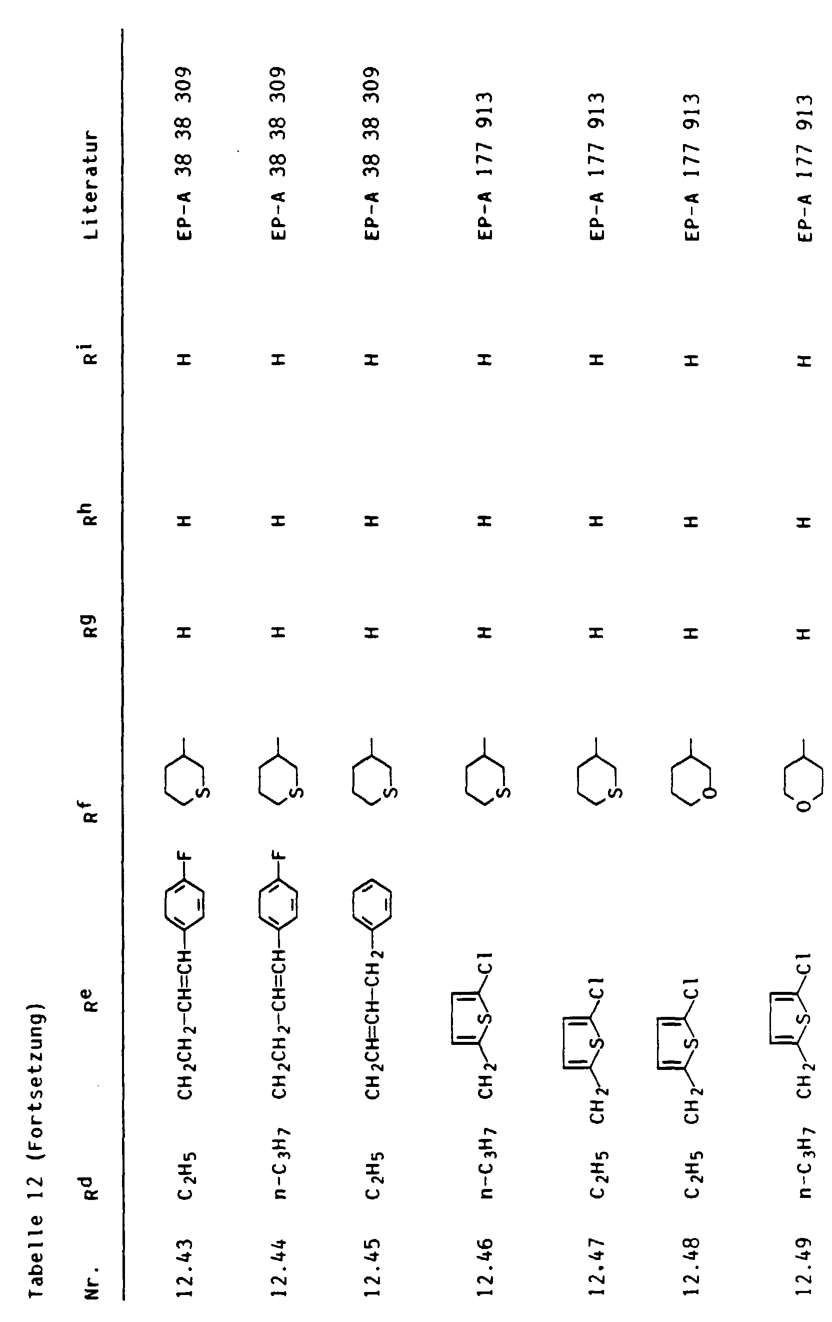

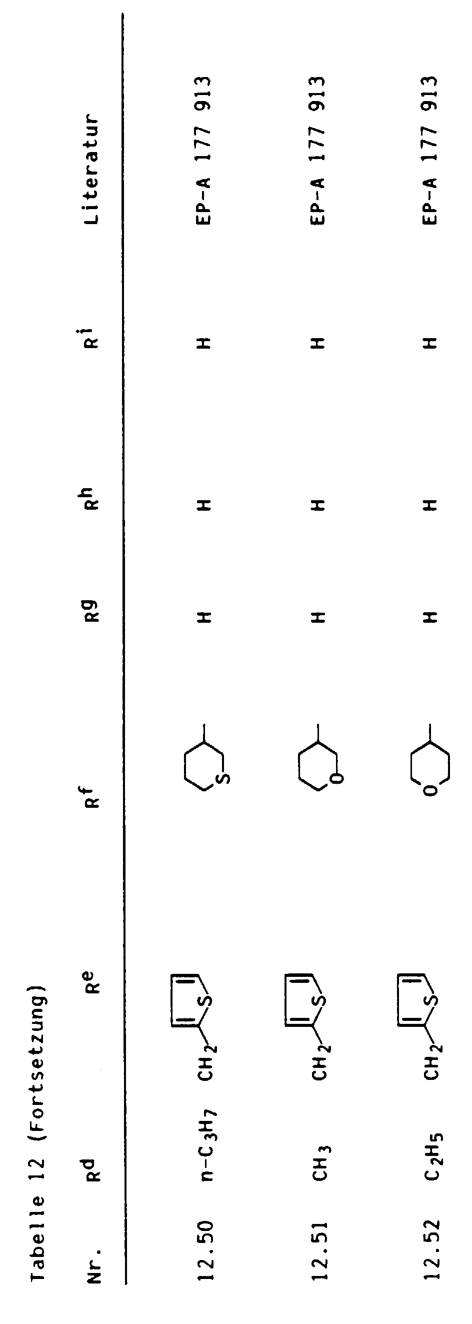

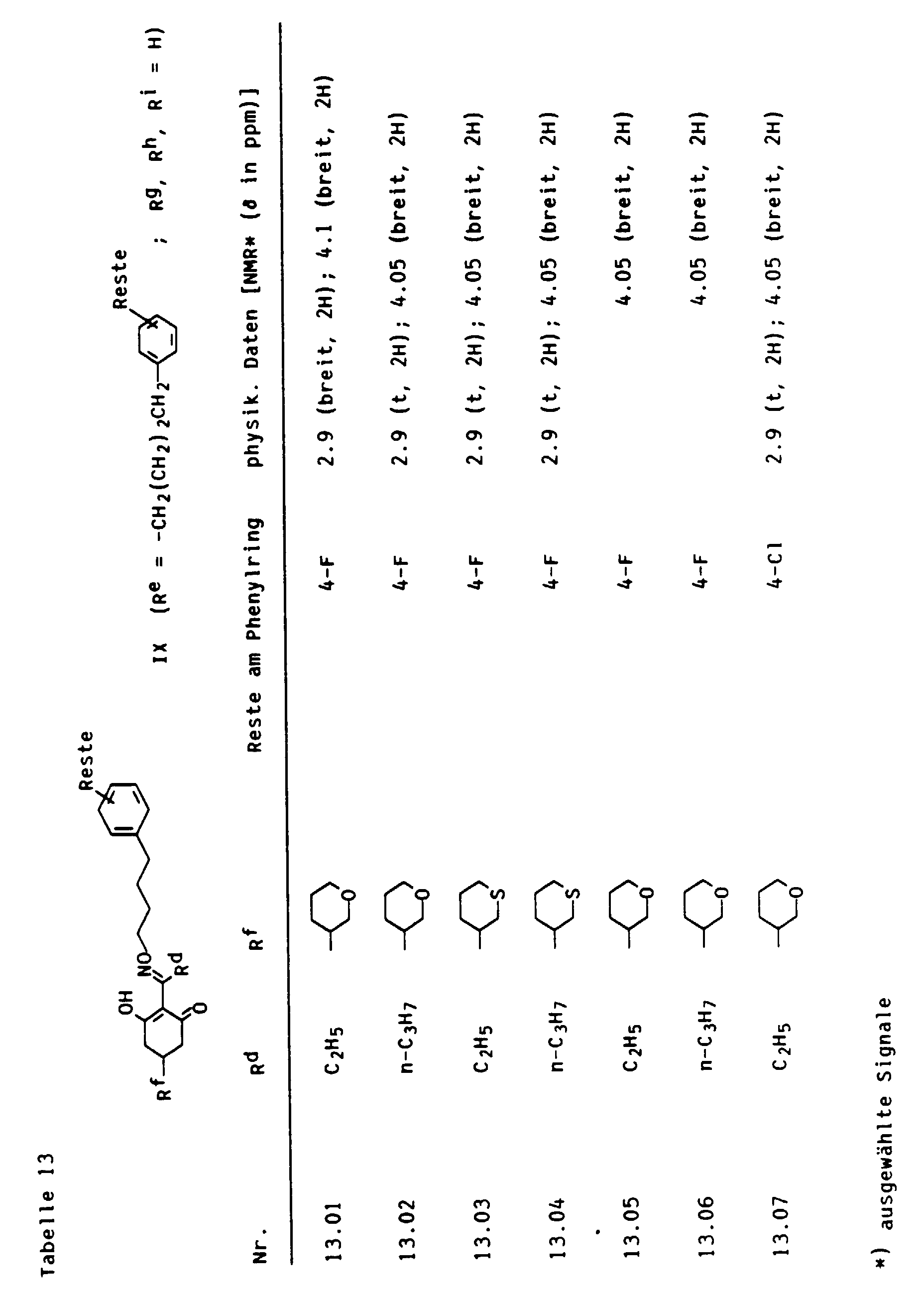

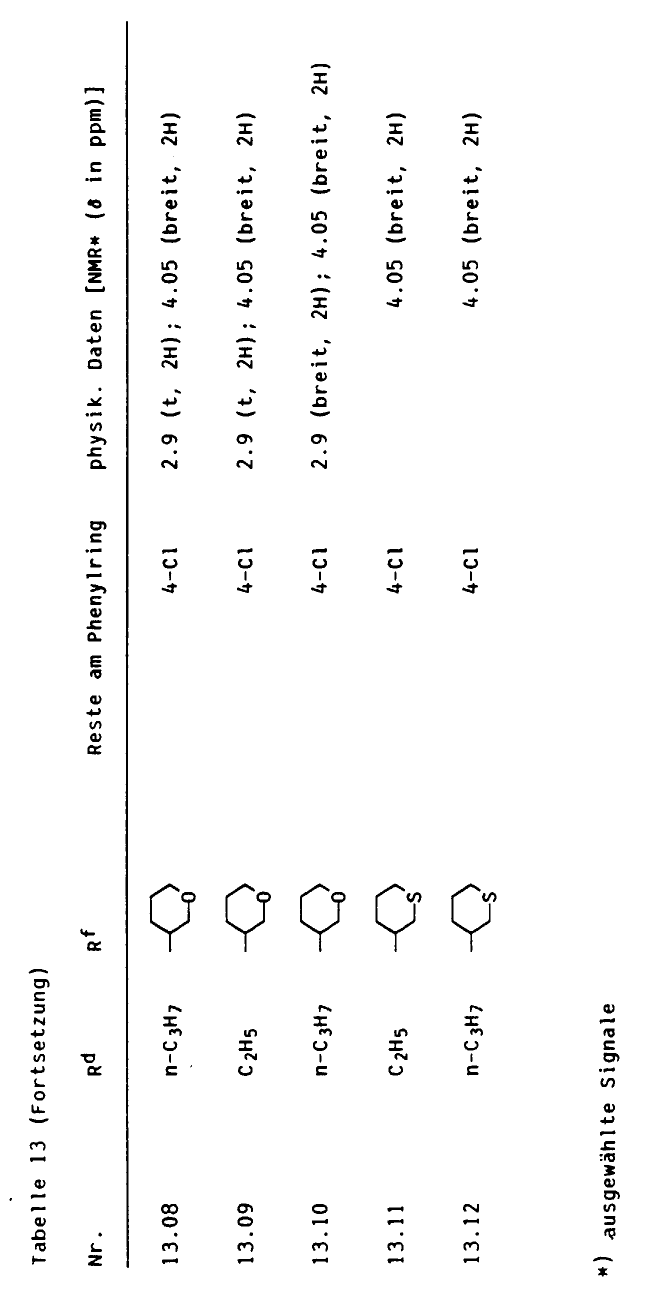

Herbizide Wirkstoffe und antidotisch wirkende Verbindungen können gemeinsam oder getrennt nach dem Auflaufen auf die Blätter und Sprosse der Kulturpflanzen und der unerwünschten Gräser ausgebracht werden. Bevorzugt wird das antidotisch wirkende Mittel gleichzeitig mit dem herbiziden Wirkstoff ausgebracht. Auch eine getrennte Ausbringung, wobei das Antidot zuerst und anschließend der herbizide Wirkstoff auf das Feld gebracht werden, ist möglich. Herbizider Wirkstoff und Antidot können hierbei als Spritzmittel in suspendierbarer, emulgierbarer oder löslicher Form gemeinsam oder getrennt formuliert vorliegen.

Antidotische Effekte werden auch durch Behandlung der Kulturpflanzensamen oder der Stecklinge mit dem Antidot vor der Aussaat bzw. vor dem Auspflanzen erzielt. Der herbizide Wirkstoff wird dann allein in der üblichen Weise appliziert.

Bei der Saatgutbehandlung werden im allgemeinen Wirkstoffmengen von 0,1 bis 10 g, vorzugsweise 1 bis 2 g, je Kilogramm Saatgut benötigt.

Bei der Applikation des Antidot durch Samenquellung oder bei der Stecklingsbehandlung werden bevorzugt Lösungen eingesetzt, die den antagonistischen Wirkstoff in einer Konzentration von 1 bis 10.000 ppm, insbesondere 100 bis 10.000 ppm, enthalten.

Für herbizide 2-(4-Heteroaryloxy)- bzw. 2-(4-Aryloxy)-phenoxyessigsäurederivate VIII werden unterschiedliche Mengen einer antidotisch wirkenden Verbindung benötigt, wenn das Herbizid in verschiedenen Kulturen eingesetzt wird. Die Mengenverhältnisse sind in breiten Bereichen variabel. Sie sind ebenfalls abhängig von der Struktur der 2-(4-Heteroaryloxy)- bzw. 2-(4-Aryloxy)-phenoxyessigsäurederivate VIII und der jeweiligen Zielkultur. Geeignete Anteilverhältnisse herbizider Wirkstoffe:antidotisch wirkender Verbindung liegen zwischen 1 : 10 bis 1 : 0,01; vorzugsweise zwischen 1 : 4 bis 1 : 0,1 Gew.-Teile.

Für das gleiche Cyclohexenonderivat IX werden unterschiedliche Mengen einer antidotisch wirkenden Verbindung benötigt, wenn das Cyclohexenonderivat IX in verschiedenen Kulturen eingesetzt wird. Die Mengenverhältnisse, in denen ein Cyclohexenonderivat IX und ein 2-Amino-4-oxo-4H-benzopyran I oder I' eingesetzt werden, sind in breiten Bereichen variabel. Sie sind abhängig von der Struktur des Cyclohexenonderivats IX, des 2-Amino-4-oxo-4H-benzopyrans I bzw. I' und der jeweiligen Kultur. Geeignete Anteilverhältnisse herbizider Wirkstoff: antidotisch wirkender Verbindung liegen zwischen 1 : 10 bis 1 : 0,01; vorzugsweise 1 : 4 bis 1 : 0,25 Gew.-Teile sowohl bei gemeinsamer als auch bei getrennter Ausbringung.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentige wäßrige, ölige oder sonstige Suspensionen, oder Dispersionen, Emulsionen, öldispersionen, Pasten, Stäubemitteln, Streumitteln oder Granulaten durch Versprühen, Vernebeln, Verstäuben, Verstreuen oder Giepen angewendet. Die Anwendungsformen richten sich ganz nach den Verwendungszwecken.

Zur Herstellung von direkt versprühbaren Lösungen, Emulsionen, Pasten und ölsdispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon und Wasser, in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können herbizider Wirkstoff und/oder Antidot als solche oder in einem öl oder Lösungsmittel gelöst, mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin-Sulfitablaugen und Methylcellulose in Betracht.

Pulver, Streu- und Stäubmittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten zwischen 0,01 und 95 Gew.-% an herbizidem Wirkstoff und Antidot, vorzugsweise zwischen 0,5 und 90 Gew.-%. Die Aufwandmengen an herbizidem Wirkstoff betragen 0,05 bis 5 kg/ha aktive Substanz (a.S.).

Die neuen herbiziden Mittel können neben dem 2-Amino-4-oxo-4H-benzopyran I oder I' als Antidot und dem Herbizid aus der Gruppe der 2-(4-Heteroaryloxy)- bzw. 2-(4-Aryloxy)phenoxyessigsäuren VIII bzw. der Cyclohexenone IX weitere herbizide oder wachstumsregulierende Wirkstoffe anderer chemischer Struktur enthalten, wobei der antagonistische Effekt erhalten bleibt.

### Herstellungsbeispiele

### Beispiel 1

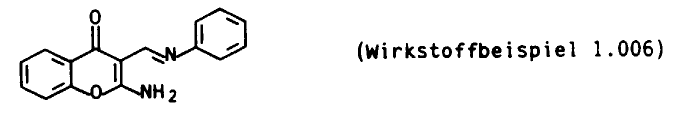

9,45 g (0,05 mol) 2-Amino-4-ox-4H-chromen-3-carbaldehyd wurden mit 4,7 ml (0,05 mol) Anilin und 0,5 g p-Toluolsulfonsäure in 150 ml Toluol 60 Minuten am Wasserabscheider erhitzt. Der beim Abkühlen ausgefallene Niederschlag wurde abgesaugt, mit Wasser gewaschen und getrocknet.
Ausbeute: 9,8 g (75 %); Fp. 210-214°C.

### Beispiel 2

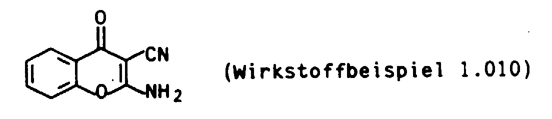

9,5 g (0,050 mol) 2-Amino-4-oxo-4H-chromen-3-carbaldehyd wurden mit 3,8 g (0,055 mol) Hydroxylammoniumchlorid in 50 ml Ameisensäure 45 Minuten zum Sieden erhitzt. Die abgekühlte Lösung wurde mit 200 ml Wasser versetzt, der Niederschlag abgesaugt, mit Wasser gewaschen und getrocknet.
Ausbeute: 8,4 g (90 %); Fp. >250°C.

### Beispiel 3

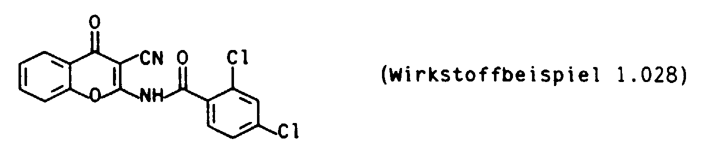

Eine Mischung aus 5,6 g (0,03 mol) des Produktes aus Beispiel 2 und 60 ml Pyridin wurde mit 5,6 ml (0,04 mol) 2,4-Dichlor-benzoylchlorid versetzt und 15 h bei 80°C gerührt. Die abgekühlte Lösung wurde mit 300 ml 5 %iger Salzsäure versetzt, wobei das Produkt als Feststoff ausfiel.
Ausbeute: 6,5 g (60 %); Fp. 96-98°C .

Die in der folgenden Tabelle 14 aufgeführten Wirkstoffe können analog diesen Verfahrensbeispielen hergestellt werden.

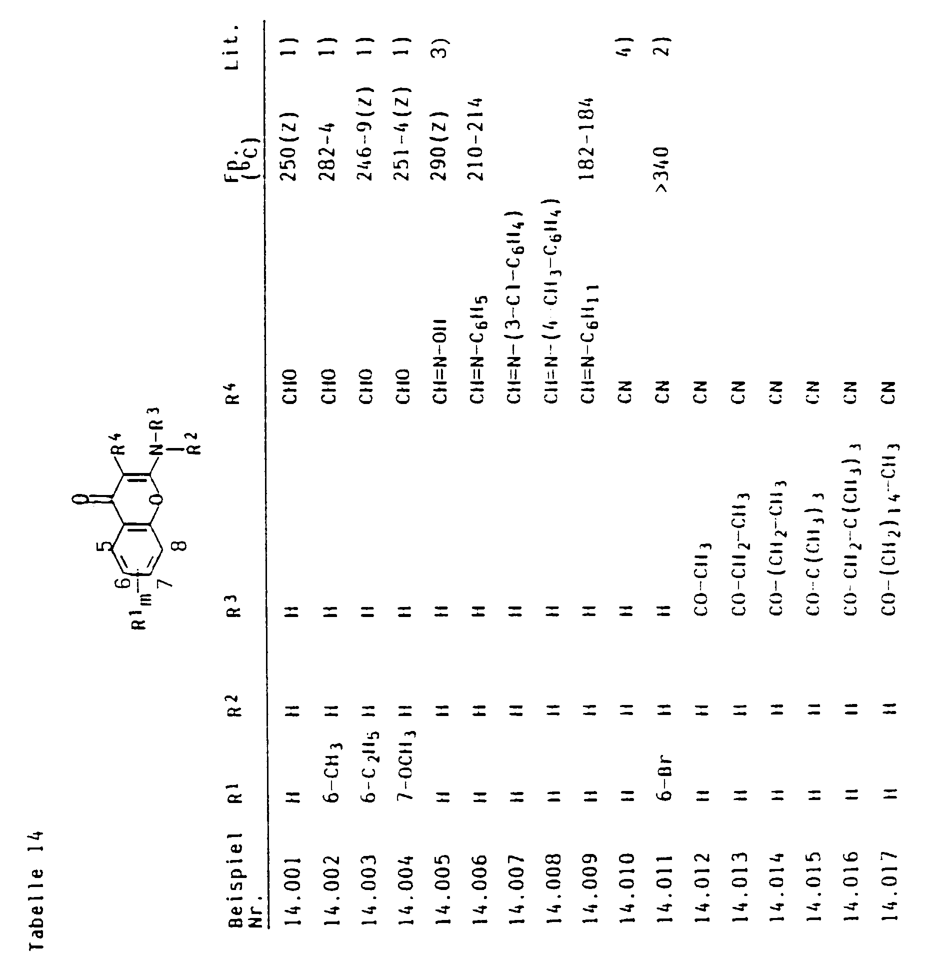

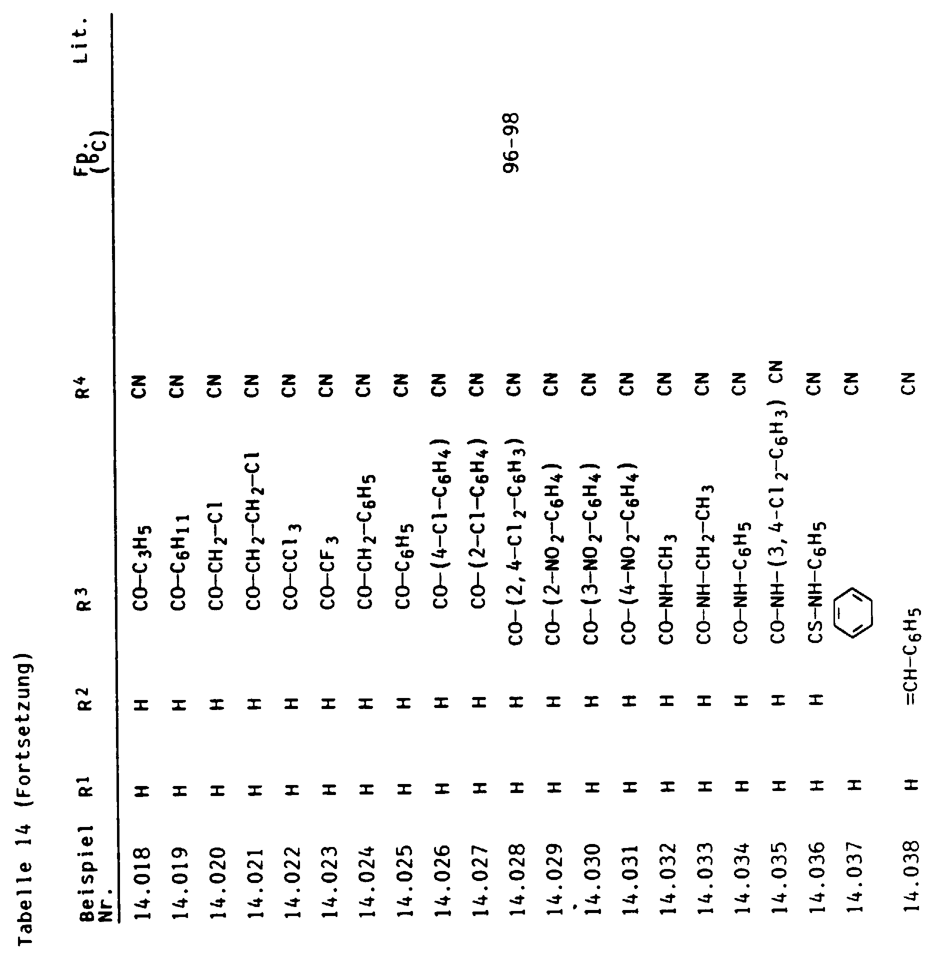

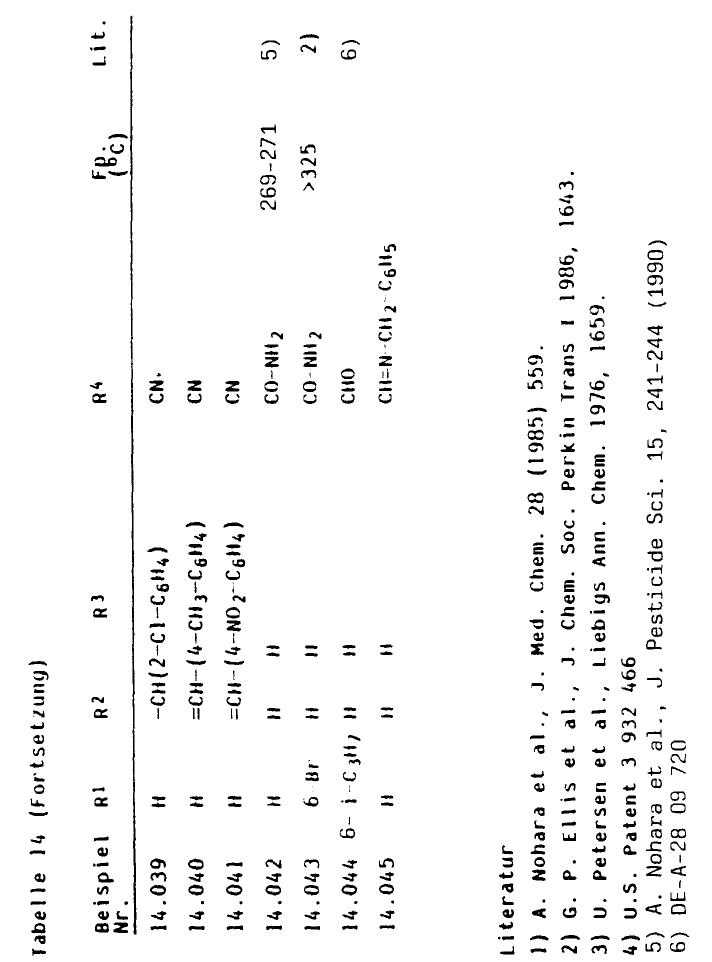

### Beispiele zur biologischen Wirkung

Der Einfluß verschiedener Vertreter der erfindungsgemäßen herbiziden Mittel bzw. Mittelkombinationen, bestehend aus Herbizid und antidotisch wirkender Verbindung, auf das Wachstum von erwünschten und unerwünschten Pflanzen im Vergleich zum herbiziden Wirkstoff allein wird durch die folgenden biologischen Beispiele aus Gewächshausversuchen belegt:

Als Kulturgefäße dienten Plastikblumentöpfe mit rund 300 cm³ Inhalt und lehmiger Sand mit etwa 3,0 % Humus als Substrat. Die Samen der Testpflanzen werden nach Arten getrennt, flach eingesät und befeuchtet. Danach wurden die Gefäße mit durchsichtigen Plastikhauben abgedeckt, bis die Samen gleichmäßig gekeimt und die Pflanzen angewachsen waren.

Für die Nachauflaufbehandlung wurden die Testpflanzen je nach Wuchsform erst bis zu einer Wuchshöhe von 3 bis 20 cm angezüchtet und erst dann behandelt. Die herbiziden Mittel wurden hierbei in Wasser als Verteilungsmittel suspendiert oder emulgiert und mittels fein verteilender Düsen gespritzt.

Die Versuchsgefäße wurden im Gewächshaus aufgestellt, wobei für wärmeliebende Arten 18 bis 30°C und für solche gemäßigter Klimate 10 bis 25°C bevorzugt wurden.

Die Versuchsperiode erstreckte sich über 3 bis 5 Wochen. Während dieser Zeit wurden die Pflanzen gepflegt, und ihre Reaktion auf die einzelnen Behandlungen wurde erfaßt.

Bewertet wurde die Schädigung der Testpflanzen anhand einer Skala von 0 bis 100 % im Vergleich zu den unbehandelten Kontrollpflanzen. Dabei bedeutet 0 keine Schädigung und 100 eine völlige Zerstörung der Pflanzen.

Die in den Gewächshausversuchen verwendeten Pflanzen setzten sich aus folgenden Arten zusammen:

| Lat.Name | Deutscher Name | Englischer Name |
|---|---|---|
| Setaria viridis | Grüne Borstenhirse | green foxtail |
| Triticum aestivum | Weichweizen | wheat |
| Zea mays | Mais | Indian corn |

Als Beispielsherbizid der Cyclohexenonderivate der Formel IX diente

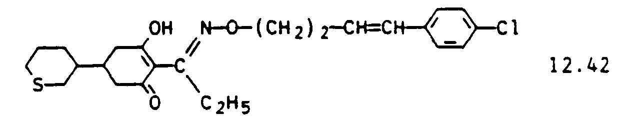

Der Wirkstoff 12.42 wurde als Emulsionskonzentrat mit 200 g Wirkstoff/l allein unter Zugabe der für die Antidots nötigen Lösungsmittelmengen, 80 Gew.-% Cyclohexenon und 20 Gew.-% Tensid (Emulphor EL^{*)}) mit 10 Gew.-% Wirkstoff, appliziert.
^{*)} ethoxyliertes Rizinusöl (castor oil)

Sämtliche antidotisch wirkenden Verbindungen wurden für die Nachauflaufbehandlung in einem Gemisch, bestehend aus 80 Gew.-% Cyclohexenon und 20 Gew.-% Tensid (Emulphor® EL^{*)}) mit 10 Gew.-% Wirkstoff aufbereitet.

Die anschließende Tabelle dokumentiert die antidotische Wirkung der erfindungsgemäßen Verbindung 14.012.

Dabei verbessert diese Verbindung deutlich die Verträglichkeit des Wirkstoffs 12.42 für zu der Familie der Gramineen (Gräser) zählende Kulturpflanzen.

**Tabelle 15**

| Verbesserung der Verträglichkeit des herbiziden Wirkstoffs 12.42 für Kulturpflanzen durch Kombination mit der Verbindung 14.012 bei Nachauflaufanwendung im Gewächshaus | | | | |
|---|---|---|---|---|
| Aufwandmenge | | Testpflanzen und Schädigung [%] | | |
| [kg/ha a.S.] | | Kulturpflanzen | | unerw.Pflanze |
| Herbizid 12.42 | Antidot 14.012 | Triticum aestivum¹⁾ | Zea mays²⁾ | Setaria viridis |
| 0.25 | - | 98 | 90 | 100 |
| 0.25 | 0.5 | 15 | 0 | 100 |

| | | | | |
|---|---|---|---|---|
| ¹⁾ Sorte "Urban" | | | | |
| ²⁾ Sorte "Lixis" | | | | |

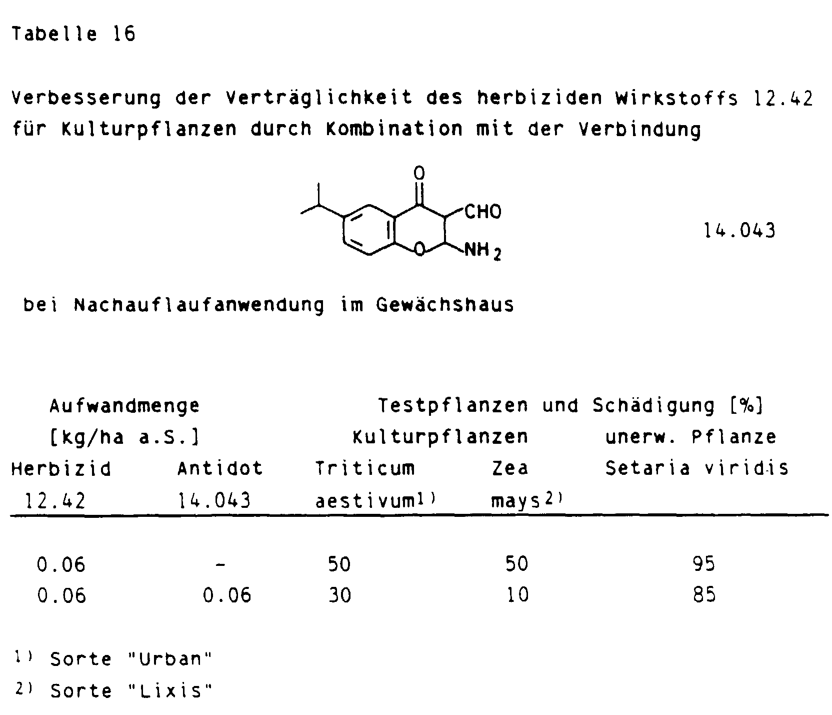

## Patentansprüche

1. 2-Amino-4-oxo-4H-benzopyrane der allgemeinen Formel I 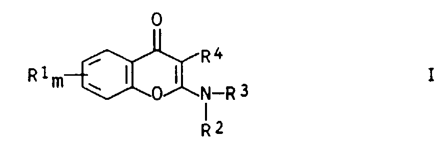 in der die Substituenten und der Index folgende Bedeutung haben:
R¹ Wasserstoff; Hydroxy; Halogen; Nitro; C₁-C₆-Alkyl, C₁-C₆-Alkoxy; C₁-C₆-Halogenalkyl; C₁-C₄-Halogenalkoxy; C₁-C₄-Alkylthio oder eine Gruppe -NR⁵R⁶ mit
R⁵ Wasserstoff oder C₁-C₄-Alkyl und
R⁶ Wasserstoff, C₁-C₄-Alkyl, -CO-R⁷, -CS-R⁷ oder -SO₂-R⁸, wobei
R⁷ C₁-C₂₀-Alkyl; C₁-C₆-Halogenalkyl; C₃-C₈-Cycloalkyl; Phenyl-C₁-C₃-alkyl; Amino; C₁-C₄-Alkylamino; Di-C₁-C₄-alkylamino; eine Phenyl- oder Phenylaminogruppe, wobei die Phenylreste jeweils unsubstituiert sind oder eine bis drei der bei R¹ genannten Gruppen tragen; und
R⁸ C₁-C₄-Alkyl oder Phenyl, das eine bis drei der für R¹ genannten Gruppen tragen kann,
bedeuten
m 0, 1 oder 2, wobei die Reste R¹ verschieden sein können, wenn m 2 bedeutet;
R² Wasserstoff oder C₁-C₄-Alkyl;
R³ Wasserstoff; C₁-C₄-Alkyl; eine Gruppe -CO-R⁹, -CS-R⁹ oder -SO₂-R¹⁰, wobei
R⁹ für eine der unter R⁷ und R¹⁰ für eine der unter R⁸ genannten Bedeutungen steht;
oder
R² und R³ zusammen mit dem Stickstoffatom, an das sie gebunden sind, einen 3- bis 8-gliedrigen gesättigten oder ungesättigten Ring, der gewünschtenfalls seinerseits noch ein bis drei C₁-C₄-Alkylreste tragen kann, oder eine Gruppe =CR²R¹¹, mit
R¹¹ Wasserstoff; Hydroxyl; C₁-C₄-Alkyl; C₁-C₈-Alkoxy; C₃-C₈-Cycloalkyl; Phenyl; Benzyl; Phenyl, welches eine bis drei der für R¹ genannten Gruppen trägt; eine Gruppe -O-CO-R¹² oder -O-CS-R¹², wobei
R¹² für C₁-C₂₀-Alkyl; C₁-C₆-Halogenalkyl; C₃-C₈-Cycloalkyl; Phenyl oder Phenyl-C₁-C₃-alkyl, wobei die Phenylreste jeweils unsubstituiert sein oder einen bis drei der für R¹ genannten Reste tragen können, steht;
R⁴ Cyano; eine Gruppe -CO-R¹³, -CS-R¹³- oder -CH=N-R¹⁴, wobei
R¹³ für Wasserstoff; C₁-C₆-Alkyl; C₁-C₆-Halogenalkyl; Amino; C₁-C₄-Alkylamino; Di-C₁-C₄-alkylamino; Hydrazino; C₁-C₆-Alkylhydrazino; Phenylhydrazino, wobei der Phenylrest unsubstituiert oder durch eine bis drei der bei R¹ genannten Gruppen substituiert ist, und
R¹⁴ für einen der Reste R¹¹ steht;
sowie die landwirtschaftlich brauchbaren Salze der Verbindungen I,
ausgenommen 2-[(Methylcarbonyl)amino]-4-oxo-4H-chromen-3-carboxamid, 2-[(Methylcarbonyl)amino]-6-(tert.-butyl)-4-oxo-4H-chromen-3-carboxamid, 2-Methylamino-6-(tert.-butyl)-4-oxo-4H-chromen-3-carboxamid, 2-Methylamino-7-(tert.-butyl)-4-oxo-4H-chromen-3-carboxamid, 2-[(Methylaminocarbonyl)amino]-6-(tert.-butyl)-4-oxo-4H-chromen-3-carboxamid, 3-Cyano-2-dimethylamino-8-isopropyl-5-methyl-4-oxo-4H-chromen, 3-Cyano-2-diethylamino-7-methoxy-4-oxo-4H-chromen, 2-Dimethylamino-8-isopropyl-5-methyl-4-oxo-4H-chromen-3-carboxaldehydoxim, 2-Diethylamino-7-methoxy-4-oxo-4H-chromen-3-carboxaldehydoxim, 2-(Piperidin-1-yl)-4-oxo-4H-chromen-3-carbaldehyd, 3-Methylcarbonyl-2-(piperidin-1-yl)-4-oxo-4H-chromen, 3-Methylcarbonyl-2-amino-4-oxo-4H-chromen, 3-Methylcarbonyl-2-(N-methyl-N-isopropylamino)-4-oxo-4H-chromen,
sowie diejenigen Verbindungen I, bei denen entweder R⁴ für Cyano, eine Gruppe CONH₂ oder die Formylgruppe, R² und R³ für Wasserstoff und R¹ für Wasserstoff, Hydroxy, Halogen, C₁-C₆-Alkyl, C₁-C₆-Alkoxy, Phenyl Nitro, Amino und/oder Dimethylamino stehen,
oder bei denen R⁴ für die Formylgruppe, R² und R³ beide für Methyl oder Ethyl und R¹ für Wasserstoff, einer Methoxygruppe, 2 Methylgruppen oder eine Methyl- und eine Isopropylgruppe stehen,
oder bei denen R⁴ für eine Gruppe -CH=N-OH und R¹, R² und R³ für Wasserstoff stehen.

2. 2-Amino-4-oxo-4H-benzopyrane der Formel I gemäß Anspruch 1, in der R³ eine Gruppe -C(O)R⁹ und R⁴ die Cyanogruppe bedeuten.

3. 2-Amino-4-oxo-4H-benzopyrane der Formel I, in der R⁴ eine Gruppe -CH=N-R¹⁴ bedeutet.

4. Verfahren zur Herstellung der Verbindung I gemäß Anspruch 1, dadurch gekennzeichnet, daß man einen 2-Amino-4-oxo-4H-benzopyran-3-carbaldehyd II 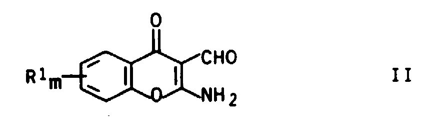 in an sich bekannter Weise in das o-Aminonitril III 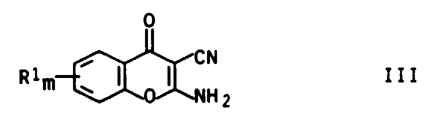 überführt und dieses durch Umsetzung mit Elektrophilen wie IV und V
Hal-CX-R⁹ IV
O=CR²R¹¹ V
an der Aminogruppe derivatisiert.

5. Herbizides Mittel, enthaltend neben inerten Zusatzstoffen mindestens ein 2-Amino-4-oxo-4H-benzopyran der allgemeinen Formel I' 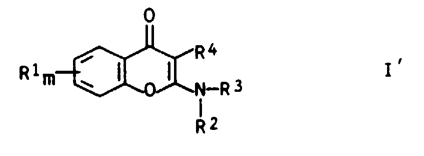 in der die Substituenten und der Index folgende Bedeutung haben:
R¹ Wasserstoff; Hydroxy; Halogen; Nitro; C₁-C₆-Alkyl, C₁-C₆-Alkoxy; C₁-C₆-Halogenalkyl; C₁-C₄-Halogenalkoxy; C₁-C₄-Alkylthio oder eine Gruppe -NR⁵R⁶ mit
R⁵ Wasserstoff oder C₁-C₄-Alkyl und
R⁶ Wasserstoff, C₁-C₄-Alkyl, -CO-R⁷, -CS-R⁷ oder -SO₂-R⁸, wobei
R⁷ C₁-C₂₀-Alkyl; C₁-C₆-Halogenalkyl; C₃-C₈-Cycloalkyl; Phenyl-C₁-C₃-alkyl; Amino; C₁-C₄-Alkylamino; Di-C₁-C₄-alkylamino; eine Phenyl- oder Phenylaminogruppe, wobei die Phenylreste jeweils unsubstituiert sind oder eine bis drei der bei R¹ genannten Gruppen tragen; und
R⁸ C₁-C₄-Alkyl oder Phenyl, das eine bis drei der für R¹ genannten Gruppen tragen kann,
bedeuten
m 0, 1 oder 2, wobei die Reste R¹ verschieden sein können, wenn m 2 bedeutet;
R² Wasserstoff oder C₁-C₄-Alkyl;
R³ Wasserstoff, C₁-C₄-Alkyl, eine Gruppe -CO-R⁹, -CS-R⁹ oder -SO₂-R¹⁰, wobei R⁹ für eine der unter R⁷ und R¹⁰ für eine der unter R⁸ genannten Bedeutungen stehen;
oder
R² und R³ zusammen mit dem Stickstoffatom, an das sie gebunden sind, einen 3- bis 8-gliedrigen gesättigten oder ungesättigten Ring, der seinerseits gewünschten falls noch ein bis drei C₁-C₄-Alkylreste tragen kann,
oder eine Gruppe =CR²R¹¹, mit
R¹¹ Wasserstoff; Hydroxyl; C₁-C₄-Alkyl; C₁-C₈-Alkoxy; C₃-C₈-Cycloalkyl; Phenyl; Benzyl; Phenyl, welches eine bis drei der für R¹ genannten Gruppen trägt; eine Gruppe -O-CO-R¹² oder -O-CS-R¹², wobei
R¹² für C₁-C₂₀-Alkyl; C₁-C₆-Halogenalkyl; C₃-C₈-Cycloalkyl; Phenyl oder Phenyl-C₁-C₃-alkyl, wobei die Phenylreste jeweils unsubstituiert sein oder einen bis drei der für R¹ genannten Reste tragen können, steht;
R⁴ Cyano; eine Gruppe -CO-R¹³, -CS-R¹³- oder -CH=N-R¹⁴, wobei
R¹³ für Wasserstoff; C₁-C₆-Alkyl; C₁-C₆-Halogenalkyl; Amino; C₁-C₄-Alkylamino; Di-C₁-C₄-alkylamino; Hydrazino; C₁-C₆-Alkylhydrazino; Phenylhydrazino, wobei der Phenylrest unsubstituiert oder durch eine bis drei der bei R¹ genannten Gruppen substituiert ist; und
R¹⁴ für einen der Reste R¹¹ steht;
sowie die landwirtschaftlich brauchbaren Salze der Verbindungen I' und mindestens einen herbiziden Wirkstoff aus der Gruppe
A) der 2-(4-Heteroaryloxy)- oder 2-(4-Aryloxy)-phenoxyessigsäurederivate der Formel VIII 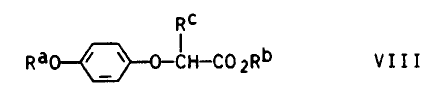
R^{a} ein Phenylring, ein Pyridylring, ein Benzoxazylrest, ein Benzthiazylrest oder ein Benzpyrazinylrest, wobei diese aromatischen Ringsysteme bis zu zwei der folgenden Reste tragen können:
Halogen, Nitro, C₁-C₄-Alkyl, C₁-C₄-Halogenalkyl und/oder C₁-C₄-Halogenalkoxy,
R^{b} Wasserstoff, eine C₁-C₄-Alkylgruppe oder das Äquivalent eines pflanzenverträglichen Kations und
R^{c} Wasserstoff oder eine Methylgruppe
und/oder
B) der Cyclohexenonoximether der Formel IX 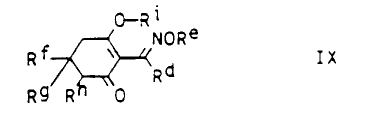 in welcher die Substituenten folgende Bedeutung haben:
R^{d} eine C₁-C₄-Alkylgruppe;
R^{e} eine C₁-C₄-Alkylgruppe, eine C₃-C₄-Alkenylgruppe, eine C₃-C₄-Alkinylgruppe oder eine partiell oder vollständig halogenierte C₃-C₄-Alkenylgruppe;
eine C₁-C₄-Alkylen- oder C₃-C₄-Alkenylenkette, die beide noch einen C₁-C₃-Alkylrest und/oder ein Halogenatom tragen können, oder eine gewünschtenfalls durch C₁-C₃-Alkyl substituierte 3- bis 6-gliedrige Alkylen- oder 4- bis 6-gliedrige Alkenylenkette, die jeweils ein dem Oximetherteil nicht direkt benachbartes Sauerstoff- oder Schwefelatom als Kettenglied enthält, wobei alle vorstehend genannten Ketten endständig den Phenylring tragen, der seinerseits durch ein bis drei Reste substituiert sein kann, ausgewählt aus einer Gruppe bestehend aus einem Benzyloxycarbonyl- oder Phenylrest und jeweils einem bis drei der folgenden Reste: Nitro, Cyano, Halogen, C₁-C₄-Alkyl, C₁-C₄-Alkoxy, C₁-C₄-Alkylthio, partiell oder vollständig halogeniertes C₁-C₄-Alkyl, partiell oder vollständig halogeniertes C₁-C₄-Alkoxy, Carboxyl und C₁-C₄-Alkoxycarbonyl, und wobei der Phenylring noch zusätzlich so viele Halogenatome tragen kann, daß die Gesamtzahl der Reste 4 oder 5 beträgt;
eine Thienylmethylgruppe, die noch ein Halogenatom tragen kann;
R^{f} eine C₁-C₄-Alkylgruppe, welche einfach durch C₁-C₄-Alkylthio oder C₁-C₄-Alkoxy substituiert sein kann;
ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, wobei dieser Ring bis zu drei der folgenden Reste tragen kann: Hydroxy, Halogen, C₁-C₄-Alkyl, partiell oder vollständig halogeniertes C₁-C₄-Alkyl, C₁-C₄-Alkoxy und/oder C₁-C₄-Alkylthio;
einen 10-gliedrigen gesättigten oder einfach ungesättigten Heterocyclus, welcher zwei Sauerstoffatome oder Schwefelatome enthält und durch bis zu drei C₁-C₄-Alkylgruppen und/oder Methoxygruppen substituiert sein kann;
die Phenylgruppe, die Pyridylgruppe, die Thiazolylgruppe, die Pyrazolylgruppe, die Pyrrolylgruppe oder die Isoxazolylgruppe, wobei diese Gruppen jeweils einen bis drei Reste tragen können, ausgewählt aus einer Gruppe bestehend aus C₁-C₄-Alkyl, partiell oder vollständig halogeniertem C₁-C₄-Alkyl, C₁-C₄-Alkoxy, C₁-C₄-Alkylthio, C₃-C₆-Alkenyloxy, C₃-C₆-Alkinyloxy, C₁-C₄-Dialkoxy-C₁-C₃-alkyl, C₂-C₈-Alkoxyalkyl, C₂-C₈-Alkylthioalkyl, Formyl, Halogen und Benzoylamino;
R^{g} Wasserstoff, Hydroxy oder, wenn R^{f} die Bedeutung C₁-C₆-Alkylgruppe hat, eine C₁-C₆-Alkylgruppe;
R^{h} Wasserstoff, die Cyanogruppe, ein Halogenatom, eine C₁-C₄-Alkoxycarbonylgruppe oder eine C₁-C₄-Alkylketoximgruppe und
Rⁱ Wasserstoff oder das Äquivalent eines landwirtschaftlich brauchbaren Kations.

6. Herbizides Mittel nach Anspruch 5, dadurch gekennzeichnet, daß das Gewichtsverhältnis 2-Amino-4-oxo-4H-benzopyran I' zu herbizidem Wirkstoff A) oder B) 10:1 bis 0,01:1 Gew.-Teile beträgt.

7. Verfahren zur selektiven Bekämpfung von unerwünschtem Pflanzenwuchs, dadurch gekennzeichnet, daß man ein 2-Amino-4-oxo-4H-benzopyran der Formel I' gemäß Anspruch 5 und
A) ein 2-(4-Heteroaryloxy)- oder 2-(4-Aryloxy)-phenoxyessigsäurederivat der Formel VIII oder
B) ein Cyclohexenonderivat der Formel IX
gemäß Anspruch 5 vor, bei oder nach der Aussaat der Kulturpflanzen, vor oder während des Auflaufens der Kulturpflanzen gleichzeitig oder nacheinander ausbringt.

8. Verfahren zur Verhinderung von Schädigungen von Kulturpflanzen durch herbizide
A) 2-(4-Heteroaryloxy)- oder 2-(4-Aryloxy)-phenoxyessigsäurederivate der Formel VIII oder
B) Cyclohexenonderivate der Formel IX
gemäß Anspruch 5, dadurch gekennzeichnet, daß man das Saatgut der Kulturpflanzen mit einer antagonistisch wirksamen Menge eines 2-Amino-4-oxo-4H-benzopyrans der Formel I' gemäß Anspruch 5 behandelt.

9. Verfahren zur selektiven Bekämpfung von unerwünschtem Pflanzenwuchs, dadurch gekennzeichnet, daß man die Blätter der Kulturpflanzen und der unerwünschten Pflanzen im Nachauflaufverfahren mit einem 2-Amino-4-oxo-4H-benzopyran der Formel I' gemäß Anspruch 5 und mit
A) einem 2-(4-Heteroaryloxy) oder 2-(4-Aryloxy)-phenoxyessigsäurederivat der Formel VIII oder
B) einem Cyclohexenonderivat der Formel IX
gemäß Anspruch 5 gleichzeitig oder nacheinander behandelt.

10. Verfahren nach Anspruch 7, dadurch gekennzeichnet, daß die Kulturpflanzen Gerste, Weizen, Mais, Kultursorghum oder Reis sind.

11. Verfahren nach Anspruch 8, dadurch gekennzeichnet, daß die Kulturpflanzen Gerste, Weizen, Mais, Kultursorghum oder Reis sind.

12. Verfahren nach Anspruch 9, dadurch gekennzeichnet, daß die Kulturpflanzen Gerste, Weizen, Mais, Kultursorghum oder Reis sind.

## Claims

1. A 2-amino-4-oxo-4H-benzopyran of the formula I 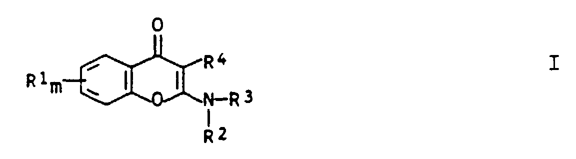 where
R¹ is hydrogen, hydroxyl, halogen, nitro, C₁-C₆-alkyl, C₁-C₆-alkoxy, C₁-C₆-haloalkyl, C₁-C₄-haloalkoxy, C₁-C₄-alkylthio or a group -NR⁵R⁶,
R⁵ is hydrogen or C₁-C₄-alkyl,
R⁶ is hydrogen, C₁-C₄-alkyl, -CO-R⁷, -CS-R⁷ or -SO₂-R⁸,
R⁷ is C₁-C₂₀-alkyl, C₁-C₆-haloalkyl, C₃-C₈-cycloalkyl, phenyl-C₁-C₃-alkyl, amino, C₁-C₄-alkylamino, di-C₁-C₄alkylamino, phenyl or phenylamino where the phenyl radicals are each unsubstituted or carry from one to three of the groups stated for R¹,
R⁸ is C₁-C₄-alkyl or is phenyl which may carry from one to three of the groups stated for R¹,
m is 0, 1 or 2, and the radicals R¹ may be different when m is 2,
R² is hydrogen or C₁-C₄-alkyl,
R³ is hydrogen, C₁-C₄-alkyl or a group -CO-R⁹, -CS-R⁹ or -SO₂-R¹⁰,
R⁹ has one of the meanings stated under R⁷,
R¹⁰ has one of the meanings stated under R⁸,
or R² and R³, together with the nitrogen atom to which they are bonded, form a 3-membered to 8-membered saturated or unsaturated ring which, if desired, may in turn furthermore carry from one to three C₁-C₄-alkyl radicals, or a group =CR²R¹¹,
R¹¹ is hydrogen, hydroxyl, C₁-C₄-alkyl, C₁-C₈-alkoxy, C₃-C₈-cycloalkyl, or benzyl or is phenyl which is unsubstituted or carries from one to three of the groups stated for R¹, or is -O-CO-R¹² or -O-CS-R¹²,
R¹² is C₁-C₂₀-alkyl, C₁-C₆-haloalkyl, C₃-C₈-cycloalkyl, phenyl or phenyl-C₁-C₃-alkyl, where the phenyl radicals may each be unsubstituted or may carry from one to three of the radicals stated for R¹,
R⁴ is cyano or a group -CO-R¹³, -CS-R¹³- or -CH=N-R¹⁴,
R¹³ is hydrogen, C₁-C₆-alkyl, C₁-C₆-haloalkyl, amino, C₁-C₄-alkylamino, di-C₁-C₄-alkylamino, hydrazino, C₁-C₆-alkylhydrazino or phenylhydrazino, where the phenyl radical is unsubstituted or substituted by from one to three of the groups stated for R¹ and
R¹⁴ is one of the radicals R¹¹,
and the agriculturally useful salts of the compounds I, with the exception of 2-[(methylcarbonyl)amino]-4-oxo-4H-chromene-3-carboxamide, 2-[(methylcarbonyl)amino]-6-(tert-butyl)-4-oxo-4H-chromene-3-carboxamide, 2-methylamino-6-(tert-butyl)-4-oxo-4H-chromene-3-carboxamide, 2-methylamino-7-(tert-butyl)-4-oxo-4H-chromene-3-carboxamide, 2-[(methylaminocarbonyl)amino]-6-(tert-butyl)-4-oxo-4H-chromene-3-carboxamide, 3-cyano-2-dimethylamino-8-isopropyl-5-methyl-4-oxo-4H-chromene, 3-cyano-2-diethylamino-7-methoxy-4-oxo-4H-chromene, 2-dimethylamino-8-isopropyl-5-methyl-4-oxo-4H-chromene-3-carboxaldehyde oxime, 2-diethylamino-7-methoxy-4-oxo-4H-chromene-3-carboxaldehyde oxime, 2-(piperidin-1-yl)-4-oxo-4H-chromene-3-carbaldehyde, 3-methylcarbonyl-2-(piperidin-1-yl)-4-oxo-4H-chromene, 3-methylcarbonyl-2-amino-4-oxo-4H-chromene, 3-methylcarbonyl-2-(N-methyl-N-isopropylamino)-4-oxo-4H-chromene,
and those compounds I in which either R⁴ is cyano, CONH₂ or formyl, R² and R³ are each hydrogen and R¹ is hydrogen, hydroxyl, halogen, C₁-C₆-alkyl, C₁-C₆-alkoxy, phenyl, nitro, amino or dimethylamino,
or in which R⁴ is formyl, R² and R³ are both methyl or ethyl and R¹ is hydrogen, methoxy, 2 methyl groups or a methyl and an isopropyl group,
or in which R⁴ is -CH=N-OH and R¹, R² and R³ are each hydrogen.

2. A 2-amino-4-oxo-4H-benzopyran of the formula I as claimed in claim 1, wherein R³ is -C(O)R⁹ and R⁴ is cyano.

3. A 2-amino-4-oxo-4H-benzopyran of the formula I, where R⁴ is -CH=N-R¹⁴.

4. A process for the preparation of a compound I as claimed in claim 1, wherein a 2-amino-4-oxo-4H-benzopyran-3-carbaldehyde II 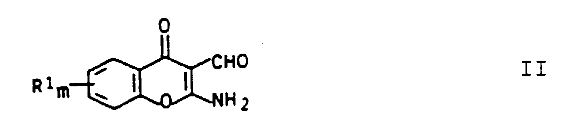 is converted in a conventional manner into an o-aminonitrile III 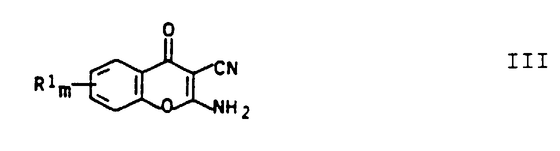 and the latter is derivatized at the amino group by reaction with an electrophile such as IV or V
Hal-CX-R⁹ IV
O=CR²R¹¹ V

5. A herbicide containing, in addition to inert additives, at least one 2-amino-4-oxo-4H-benzopyran of the formula I' 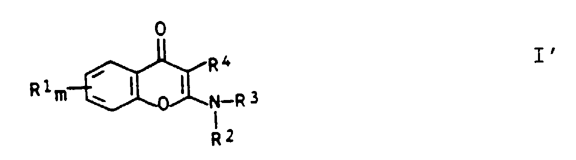 where
R¹ is hydrogen, hydroxyl, halogen, nitro, C₁-C₆-alkyl, C₁-C₆-alkoxy, C₁-C₆-haloalkyl, C₁-C₄-haloalkoxy, C₁-C₄-alkylthio or a group -NR⁵R⁶,
R⁵ is hydrogen or C₁-C₄-alkyl,
R⁶ is hydrogen, C₁-C₄-alkyl, -CO-R⁷, -CS-R⁷ or -SO₂-R⁸,
R⁷ is C₁-C₂₀-alkyl, C₁-C₆-haloalkyl, C₃-C₈-cycloalkyl, phenyl-C₁-C₃-alkyl, amino, C₁-C₄-alkylamino, di-C₁-C₄-alkylamino, phenyl or phenylamino where the phenyl radicals are each unsubstituted or carry from one to three of the groups stated for R¹,
R⁸ is C₁-C₄-alkyl or is phenyl which may carry from one to three of the groups stated for R¹,
m is 0, 1 or 2, and the radicals R¹ may be different when m is 2,
R² is hydrogen or C₁-C₄-alkyl,
R³ is hydrogen, C₁-C₄-alkyl or a group -CO-R⁹, -CS-R⁹ or -SO₂R¹⁰,
R⁹ has one of the meanings stated under R⁷,
R¹⁰ has one of the meanings stated under R⁸,
or R² and R³, together with the nitrogen atom to which they are bonded, form a 3-membered to 8-membered saturated or unsaturated ring which, if desired, may in turn furthermore carry from one to three C₁-C₄-alkyl radicals, or a group =CR²R¹¹,
R¹¹ is hydrogen, hydroxyl, C₁-C₄-alkyl, C₁-C₈-alkoxy, C₃-C₈-cycloalkyl, or benzyl or is phenyl which is unsubstituted or carries from one to three of the groups stated for R¹, or is -O-CO-R¹² or -O-CS-R¹²,
R¹² is C₁-C₂₀-alkyl, C₁-C₆-haloalkyl, C₃-C₈-cycloalkyl, phenyl or phenyl-C₁-C₃-alkyl, where the phenyl radicals may each be unsubstituted or may carry from one to three of the radicals stated for R¹,
R⁴ is cyano or a group -CO-R¹³, -CS-R¹³ or -CH=N-R¹⁴,
R¹³ is hydrogen, C₁-C₆-alkyl, C₁-C₆-haloalkyl, amino, C₁-C₄-alkylamino, di-C₁-C₄-alkylamino, hydrazino, C₁-C₆-alkylhydrazino or phenylhydrazino, where the phenyl radical is unsubstituted or substituted by from one to three of the groups stated for R¹ and
R¹⁴ is one of the radicals R¹¹,
or the agriculturally useful salts of the compounds I' and at least one herbicidal active ingredient selected from the group consisting of
A) the 2-(4-hetaryloxy)- or 2-(4-aryloxy)-phenoxyacetic acid derivatives of the formula VIII 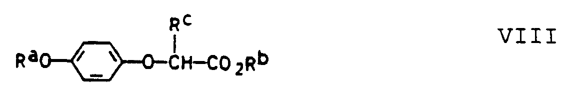 where
R^{a} is a phenyl ring, a pyridyl ring, benzoxazyl, benzothiazyl or benzopyrazinyl, where these aromatic ring systems may carry up to two of the following radicals: halogen, nitro, C₁-C₄-alkyl, C₁-C₄-haloalkyl or C₁-C₄-haloalkoxy,
R^{b} is hydrogen, C₁-C₄-alkyl or one equivalent of a plant-tolerated cation and
R^{c} is hydrogen or methyl,
and/or
B) the cyclohexenone oxime ethers of the formula IX 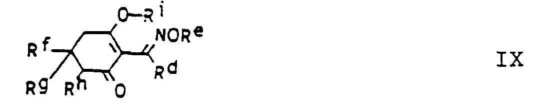 where
R^{d} is C₁-C₄-alkyl,
R^{e} is C₁-C₄-alkyl, C₃- or C₄-alkenyl, C₃- or C₄-alkynyl or partially or completely halogenated C₃- or C₄-alkenyl, a C₁-C₄-alkylene or C₃- or C₄-alkenylene chain, both of which may furthermore carry C₁-C₃-alkyl or halogen, or a 3-membered to 6-membered alkylene or 4-membered to 6-membered alkenylene chain which, if desired, is substituted by C₁-C₃-alkyl and each of which contains, as a chain member, an oxygen or sulfur atom not directly adjacent to the oxime ether moiety, all abovementioned chains carrying a terminal phenyl ring which in turn may be substituted by from one to three radicals selected from the group consisting of a benzyloxycarbonyl or phenyl radical and from one to three of each of the following radicals: nitro, cyano, halogen, C₁-C₄-alkyl, C₁-C₄-alkoxy, C₁-C₄-alkylthio, partially or completely halogenated C₁-C₄-alkyl, partially or completely halogenated C₁-C₄-alkoxy, carboxyl and C₁-C₄-alkoxycarbonyl, and it being possible for the phenyl ring additionally to carry a number of halogen atoms such that the total number of radicals is 4 or 5,
or thienylmethyl which may furthermore carry a halogen atom,
R^{f} is C₁-C₄-alkyl which may be monosubstituted by C₁-C₄-alkylthio or C₁-C₄-alkoxy,
a 5-membered or 6-membered saturated or monounsaturated ring system which, in addition to carbon members, may contain an oxygen or sulfur atom or a sulfoxyl or sulfonyl group, where this ring may carry up to three of the following radicals: hydroxyl, halogen, C₁-C₄-alkyl, partially or completely halogenated C₁-C₄-alkyl, C₁-C₄-alkoxy or C₁-C₄-alkylthio,
a 10-membered saturated or monounsaturated heterocyclic structure which contains two oxygen atoms or sulfur atoms and may be substituted by up to three C₁-C₄-alkyl groups or methoxy groups,
phenyl, pyridyl, thiazolyl, pyrazolyl, pyrrolyl or isoxazolyl, where each of these groups may carry from one to three radicals selected from the group consisting of C₁-C₄-alkyl, partially or completely halogenated C₁-C₄-alkyl, C₁-C₄-alkoxy, C₁-C₄-alkylthio, C₃-C₆-alkenyloxy, C₃-C₆-alkynyloxy, C₁-C₄-dialkoxy-C₁-C₃-alkyl, C₂-C₈-alkoxyalkyl, C₂-C₈-alkylthioalkyl, formyl, halogen and benzoylamino,
R^{g} is hydrogen or hydroxyl or, if R^{f} is C₁-C₆-alkyl, R⁹ is C₁-C₆-alkyl,
R^{h} is hydrogen, cyano, halogen, C₁-C₄-alkoxycarbonyl or C₁-C₄-alkylketoxime and
Rⁱ is hydrogen or one equivalent of an agriculturally useful cation.

6. A herbicide as claimed in claim 5, wherein the weight ratio of 2-amino-4-oxo-4H-benzopyran I' to herbicidal active ingredient A) or B) is from 10 : 1 to 0.01 : 1.

7. A method for selectively controlling undesirable plant growth, wherein a 2-amino-4-oxo-4H-benzopyran of the formula I' as claimed in claim 5 and
A) a 2-(4-hetaryloxy)- or 2-(4-aryloxy)-phenoxyacetic acid derivative of the formula VIII or
B) a cyclohexenone derivative of the formula IX as claimed in claim 5 are applied simultaneously or in succession, before, during or after sowing of the crops or before or during emergence of the crops.

8. A method for preventing damage to crops by herbicidal
A) 2-(4-hetaryloxy)- or 2-(4-aryloxy)-phenoxyacetic acid derivatives of the formula VIII or
B) cyclohexenone derivatives of the formula IX as claimed in claim 5, wherein the seed of the crops is treated with an antagonistic amount of a 2-amino-4-oxo-4H-benzopyran of the formula I' as claimed in claim 5.

9. A method for selectively controlling undesirable plant growth, wherein the leaves of the crops and of the undesirable plants are treated by the postemergence method, simultaneously or in succession, with a 2-amino-4-oxo-4H-benzopyran of the formula I' as claimed in claim 5 and with
A) a 2-(4-hetaryloxy)- or 2-(4-aryloxy)-phenoxyacetic acid derivative of the formula VIII or
B) a cyclohexenone derivative of the formula IX as claimed in claim 5.

10. A method as claimed in claim 7, wherein the crops are barley, wheat, corn, millet or rice.

11. A method as claimed in claim 8, wherein the crops are barley, wheat, corn, millet or rice.

12. A method as claimed in claim 9, wherein the crops are barley, wheat, corn, millet or rice.

## Revendications

1. 2-amino-4-oxo-4H-benzopyrannes de formule générale I 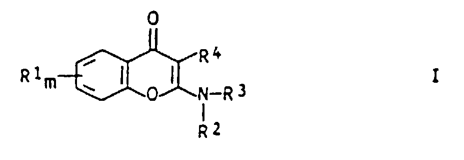 dans laquelle les symboles et l'indice ont les significations suivantes :
R¹ ; l'hydrogène ; un groupe hydroxy ; un halogène ; un groupe nitro ; un groupe alkyle en C1-C6, alcoxy en C1-C6 ; un groupe halogénoalkyle en C1-C6 ; un groupe halogénoalcoxy en C1-C4 ; un groupe alkylthio en C1-C4 ou un groupe -NR⁵R⁶ dans lequel
R⁵ représente l'hydrogène ou un groupe alkyle en C1-C4 et
R⁶ représente l'hydrogène, un groupe alkyle en C1-C4, -CO-R⁷, -CS- R⁷ ou -SO₂-R⁸ dans lesquels
R⁷ représente un groupe alkyle en C1-C20 ; halogénoalkyle en C1-C6 ; cycloalkyle en C3-C8 ; phényl-alkyle en C1-C3 ; amino ; alkylamino en C1-C4 ; di-(alkyle en C1-C4)-amino ; un groupe phényle ou phénylamino, dont les radicaux phényles peuvent être non substitués ou porter un à trois des substituants mentionnés en référnece à R¹ ; et
R⁸ représente un groupe alkyle en C1-C4 ou phényle, lequel peut porter un à trois des substituants mentionnés en référence à R¹,
m est égal à 0, 1 ou 2, les symboles R¹ pouvant avoir des significations différentes lorsque m est égal à 2 ;
R² représente l'hydrogène ou un groupe alkyle en C1-C4 ;
R³ représente l'hydrogène ; un groupe alkyle en C1-C4 ; un groupe -CO-R⁹, -CS-R⁹ ou -SO₂-R¹⁰, dans lesquels
R⁹ a l'une des significations indiquées pour R⁷ et R¹⁰ a l'une des significations indiquées pour R⁸ ;
ou bien
R² et R³ forment ensemble et avec l'atome d'azote auquel ils sont reliés un cycle saturé ou insaturé de trois à huit chaînons qui peut lui-même porter un trois groupes alkyle en C1-C4 ou un groupe =CR²R¹¹, dans lequel
R¹¹ représente l'hydrogène ; un groupe hydroxy ; alkyle en C1-C4 ; alcoxy en C1-C8 ; cycloalkyle en C3-C8 ; phényle ; benzyle ; phényle portant un à trois des substituants mentionnés en référence à R¹ ; un groupe -O-CO-R¹² ou -O-CS-R¹² dans lequel
R¹² représente un groupe alkyle en C1-C20 ; halogénoalkyle en C1-C6 ; cycloalkyle en C3-C8 ; phényle ou phényl-alkyle en C1-C3 dont les radicaux phényles peuvent eux-mêmes être non substitués ou porter un à trois des substituants mentionnés en référence à R¹ ;
R⁴ représente un groupe cyano ; un groupe -CO-R¹³, -CS-R¹³ ou -CH= N-R¹⁴ dans lesquels R¹³ représente l'hydrogène ; un groupe alkyle en C1-C6 ; halogénoalkyle en C1-C6 ; amino ; alkylamino en C1-C4 ; di-(alkyle en C1-C4)-amino ; hydrazino ; (alkyle en C1-C6)-hydrazino ; phénylhydrazino dont le radical phényle peut être non substitué ou porter un à trois des substituants mentionnés en référence à R¹, et
R¹⁴ a l'une des significations indiquées en référence à R¹¹ ;
et les sels des composés I convenant pour des applications agricoles, à l'exception des composés suivants : 2-[(méthylcarbonyl)amino]-4-oxo-4H-chromène-3-carboxamide, 2-[(méthylcarbonyl)amino]-6-(tert.-butyl)-4-oxo-4H-chromène-3-carboxamide, 2-méthylamino-6-(tert.-butyl)-4-oxo-4H-chromène-3-carboxamide, 2-méthylamino-7-(tert.-butyl)-4-oxo-4H-chormène-3-carboxamide, 2-[(méthylamino-carbonyl)-amino]-6-(tert.-butyl)-4-oxo-4H-chromène-3-carboxamide, 3-cyano-2-diméthylamino-8-isopropyl-5-méthyl-4-oxo-4H-chromène, 3-cyano-2-diéthylamino-7-méthoxy-4-oxo-4H-chromène, 2-diméthyl-amino-8-isopropyl-5-méthyl-4-oxo-4H-chromène-3-carboxaldoxime, 2-diéthylamino-7-méthoxy-4-oxo-4H-chromène-3-carboxaldoxime, 2-(pipéridin-1-yl)-4-oxo-4H-chromène-3-carbaldéhyde, 3-méthylcarbo-nyl-2-(pipéridin-1-yl)-4-oxo-4H-chromène, 3-méthyl-carbonyl-2-amino-4-oxo-4H-chromène, 3-méthylcarbonyl-2-(N-méthyl-N-isopro-pylamino)-4-oxo-4H-chromène,
et des composés I pour lesquels ou bien R⁴ représente un groupe cyano, un groupe CONH₂ ou le groupe formyle, R² et R³ l'hydrogène et R¹ l'hydrogène, un groupe hydroxy, un halogène, un groupe alkyle en C1-C6, alcoxy en C1-C6, phényle, nitro, amino et/ou diméthylamino,
ou bien R⁴ représente le groupe formyle, R² et R³ tous deux des groupes méthyle ou éthyle, R¹ l'hydrogène, un groupe méthoxy, deux groupes méthyle ou un groupe méthyle et un groupe isopropyle,
ou bien R⁴ représente un groupe -CH=N-OH et R¹, R² et R³ représentent l'hydrogène.

2. 2-amino-4-oxo-4H-benzopyrannes de formule I selon la revendicaiton 1, dans laquelle R³ représente un groupe -C(O)R⁹ et R⁴ représente un groupe cyano.

3. 2-amino-4-oxo-4H-benzopyrannes de formule I dans laquelle R⁴ représente un groupe -CH=N-R¹⁴.

4. Procédé de préparation des composés I selon la revendication 1, caractérisé par le fait que l'on convertit un 2-amino-4-oxo-4H-benzopyranne-3-carbaldéhyde II 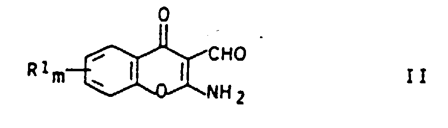 de manière connue en soi, en l'o-aminonitrile III 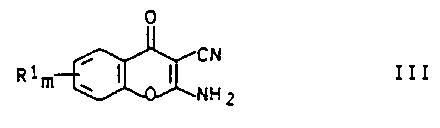 dont on convertit le groupe amino par réaction avec des agents électrophiles tels que IV et V
Hal-CX-R⁹ IV
O=CR²R¹¹ V

5. Produit herbicide contenant, avec des additifs inertes, au moins un 2-amino-4-oxo-4H-benzopyranne de formule générale I' 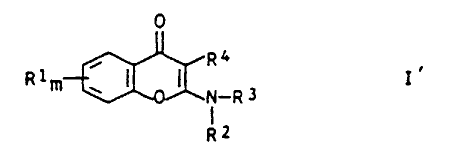 dans laquelle les symboles et l'indice ont les significations suivantes :
R¹ : l'hydrogène ; un groupe hydroxy ; un halogène ; un groupe nitro ; un groupe alkyle en C1-C6, alcoxy en C1-C6 ; un groupe halogénoalkyle en C1-C6 ; un groupe halogénoalcoxy en C1-C6 ; un groupe alkylthio en C1-C4 ou un groupe -NR⁵R⁶ dans lequel
R⁵ représente l'hydrogène ou un groupe alkyle en C1-C4 et
R⁶ représente l'hydrogène, un groupe alkyle en C1-C4, -CO-R⁷, -CS-R⁷ ou -SO₂-R⁸ dans lesquels
R⁷ représente un groupe alkyle en C1-C20 ; halogénoalkyle en C1-C6 ; cycloalkyle en C3-C8 ; phényl-alkyle en C1-C3 ; amino ; alkylamino en C1-C4 ; di-(alkyle en C1-C4)-amino ; un groupe phényle ou phénylamino, dont les radicaux phényles peuvent être non substitués ou porter un à trois des substituants mentionnés en référnece à R¹ ; et
R⁸ représente un groupe alkyle en C1-C4 ou phényle, lequel peut porter un à trois des substituants mentionnés en référence à R¹,
m est égal à 0, 1 ou 2, les symboles R¹ pouvant avoir des significations différentes lorsque m est égal à 2 ;
R² représente l'hydrogène ou un groupe alkyle en C1-C4 ;
R³ représente l'hydrogène ; un groupe alkyle en C1-C4 ; un groupe -CO-R⁹, -CS-R⁹ ou -SO₂-R¹⁰, dans lesquels
R⁹ a l'une des significations indiquées pour R⁷ et R¹⁰ a l'une des significations indiquées pour R⁸ ;
ou bien
R² et R³ forment ensemble et avec l'atome d'azote auquel ils sont reliés un cycle saturé ou insaturé de trois à huit chaînons qui peut lui-même porter un trois groupes alkyle en C1-C4 ou un groupe =CR²R¹¹, dans lequel
R¹¹ représente l'hydrogène ; un groupe hydroxy ; alkyle en C1-C4 ; alcoxy en C1-C8 ; cycloalkyle en C3-C8 ; phényle ; benzyle ; phényle portant un à trois des substituants mentionnés en référence à R¹ ; un groupe -O-CO-R¹² ou -O-CS-R¹² dans lequel
R¹² représente un groupe alkyle en C1-C20 ; halogénoalkyle en C1-C6 ; cycloalkyle en C3-C8 ; phényle ou phényl-alkyle en C1-C3 dont les radicaux phényles peuvent eux-mêmes être non substitués ou porter un à trois des substituants mentionnés en référence à R¹ ;
R⁴ représente un groupe cyano ; un groupe -CO-R¹³, -CS-R¹³ ou -CH= N-R¹⁴ dans lesquels
R¹³ représente l'hydrogène ; un groupe alkyle en C1-C6 ; halogénoalkyle en C1-C6 ; amino ; alkylamino en C1-C4 ; di-(alkyle en C1-C4)-amino ; hydrazino ; (alkyle en C1-C6)-hydrazino ; phénylhydrazino dont le radical phényle peut être non substitué ou porter un à trois des substituants mentionnés en référence à R¹, et
R¹⁴ a l'une des significations indiquées en référence à R¹¹,
et les sels de composés I' convenant pour les applications agricoles, et au moins une substance active herbicide du groupe
A) des dérivés d'acides 2-(4-hétéroaryloxy)- ou 2-(4-aryloxy)-phénoxyacétiques de formule VIII 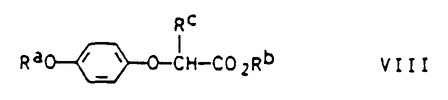 dans laquelle
R^{a} représente un cycle phényle, un cycle pyridyle, un radical benzoxazyle, un radical benzothiazyle ou un radical benzopyrazinyle, ces systèmes cycliques aromatiques pouvant porter jusqu'à deux des substituants suivants : les halogènes, les groupes nitro, alkyle en C1-C4, halogénoalkyle en C1-C4 et/ou halogénoalcoxy en C1-C4,
R^{b} représente l'hydrogène, un groupe alkyle en C1-C4 ou l'équivalent d'un cation toléré par les végétaux et
R^{c} représente l'hydrogène ou un groupe méthyle,
et/ou
B) des éthers de cyclohexénonoximes de formule IX 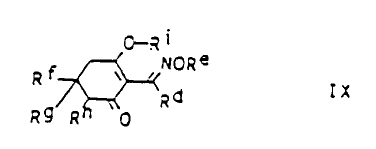 dans laquelle les symboles ont les significations suivantes :
R^{d} représente un groupe alkyle en C1-C4 ;
R^{e} représente un groupe alkyle en C1-C4, alcényle en C3-C4, alcynyle en C3-C4 ou un groupe alcényle en C3-C4 partiellement ou totalement halogéné ;
une chaîne alkylène en C1-C4 ou alcénylène en C3-C4 qui peuvent encore porter un groupe alkyle en C1-C3 et/ou un atome d'halogène, ou une chaîne alkylène de trois à six chaînons ou alcénylène de quatre à six chaînons éventuellement substituée par des groupes alkyle en C1-C4 et qui contiennent chacune en tant que chaînon un atome d'oxygène ou de soufre qui n'est pas directement voisin de la partie éther d'oxime, toutes les chaînes mentionnés ci-dessus pouvant porter en position terminale le cycle phényle qui peut lui-même porter un à trois substituants choisis dans un groupe consistant en un radical benzyloxycarbonyle ou phényle qui peuvent porter chacun un à trois des substituants suivants : nitro, cyano, halogéno, alkyle en C1-C4, alcoxy en C1-C4, alkylthio en C1-C4, alkyle en C1-C4 partiellement ou totalement halogéné, alcoxy en C1-C4 partiellement ou totalement halogéné, carboxyle et (alcoxy en C1-C4)carbonyle, le cycle phényle pouvant encore porter des atomes d'halogènes en quantité telle que le nombre total des substituants soit de 4 ou 5 ;
un groupe thiénylméthyle qui peut encore porter un atome d'halogène ;
R^{f} représente un groupe alkyle en C1-C4 qui peut porter un substituant alkylthio en C1-C4 ou alcoxy en C1-C4 ;
un système cyclique saturé ou mono-insaturé à cinq ou six chaînons qui, en plus des chaînons carbonés, peut contenir un atome d'oxygène, un atome de soufre ou un groupe sulfoxyde ou sulfone, ce cycle pouvant porter jusqu'à trois des substituants suivants : hydroxy, halogéno, alkyle en C1-C4, alkyle en C1-C4 partiellement ou totalement halogéné, alcoxy en C1-C4 et/ou alkylthio en C1-C4 ;
un hétérocycle saturé ou mono-insaturé à dix chaînons qui contient deux atomes d'oxygène ou de soufre et peut porter jusqu'à trois substituants alkyle en C1-C4 et/ou méthoxy ;
le groupe phényle, le groupe pyridyle, le groupe thiazolyle, le groupe pyrazolyle, le groupe pyrrolyle ou le groupe isoxazolyle, ces groupes pouvant porter chacun jusqu'à trois substituants choisis parmi les groupes alkyle en C1-C4, alkyle en C1-C4 partiellement ou totalement halogénés, alcoxy en C1-C4, alkylthio en C1-C4, alcényloxy en C3-C6, alcynyloxy en C3-C6, di-(alcoxy en C1-C4)alkyle en C1-C3, alcoxyalkyle en C2C-8, alkylthioalkyle en C2-C8, formyle, halogéno et benzoylamino ;
R^{g} représente l'hydrogène, un groupe hydroxy, ou bien encore un groupe alkyle en C1-C6 lorsque R^{f} représente un groupe alkyle en C1-C6;
R^{h} représente l'hydrogène, le groupe cyano, un atome d'halogène, un groupe (alcoxy en C1-C4)carbonyle ou un groupe (alkyle en C1-C4)cétoxime et
Rⁱ représente l'hydrogène ou un équivalent d'un cation toléré par les végétaux.

6. Produit herbicide selon la revendication 5, caractérisé par le fait que les proportions relatives en poids entre le 2-amino-4-oxo-4H-benzopyranne I' et la substance herbicide active A) ou B) sont de 10 : 1 à 0,01 : 1 parties en poids.

7. Procédé pour combattre sélectivement les croissances de végétaux indésirables, caractérisé par le fait que l'on applique simultanément ou successivement lors des semis des végétaux cultivés ou après les semis, avant ou durant l'émergence des végétaux cultivés, un 2-amino-4-oxo-4H-benzopyranne de formule I' selon la revendication 5 et
A) un dérivé d'acide 2-(4-hétéroaryloxy)- ou 2-(4-hétéroaryloxy)- ou 2-(4-aryloxy)-phénoxyacétique de formule VIII ou
B) un dérivé de cyclohexénone de formule IX.

8. Procédé pour protéger les végétaux cultivés contre les dommages provoqués par les produits herbicides
A) dérivés d'acides 2-(4-hétéroaryloxy)- ou 2-(4-aryloxy)-phénoxyacétiques de formule VIII ou
B) dérivés de cyclohexénonomes de formule IX,
selon la revendication 5, caractérisé par le fait que l'on traite les semences des végétaux cultivés par une quantité antagoniste efficace d'un 2-amino-4-oxo-4H-benzopyranne de formule I' selon la revendication 5.

9. Procédé pour combattre sélectivement les croissances de végétaux indésirables, caractérisé par le fait que l'on traite les feuilles des végétaux cultivés et des végétaux indésirables en post-levée, simultanément ou successivement, par un 2-amino-4-oxo-4H-benzopyranne de formule I' selon la revendication 5 et par
A) un dérivé d'acide 2-(4-hétéroaryloxy)- ou 2-(4-aryloxy)-phénoxyacétique de formule VIII, ou
B) un dérivé de cylohexénone de formule IX.

10. Procédé selon la revendication 7, caractérisé par le fait que la plante cultivée est l'orge, le blé, le maïs, le sorgho cultivé ou le riz.

11. Procédé selon la revendication 8, caractérisé par le fait que la plante cultivée est l'orge, le blé, le maïs, le sorgho cultivé ou le riz.

12. Procédé selon la revendication 9, caractérisé par le fait que la plante cultivée est l'orge, le blé, le maïs, le sorgho cultivé ou le riz.
